# EUROPEAN PATENT SPECIFICATION

(11) **EP 1 601 673 B1**
(45) Date of publication and mention of the grant of the patent: **10.06.2009**
(21) Application number: 04717339.8
(22) Date of filing: 04.03.2004
(51) Int. Cl.: C07D 471/04, C07D 487/04, C07D 491/044, C07D 495/04, C07D 513/04, A61K 31/519, A61P 35/00, A61P 29/00, A61P 19/08, A61P 19/02, A61P 17/06

(54) **FUSED HETEROCYCLES AND USES THEREOF**
KONDENSIERTE HETEROCYCLEN UND DEREN VERWENDUNGEN
HETEROCYCLES FUSIONNES ET LEURS UTILISATIONS

(30) Priority: 07.03.2003 SE 0300627; 15.04.2003 SE 0301138; 10.06.2003 SE 0301697; 24.10.2003 SE 0302826
(43) Date of publication of application: 07.12.2005
(73) Proprietor: AstraZeneca AB, 151 85 Södertälje (SE)
(72) Inventor: AQUILA, Brian, Astrazeneca R & D Boston, Waltham, Massachusetts 02451 (US); BLOCK, Michael, Howard, Astrazeneca R & D Boston, Waltham, Massachusetts 02451 (US); DAVIES, Audrey, Astrazeneca R & D Boston, Waltham, Massachusetts 02451 (US); EZHUTHACHAN, Jayachandran, Astrazeneca R&D Boston, Waltham, Massachusetts 02451 (US); FILLA, Sandra, Brownsberg, Indiana 46112 (US); LUKE, Richard, William, Astrazeneca R & D Alderley, Macclesfield, Cheshire SK10 4TG (GB); PONTZ, Timothy, Astrazeneca R & D Boston, Waltham, Massachusetts 02451 (US); THEOCLITOU, Maria-Elena, Astrazeneca R & D Boston, Waltham, Massachusetts 02451 (US); ZHENG, XiaoLan, Astrazeneca R & D Boston, Waltham, Massachusetts 02451 (US)
(86) International application number: PCT/SE2004/000304
(87) International publication number: WO 2004/078758

(56) References cited:
- WO-A1-01/98278
- WO-A1-02/083143
- WO-A2-03/049527
- WO-A2-03/049679
- WO-A2-03/050064
- WO-A2-03/050122

## Description

### Field of the invention

The present invention relates to novel fused heterocycles, their pharmaceutical compositions and methods of use. In addition, the present invention relates to therapeutic methods for the treatment and prevention of cancers.

### Background of the invention

One sub-class of anti-cancer drugs now used extensively in the clinic (taxanes, vinca-alkaloids) are directed at microtubules and block the cell division cycle by interfering with normal assembly or disassembly of the mitotic spindle (see Chabner, B. A., Ryan, D. P., Paz-Ares, l., Garcia-Carbonero, R., and Calabresi, P: Antineoplastic agents. In Hardman, J. G., Limbird, L.E., and Gilman, A. G., eds. Goodman and Gilman's The Pharmacological Basis of Therapeutics, 10th edition, 2001, The MacGraw-Hill Companies, Inc). Taxol® (paclitaxel), one of the most effective drugs of this class, is a microtubule stabilizer. It interferes with the normal growth and shrinkage of microtubules thus blocking cells in the metaphase of mitosis. Mitotic block is often followed by slippage into the next cell cycle without having properly divided, and eventually by apoptosis of these abnormal cells (Blagosklonny, M.V. and Fojo, T.: Molecular effects of paclitaxel: myths and reality (a critical review). Int J Cancer 1999, 83:151-156.)

Some of the side effects of treatment with paclitaxel are neutropenia and peripheral neuropathy. Paclitaxel is known to cause abnormal bundling of microtubules in interphase cells. In addition, some tumour types are refractory to treatment with paclitaxel, and other tumours become insensitive during treatment. Paclitaxel is also a substrate for the multi-drug resistance pump, P-glycoprotein ((see Chabner *et al*., 2001).

Thus, there is a need for effective anti-mitotic agents that are more specific and have fewer side effects than anti-microtubule drugs, and also for agents that are effective against taxane-resistant tumours.

Kinesins are a large family of molecular motor proteins, which use the energy of ATP hydrolysis to move in a stepwise manner along microtubules. For a review, see Sablin, E.P.: Kinesins and microtubules: their structures and motor mechanisms. Curr Opin Cell Biol 2000, 12:35-41 and Schief, W. R. and Howard, J.: Conformational changes during kinesin motility. Curr Opin Cell Biol 2001, 13:19-28.

Some members of this family transport molecular cargo along microtubules to the sites in the cell where they are needed. For example, some kinesins bind to vescicles and transport them for long distances along microtubules in axons. Several family members are mitotic kinesins, as they play roles in the reorganization of microtubules that establishes a bipolar mitotic spindle. The minus ends of the microtubules originate at the centrosomes, or spindle poles, whilst the plus ends bind to the kinetochore at the centromeric region of each chromosome. Thus the mitotic spindle lines up the chromosomes at metaphase of mitosis and coordinates their movement apart and into individual daughter cells at anaphase and telophase (cytokinesis). See Alberts, B., Bray, D., Lewis, J., Raff, M., Roberts, K., and Watson, J. D., Molecular Biology of the Cell, 3rd edition, Chapter 18, The Mechanics of Cell Division, 1994, Garland Publishing, Inc. New York.

HsEg5 (Accession X85137; see Blangy, A., Lane H.A., d'Heron, P., Harper, M., Kress, M. and Nigg, E.A.: Phosphorylation by p34cdc2 regulates spindle association of human Eg5, a kinesin-related motor essential for bipolar spindle formation in vivo. Cell 1995, 83(7): 1159-1169) or, KSP, is a mitotic kinesin whose homologs in many organisms have been shown to be required for centrosome separation in the prophase of mitosis, and for the assembly of a bipolar mitotic spindle. For a review see Kashina, A.S., Rogers, G.C., and Scholey, J.M.: The bimC family of kinesins: essential bipolar mitotic motors driving centrosome separation. Biochem Biophys Acta 1997, 1357: 257-271. Eg5 forms a tetrameric motor, and it is thought to cross-link microtubules and participate in their bundling (Walczak, C. E., Versos, I., Mitchison, T. J., Karsenti, E., and Heald, R.: A model for the proposed roles of different microtubule-based motor proteins in establishing spindle bipolarity. Curr Biol 1998, 8:903-913). Several reports have indicated that inhibition of Eg5 function leads to metaphase block in which cells display monastral spindles. Recently an Eg5 inhibitor called monastrol was isolated in a cell-based screen for mitotic blockers (Mayer, T.U., Kapoor, T. M., Haggarty, S.J., King, R.W., Schreiber, S.L., and Mitchison, T.J.: Small molecule inhibitor of mitotic spindle bipolarity identified in a phenotype-based screen. Science 1999,286: 971-974).

Monastrol treatment was shown to be specific for Eg5 over kinesin heavy chain, another closely related motor with different functions (Mayer *et al*., 1999). Monastrol blocks the release of ADP from the Eg5 motor (Maliga, Z., Kapoor, T. M., and Mitchison, T.J.: Evidence that monastrol is an allosteric inhibitor of the mitotic kinesin Eg5. Chem & Biol 2002, 9: 989-996 and DeBonis, S., Simorre, J.-P., Crevel, I., Lebeau, L, Skoufias, D. A., Blangy, A., Ebel, C., Gans, P., Cross, R., Hackney, D. D., Wade, R. H., and Kozielski, F.: Interaction of the mitotic inhibitor monastrol with human kinesin Eg5. Biochemistry 2003, 42: 338-349) an important step in the catalytic cycle of kinesin motor proteins (for review, see Sablin, 2000; Schief and Howard, 2001). Treatment with monastrol was also shown to be reversible and to activate the mitotic spindle checkpoint which stops the progress of the cell division cycle until all the DNA is in place for appropriate division to occur (Kapoor, T.M., Mayer, T. U., Coughlin, M. L., and Mitchison, T.J.: Probing spindle assembly mechanisms with monastrol, a small molecule inhibitor of the mitotic kinesin, Eg5. J Cell Biol 2000, 150(5): 975-988). Recent reports also indicate that inhibitors of Eg5 lead to apoptosis of treated cells and are effective against several tumour cell lines and tumour models (Mayer *et al*., 1999).

Although Eg5 is thought to be necessary for mitosis in all cells, one report indicates that it is over-expressed in tumour cells (International Patent Application WO 01/31335), suggesting that they may be particularly sensitive to its inhibition. Eg5 is not present on the microtubules of interphase cells, and is targeted to microtubules by phosphorylation at an early point in mitosis (Blangy *et al*., 1995). See also; Sawin, K. E. and Mitchison, T.J.: Mutations in the kinesin-like protein Eg5 disrupting localization to the mitotic spindle. Proc Natl Acad Sci USA 1995, 92(10): 4289-4293, thus monastrol has no detectable effect on microtubule arrays in interphase cells (Mayer et al., 1999). Another report suggests that Eg5 is involved in neuronal development in the mouse, but it disappears from neurons soon after birth, and thus Eg5 inhibition may not produce the peripheral neuropathy associated with treatment with paclitaxel and other anti-microtubule drugs (Ferhat, L., Expression of the mitotic motor protein Eg5 in postmitotic neurons: implications for neuronal development. J Neurosci 1998, 18(19): 7822-7835). Herein we describe the isolation of a class of specific and potent inhibitors of Eg5, expected to be useful in the treatment of neoplastic disease.

Cytokinetics Inc describes certain quinazolines as useful for treating cellular proliferative diseases and disorders associated with KSP kinesin activity in WO 01/98278. Tularik Inc describes compounds, compositions and methods that are useful in the treatment if inflammatory and immune conditions and diseases (CXCR3 antagonists) in WO 02/083143. Merck and Co describe (i) in WO 03/049527 and WO 03/049679, azolopyrimidinones, (ii) in WO 03/050064, thienopyrimidinones and (iii) in WO 03/050122, furanopyrimidinones, that are useful for treating cellular proliferative diseases, for treating disorders associated with KSP kinesin activity, and for inhibiting KSP kinesin.

### Summary of the invention

In accordance with the present invention, the applicants have hereby discovered novel compounds which possess cell-cycle inhibitory activity and are accordingly useful for their anti-cell-proliferation activity (such as anti-cancer) and are therefore useful in methods of treatment of diseases having cell-proliferation activity in human or animal subjects. In addition to novel compounds the present invention also includes pharmaceutical compositions containing such compounds and to the use of such compounds in the manufacture of medicaments having an anti-cell proliferation effect in human or animal subjects. The invention also relates to processes for the manufacture of said compounds.

The present invention includes pharmaceutically acceptable salts or prodrugs of such compounds. Also in accordance with the present invention applicants provide pharmaceutical compositions and a method to use such compounds in the treatment of cancer.

Such properties are expected to be of value in the treatment of disease states associated with cell cycle and cell proliferation such as cancers (solid tumours and leukaemias), fibroproliferative and differentiative disorders, psoriasis, rheumatoid arthritis, Kaposi's sarcoma, haernangioma, acute and chronic nephropathies, atheroma, atherosclerosis, arterial restenosis, autoimmune diseases, acute and chronic inflammation, bone diseases and ocular diseases with retinal vessel proliferation.

### Detailed description of the invention

In a first embodiment, the present invention provides a novel compound having structural formula (I): 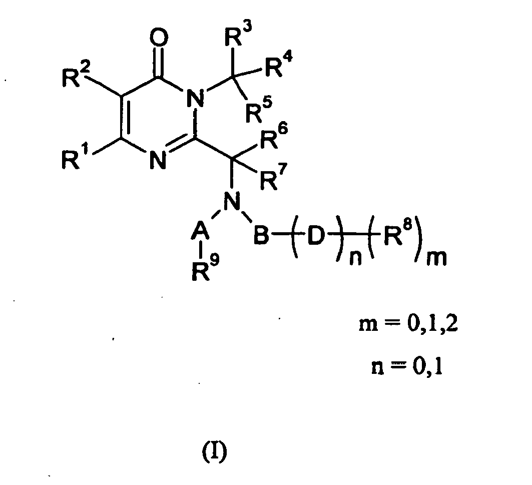 wherein,
A is C=O, CH₂, or SO₂;
B represents optionally substituted C₁₋₁₂alkyl, optionally substituted C₂₋₁₂alkenyl, optionally substituted C₂₋₁₂alkynyl, optionally substituted aryl, optionally substituted C₃₋₁₂cycloalkyl, or optionally substituted heterocycle;
D is O or N wherein O is optionally substituted with one R⁸, wherein N is optionally substituted with one or more R⁸, and when n is 0 and m is not 0, R⁸ is attached directly to B;
R¹ and R² in combination form an optionally substituted fused isothiazole, or an optionally substituted fused isoxazole that is optionally substituted with 1 or 2 substituents;
R³ is independently selected from H, optionally substituted C₁₋₁₂alkyl, optionally substituted C₂₋₁₂alkenyl, optionally substituted C₂₋₁₂alkynyl, optionally substituted C₃₋₁₂cycloalkyl, optionally substituted C₃₋₁₂cycloalkenyl, optionally substituted C₇₋₁₂cycloalkynyl, optionally substituted aryl or optionally substituted heterocycle;
R⁴ and R⁵ are independently selected from H or optionally substituted C₁₋₁₂alkyl, or R⁴ and R⁵ in combination form a 3-, 4-, 5- or 6- membered ring, which may also be optionally substituted;
R⁶ and R⁷ are independently selected from H, optionally substituted C₁₋₁₂alkyl, optionally substituted C₂₋₁₂alkenyl, optionally substituted C₂₋₁₂alkynyl, optionally substituted C₃₋₁₂cycloalkyl, optionally substituted C₃₋₁₂cycloalkenyl, optionally substituted C₇₋₁₂cycloalkynyl,
optionally substituted heterocycle, optionally substituted aryl, or R⁶ and R⁷ in combination form a 3-, 4-, 5- or 6- membered ring, which may also be substituted;
R⁸ is independently selected from H, optionally substituted C₁₋₁₂alkyl, optionally substituted C₂₋₁₂alkenyl, optionally substituted C₂₋₁₂alkynyl, optionally substituted C₃₋₁₂cycloalkyl, optionally substituted C₃₋₁₂cycloalkenyl, optionally substituted C₇₋₁₂cydoalkynyl, optionally substituted aryl, or optionally substituted heterocycle;
R⁹ is independently selected from H, optionally substituted C₁₋₁₂alkyl, optionally substituted C₂₋₁₂alkenyl, optionally substituted C₂₋₁₂alkynyl, optionally substituted C₃₋₁₂cycloalkyl, optionally substituted C₃₋₁₂cycloalkenyl, optionally substituted C₇₋₁₂cycloalkynyl, optionally substituted aryl, or optionally substituted heterocycle; wherein:
   the term "aryl" refers to a hydrocarbon radical having one or more polyunsaturated carbon rings having aromatic character, comprising 5 up to about 14 carbon atoms;
   the term "heterocycle" refers to a ring-containing structure or molecule having one or more multivalent heteroatoms, independently selected from N, O, P and S, as a part of the ring structure and including at least 3 and up to about 20 atoms in the ring(s); heterocycle may be saturated or unsaturated, containing one or more double bonds, and heterocycle may contain more than one ring; when a heterocycle contains more than one ring, the rings may be fused or unfused; heterocycle may have aromatic character or may not have aromatic character;
   and wherein said substituents are selected from -OC₁₋₆alkyl, -C₁₋₆alkyl, F, Cl, Br, I, =O, =S, -NH₂, -OH, -NHCH₃, -N(CH₃)₂, -NH-cyclopropane, -NH-cyclobutane, azetidine, pyrrolidine and piperidine;
or a pharmaceutically acceptable salt thereof, provided that said compound is not:
(±)-N-(3-Amino-propyl)-N-[1-(5-benzyl-3-methyl-4-oxo-4,5-dihydro-isoxazolo[5,4-d]pyrimidin-6-yl)-propyl]-4-methyl-benzamide;
(±)-{3-[[1-(5-Benzyl-3-methyl-4-oxo-4,5-dihydro-isoxazolo[5,4-d]pyrimidin-6-yl)-propyl]-(4-methyl-benzoyl)-amino]-propyl}-carbamic acid tert-butyl ester;
(±)-N-[1-(5-Benzyl-3-methyl-4-oxo-4,5-dihydro-isoxazolo[5,4-d]pyrimidin-6-yl)-propyl]-4-methyl-N-(3-morpholin-4-yl-propyl)-benzamide;
(±)-N-[1-(5-Benzyl-3-methyl-4-oxo-4,5-dihydro-isoxazolo[5,4-d]pyrimidin-6-yl)-propyl]-N-butyl-4-methyl-benzamide;
(±)-N-[1-(5-Benzyl-3-methyl-4-oxo-4,5-dihydro-isoxazolo[5,4-d]pyrimidin-6-yl)-propyl]-N-(3-methoxy-propyl)-4-methyl-benzamide;
(±)-N-[1-(5-Benzyl-3-methyl-4-oxo-4,5-dihydro-isoxazolo[5,4-d]pyrimidin- -6-yl)-propyl]-4-methyl-N-phenethyl-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-4-methyl-benzamide;
(±)-N-[1-(5-Benzyl-3-methyl-4-oxo-4,5-dihydro-isoxazolo[5,4-d]pyrimidin- -6-yl)-propyl]-N-(3-imidazol-1-yl-propyl)-4-methyl-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-3-fluoro-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-3,4-dichloro-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-4-methoxy-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-2-methyl-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-3-methyl-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-4-fluoro-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-4-chloro-benzamide;
(±)-N-[1-(5-Benzyl-3-methyl-4-oxo-4,5-dihydro-isoxazolo[5,4-d]pyrimidin-6-yl)-propyl]-4-tert-butyl-N-(2-carbamoyl-ethyl)-benzamide;
(±)-N-(3-amino-3-oxopropyl)-N-[1-(5-benzyl-3-methyl-4-oxo-4,5-dihydroisoxazolo[5,4-d]pyrimidin-6-yl)propyl]-4-butylpyridine-3-carboxamide;
(±)-3-{[1-(5-Benzyl-3-methyl-4-oxo-4,5-dihydro-isoxazolo[5,4-d]pyrimidin-6-yl)-propyl]-[2-(4-chloro-phenyl)-2-oxo-ethyl]-amino}-propionamide;
(±)-3-[[1-(5-Benzyl-3-methyl-4-oxo-4,5-dihydro-isoxazolo[5,4-d]pyrimidin-6-yl)propyl]-(toluene-3-sulfonyl)-amino]-propionamide;
(±)-3-[[1-(5-Benzyl-3-methyl-4-oxo-4,5-dihydro-isoxazolo[5,4-d]pyrimidin-6-yl)-propyl]-(1-methyl-1H-imidazole-4-sulfonyl)-amino]-propionamide;
(±)-N-(3-amino-3-oxopropyl)-N-[1-(5-benzyl-3-methyl-4-oxo-4,5-dihydroisoxazolo[5,4-d]pyrimidin-6-yl)propyl]-5-methylisoxazole-3-carboxamide;
(±)-N-(3-amino-3-oxopropyl)-N-[1-(5-benzyl-3-methyl-4-oxo-4,5-dihydroisoxazolo[5,4-d]pyrimidin-6-yl)propyl]-2,2-dimethylhexanamide;
(±)-N-(3-amino-3-oxopropyl)-N-[1-(5-benzyl-3-methyl-4-oxo-4,5-dihydroisoxazolo[5,4-d]pyrimidin-6-yl)propyl]-2-(1-methylethyl)hexanamide;
(±)-3-{Acetyl-[1-(5-benzyl-3-methyl-4-oxo-4,5-dihydro-isoxazolo[5,4-d]pyrimidin-6-yl)-propyl]-amino}-propionamide;
(±)-3-[[1-(5-Benzyl-3-methyl-4-oxo-4,5-dihydro-isoxazolo[5,4-d]pyrimidin-6-yl)-propyl]-(2-methoxy-acetyl)-amino]-propionamide;
(±)-3-[[1-(5-Benzyl-3-methyl-4-oxo-4,5-dihydro-isoxazolo[5,4-d]pyrimidin-6-yl)-propyl]-(2,2,2-trifluoro-ethanesulfonyl)-amino]-propionamide;
(±)-N-[1-(5-Benzyl-3-methyl-4-oxo-4,5-dihydro-isoxazolo[5,4-d]pyrimidin-6-yl)-propyl]-4-bromo-N-(2-carbamoyl-ethyl)-benzamide;
(±)-N-(3-Amino-propyl)-N-[1-(5-benzyl-3-methyl-4-oxo-4,5-dihydro-isoxazolo[5,4-d]pyrimidin-6-yl)-propyl]-2-methoxy-benzamide;
tert-butyl (±)-(3-{[1-(5-benzyl-3-methyl-4-oxo-4,5-dihydroisoxazolo[5,4-d]pyrimidin-6-yl)propyl][(2-methoxyphenyl)carbonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-benzamide;
tert-butyl (±)-(3-{[1-(5-benzyl-3-methyl-4-oxo-4,5-dihydroisoxazolo[5,4-d]pyrimidin-6-yl)propyl](phenylcarbonyl)amino}propyl)carbamate;
(±)-N-(3-Amino-propyl)-N-[1-(5-benzyl-3-methyl-4-oxo-4,5-dihydro-isoxazolo[5,4-d]pyrinudin-6-yl)-propyl]-3-fluoro-benzamide;
tert-butyl (±)-(3-{[1-(5-benzyl-3-methyl-4-oxo-4,5-dihydroisoxazolo[5,4-d]pyrinudin-6-yl)propyl][(3-fluorophenyl)carbonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-3,4-dichloro-benzamide;
tert-butyl (±)-(3-{[1-(5-benzyl-3-methyl-4-oxo-4,5-dihydroisoxazolo[5,4-d]pyrimidin-6-yl)propyl][(3,4-dichlorophenyl)carbonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-3-propyl-benzamide;
tert-butyl (±)-(3-{[1-(5-benzyl-3-methyl-4-oxo-4,5-dihydroisoxazolo[5,4-d]pyrimidin-6-yl)propyl] [(3-propylphenyl)carbonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-2-methyl-benzamide;
tert-butyl (±)-(3-{[1-(5-benzyl-3-methyl-4-oxo-4,5-dihydroisoxazolo[5,4-d]pyrimidin-6-yl)propyl][(2-methylphenyl)carbonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-3-methyl-benzamide;
tert-butyl (±)-(3-{[1-(5-benzyl-3-methyl-4-oxo-4,5-dihydroisoxazolo[5,4-d]pyrimidin-6-yl)propyl][(3-methylphenyl)carbonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-4-chloro-benzamide;
tert-butyl (±)-(3-{[1-(5-benzyl-3-methyl-4-oxo-4,5-dihydroisoxazolo[5,4-d]pyrimidin-6-yl)propyl][(4-chlorophenyl)carbonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-isonicotinamide;
tert-butyl (±)-(3-{[1-(5-benzyl-3-methyl-4-oxo-4,5-dihydroisoxazolo[5,4-d]pyrimidin-6-yl)propyl](pyridin-4-ylcarbonyl)amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-benzenesulfonamide;
tert-butyl (±)-(3-{[1-(5-benzyl-3-methyl-4-oxo-4,5-dihydroisoxazolo[5,4-d]pyrimidin-6-yl)propyl](phenylsulfonyl)amino}propyl)carbamate;
(±)-Biphenyl-4-carboxylic acid (3-amino-propyl)-[1-(5-benzyl-3-methyl-4-oxo-4,5-dihydro-isoxazolo[5,4-d]pyrimidin-6-yl)-propyl]-amide;
tert-butyl (±)-acetate-N-(3-aminopropyl)-N-[1-(5-benzyl-3-methyl-4-oxo-4,5-dihydroisoxazolo[5,4-d]pyrimidin-6-yl)propyl]biphenyl-4-carboxamide;
(±)-N-(3-Amino-propyl)-N-[1-(5-benzyl-3-methyl-4-oxo-4,5-dihydro-isoxazolo[5,4-d]pyrimidin-6-yl)-propyl]-4-ethyl-benzamide;
tert-butyl (±)-(3-{[1-(5-benzyl-3-methyl-4-oxo-4,5-dihydroisoxazolo[5,4-d]pyrimidin-6-yl)propyl][(4-ethylphenyl)carbonyl]amino}propyl)carbamate;
(±)-6-(1-{(3-Amino-propyl)-[2-(4-chloro-phenyl)-2-oxo-ethyl]-amino}-propyl)-5-benzyl-3-methyl-5H-isoxazolo[5,4-d]pyrimidin-4-one;
tert-butyl (±)-(3-{[1-(5-benzyl-3-methyl-4-oxo-4,5-dihydroisoxazolo[5,4-d]pyrimidin-6-yl)propyl][2-(4-chlorophenyl)-2-oxoethyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-4-chloro-benzenesulfonamide;
tert-butyl (±)-(3-{[1-(5-benzyl-3-methyl-4-oxo-4,5-dihydroisoxazolo[5,4-d]pyrimidin-6-yl)propyl][(4-chlorophenyl)sulfonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-4-tert-butyl-benzenesulfonamide;
tert-butyl (±)-(3-{[1-(5-benzyl-3-methyl-4-oxo-4,5-dihydroisoxazolo[5,4-d]pyrimidin-6-yl)propyl][(4-tert-butylphenyl)sulfonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-3-methyl-benzenesulfonamide;
tert-butyl (±)-(3-{[1-(5-benzyl-3-methyl-4-oxo-4,5-dihydroisoxazolo[5,4-d]pyrimidin-6-yl)propyl][(3-methylphenyl)sulfonyl]amino}propyl)carbamate;
(±)-Quinoline-2-carboxylic acid (3-amino-propyl)-[1-(5-benzyl-3-methyl-4-oxo-4,5-dihydro-isoxazolo[5,4-d]pyrimidin-6-yl)-propyl]-amide;
tert-butyl (±)-(3-{[1-(5-benzyl-3-methyl-4-oxo-4,5-dihydroisoxazolo[5,4-d]pyrimidin-6-yl)propyl](quinolin-2-ylcarbonyl)amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-4-methyl-benzenesulfonamide;
tert-butyl (±)-(3-{[1-(5-benzyl-3-methyl-4-oxo-4,5-dihydroisoxazolo[5,4-d]pyrimidin-6-yl)propyl][(4-methylphenyl)sulfonyl]amino}propyl)carbamate;
(±)-5-methyl-isoxazole-3-carboxylic acid (3-amino-propyl)-[1-(5-benzyl-3-methyl-4-oxo-4,5-dihydro-isoxazolo[5,4-d]pyrimidin-6-yl)-propyl]-amide;
tert-butyl (±)-(3-{[1-(5-benzyl-3-methyl-4-oxo-4,5-dihydroisoxazolo[5,4-d]pyrimidin-6-yl)propyl][(5-methylisoxazol-3-yl)carbonyl]amino}propyl)carbamate;
(±)-Cyclobutanecarboxylic acid (3-amino-propyl)-[1-(5-benzyl-3-methyl-4-oxo-4,5-dihydro-isoxazolo[5,4-d]pyrimidin-6-yl)-propyl]-amide;
tert-butyl (±)-(3-{[1-(5-benzyl-3-methyl-4-oxo-4,5-dihydroisoxazolo[5,4-d]pyrimidin-6-yl)propyl](cyclobutylcarbonyl)amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-isobutyramide;
tert-butyl (±)-(3-{[1-(5-benzyl-3-methyl-4-oxo-4,5-dihydroisoxazolo[5,4-d]pyrimidin-6-yl)propyl](2-methylpropanoyl)amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-3-methyl-butyramide;
tert-butyl (±)-(3-{[1-(5-benzyl-3-methyl-4-oxo-4,5-dihydroisoxazolo[5,4-d]pyrimidin-6-yl)propyl](3-methylbutanoyl)amino}propyl)carbamate;
(±)-N-(3-aminopropyl)-N-[1-(5-benzyl-3-methyl-4-oxo-4,5-dihydroisoxazolo[5,4-d]pyrimidin-6-yl)propyl]-4-chlorobenzenesulfonamide;
tert-butyl (±)-(3-{[1-(5-benzyl-3-methyl-4-oxo-4,5-dihydroisoxazolo[5,4-d]pyrimidin-6-yl)propyl][(4-chlorophenyl)sulfonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-2-methoxy-acetamide;
tert-butyl (±)-(3-{[1-(5-benzyl-3-methyl-4-oxo-4,5-dihydroisoxazolo[5,4-d]pyrimidin-6-yl)propyl](methoxyacetyl)amino}propyl)carbamate;
(±)-N-(3-aminopropyl)-N-[1-(5-benzyl-3-methyl-4-oxo-4,5-dihydroisoxazolo[5,4-d]pyrimidin-6-yl)propyl]-4-propylbenzamide;
tert-butyl (±)-(3-{[1-(5-benzyl-3-methyl-4-oxo-4,5-dihydroisoxazolo[5,4-d]pyrimidin-6-yl)propyl][(4-propylphenyl)carbonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-4-bromo-benzamide;
tert-butyl (±)-(3-{[1-(5-benzyl-3-methyl-4-oxo-4,5-dihydroisoxazolo[5,4-d]pyrimidin-6-yl)propyl] [(4-bromophenyl)carbonyl]amino}propyl)carbamate;
(±)-N-(3-Aminopropyl)-N-[1-(5-benzyl-3-isopropyl-4-oxo-4,5-dihydroisoxazolo[5,4-d]pyrimidin-6-yl)-propyl]-4-methylbenzamide Hydrochloride;
(±)-N-(3-Aminopropyl)-N-[1-(5-benzyl-3-isopropyl-4-oxo-4,5-dihydroisoxazolo[5,4-d]pyrimidin-6-yl)-propyl]-4-methylbenzamide;
tert-butyl (±)-[3-({1-[5-benzyl-3-(1-methylethyl)-4-oxo-4,5-dihydroisoxazolo[5,4-d]pyrimidin-6-yl]propyl}[(4-methylphenyl)carbonyl]amino)propyl]carbamate;
(±)-N-(3-Aminopropyl)-N-{1-[5-(4-chlorobenzyl)-3-isopropyl-4-oxo-4,5-dihydro-isoxazolo[5,4-d]pyrimidin-6-yl]-propyl}-4-methylbenzamide Hydrochloride;
(±)-N-(3-Aminopropyl)-N-{1-[5-(4-chlorobenzyl)-3-isopropyl-4-oxo-4,5-dihydro-isoxazolo[5,4-d]pyrimidin-6-yl]-propyl}-4-methylbenzamide;
tert-butyl (±)-[3-({1-[5-(4-chlorobenzyl)-3-(1-methylethyl)-4-oxo-4,5-dihydroisoxazolo[5,4-d]pyrimidin-6-yl]propyl}[(4-methylphenyl)carbonyl]amino)propyl]-carbamate;
(±)-N-(3-Amino-propyl)-N-[1-(5-benzyl-3-methyl-4-oxo-4,5-dihydro-isothiazolo[5,4-d]pyrimidin-6-yl)-propyl]-4-chloro-benzamide;
(±)-{3-[[1-(5-benzyl-3-methyl-4-oxo-4,5-dihydro-isothiazolo[5,4-d]pyrimidin-6-yl)-propyl]-(4-chloro-benzoyl)-amino]-propyl}-carbamic acid tert-butyl ester;
(±)-N-(3-Amino-3-methyl-butyl)-N-[1-(5-benzyl-3-methyl-4-oxo-4,5-dihydro-isothiazolo[5,4-d]pyrimidin-6-yl)-propyl]-4-chloro-benzamide; or
(±)-{3-[[1-(5-benzyl-3-methyl-4-oxo-4,5-dihydro-isothiazolo[5,4-d]pyrimidin-6-yl)-propyl]-(4-chloro-benzoyl)-amino]-1,1-dimethyl-propyl}-carbamic acid tert-butyl ester.

In a particular embodiment the present invention provides a compound or a pharmaceutically acceptable salt thereof having a structural formula (I) as recited above wherein A is C=O or CH₂.

In a particular embodiment the present invention provides a compound or a pharmaceutically acceptable salt thereof having a structural formula (I) as recited above wherein A is C=O.

In a particular embodiment the present invention provides a compound or a pharmaceutically acceptable salt thereof having a structural formula (I) as recited above wherein B is optionally substituted C₁₋₁₂alkyl or optionally substituted heterocycle.

In a particular embodiment the present invention provides a compound or a pharmaceutically acceptable salt thereof having a structural formula (I) as recited above wherein B is optionally substituted C₁₋₄alkyl.

In a particular embodiment the present invention provides a compound or a pharmaceutically acceptable salt thereof having a structural formula (I) as recited above wherein B is an optionally substituted C₁₋₄alkyl wherein such substituent is independently selected from -NH₂, -OH, -NHCH₃, -N(CH₃)₂, -NH-cyclopropane, -NH-cyclobutane, azetidine, pyrrolidine, or piperidine

In a particular embodiment the present invention provides a compound or a pharmaceutically acceptable salt thereof having a structural formula (I) as recited above wherein D is O optionally substituted with R⁸.

In a particular embodiment the present invention provides a compound or a pharmaceutically acceptable salt thereof having a structural formula (I) as recited above wherein D is N optionally substituted with one or more R⁸.

In a particular embodiment the present invention provides a compound or a pharmaceutically acceptable salt thereof having a structural formula (I) as recited above wherein R¹ and R² in combination form an optionally substituted fused isothiazole, or an optionally substituted fused isoxazole that is optionally substituted with 1 or 2 substituents wherein said substituent is selected from C₁₋₆alkyl, or halogen.

In a particular embodiment the present invention provides a compound or a pharmaceutically acceptable salt thereof having a structural formula (I) as recited above wherein R³ is optionally substituted aryl.

In a particular embodiment the present invention provides a compound or a pharmaceutically acceptable salt thereof having a structural formula (I) as recited above wherein R³ is optionally substituted C₅₋₇aryl.

In a particular embodiment the present invention provides a compound or a pharmaceutically acceptable salt thereof having a structural formula (I) as recited above wherein R³ is optionally substituted C₅₋₇aryl wherein said substituent is independently selected from C₁₋₆alkyl, F, Cl, Br, or I.

In a particular embodiment the present invention provides a compound or a pharmaceutically acceptable salt thereof having a structural formula (I) as recited above wherein R⁴ and R⁵ are H.

In a particular embodiment the present invention provides a compound or a pharmaceutically acceptable salt thereof having a structural formula (I) as recited above wherein R⁶ and R⁷ are independently selected from H, or optionally substituted C₁₋₁₂alkyl.

In a particular embodiment the present invention provides a compound or a pharmaceutically acceptable salt thereof having a structural formula (I) as recited above wherein R⁶ and R⁷ are independently selected from H, or C₁₋₆alkyl.

In a particular embodiment the present invention provides a compound or a pharmaceutically acceptable salt thereof having a structural formula (I) as recited above wherein R⁸ is independently selected from H, optionally substituted C₁₋₁₂alkyl, or optionally substituted heterocycle.

In a particular embodiment the present invention provides a compound or a pharmaceutically acceptable salt thereof having a structural formula (I) as recited above wherein R⁹ is independently selected from optionally substituted aryl or optionally substituted heterocycle.

In a particular embodiment the present invention provides a compound or a pharmaceutically acceptable salt thereof having a structural formula (I) as recited above wherein R⁹ is independently selected from aryl or heterocycle either of which is optionally substituted with 1 or 2 substituents wherein said substituent is independently selected from -C₁₋₆alkyl, -OC₁₋₆alkyl, F, Cl, Br, I.

In a particular embodiment the present invention provides a compound or a pharmaceutically acceptable salt thereof having a structural formula (I) as recited above wherein R⁹ is C₅₋₇aryl optionally substituted with 1 or 2 substituents wherein said substituent is independently selected from -C₁₋₆alkyl, -OC₁₋₆alkyl, F, Cl, Br, I.

In a particular embodiment the present invention provides a compound or a pharmaceutically acceptable salt thereof having a structural formula (I) as recited above wherein:
n is 0;
A is CO or CH₂;
B is optionally substituted C₁₋₆alkyl;
R¹ and R² in combination form a fused 5 membered heteroaryl;
R³ is optionally substituted C₅₋₇aryl;
R⁴ and R⁵ are H;
R⁶ and R⁷ are independently selected from H or optionally substituted C₁₋₁₂alkyl;
R⁹ is optionally substituted aryl or optionally substituted heterocycle.

In a particular embodiment the present invention provides a compound or a pharmaceutically acceptable salt thereof having a structural formula (I) as recited above wherein:
n is 1;
A is CO or CH₂;
B is optionally substituted C₁₋₆alkyl;
D is N or O;
R¹ and R² in combination form a fused isothiazole, isoxazole;
R³ is optionally substituted phenyl;
R⁴ and R⁵ are H;
R⁶ and R⁷ are H or optionally substituted C₁₋₁₂alkyl;
R⁸ is H or optionally substituted C₁₋₆alkyl;
R⁹ is optionally substituted phenyl.

In a particular embodiment the present invention provides a compound or a pharmaceutically acceptable salt thereof having a structural formula (I) selected from:
N-(3-Amino-propyl)-N-{1-[5-(4-fluoro-benzyl)-3-methyl-4-oxo-4,5-dihydro-isothiazolo[5,4-d]pyrimidin-6-yl]-propyl}-4-methyl-benzamide
N-(3-Amino-propyl)-N-[1-(5-benzyl-3-methyl-4-oxo-4,5-dihydro-isothiazolo[5,4-d]pyrimidin-6-yl)-propyl]-4-bromo-benzamide;
N-(3-Amino-propyl)-N-[1-(5-benzyl-3-methyl-4-oxo-4,5-dihydro-isothiazolo[5,4-d]pyrimidin-6-yl)-propyl]-3-fluoro-4-methyl-benzamide;
N-(3-Amino-propyl)-N-[1-(5-benzyl-methyl-4-oxo-4,5-dihydro-isothiazolo[5,4-d]pyrimidin-6-yl)-propyl]-2,3-dichloro-benzamide;
Naphthalene-2-carboxylic acid (3-amino-propyl)-[1-(5-benzyl-3-methyl-4-oxo-4,5-dihydro-isothiazolo[5,4-d]pyrimidin-6-yl)-propyl]-amide;
Benzo[b]thiophene-2-carboxylic acid (3-amino-propyl)-[1-(5-benzyl-3-methyl-4-oxo-4,5-dihydro-isothiazolo[5,4-d]pyrimidin-6-yl)-propyl]-amide;
N-Azetidin-3-ylmethyl-N-[1-(5-benzyl-3-methyl-4-oxo-4,5-dihydro-isothiazolo[5,4 d]pyrimidin-6-yl)-propyl]-4-methyl-benzamide;
N-[1-(5-Benzyl-3-methyl-4-oxo-4,5-dihydro-isothiazolo[5,4-d]pyrimidin-6-yl)-propyl]-4-methyl-N-piperidin-3-ylmethyl-benzamide;
N-(2-Amino-ethyl)-N-[1-(5-benzyl-3-methyl-4-oxo-4,5-dihydro-isothiazolo[5,4-d]pyrimidin-6-yl)-propyl]-4-methyl-benzamide;
N-[1-(5-Benzyl-3-methyl-4-oxo-4,5-dihydro-isothiazolo[5,4-d]pyrimidin-6-yl)-propyl]-N-(2-dimethylamino-ethyl)-4-methyl-benzamide;
N-[1-(5-Benzyl-3-methyl-4-oxo-4,5-dihydro-isothiazolo[5,4-d]pyrimidin-6-yl)-propyl]-N-(3-dimethylamino-propyl)-4-methyl-benzamide;
*N*-[1-(5-Benzyl-3-methyl-4-oxo-4,5-dihydro-isothiazolo[5,4-*d*]pyrimidin-6-yl)propyl]-N-[3-(isopropylamino)propyl]-4-methylbenzamide;
*N*-[1-(5-Benzyl-3-methyl-4-oxo-4,5-dihydro-isothiazolo[5,4-*d*]pyrimidin-6-yl)propyl]-N-[3-(cyclopropylamino)propyl]-4-methylbenzamide;
*N*-(3-Azetidin-1-ylpropyl)-N-[1-(5-benzyl-3-methyl-4-oxo-4,5-dihydro-isothiazolo[5,4-*d*]pyrimidin-6-yl)propyl]-4-methylbenzamide;
*N*-[1-(5-Benzyl-3-methyl-4-oxo-4,5-dihydro-isothiazolo[5,4-*d*]pyrimidin-6-yl)propyl]-4-methyl-N-[3-(3-pyrrolidin-1-ylpropyl) benzamide;
*N*-[1-(5-Benzyl-3-methyl-4-oxo-4,5-dihydro-isothiazolo[5,4-*d*]pyrimidin-6-yl)propyl] -4-methyl-N-[3-(methylamino) propyl] benzamide;
N-[1-(5-Benzyl-3-methyl-4-oxo-4,5-dihydro-isothiazolo[5,4-d]pyrimidin-6-yl)-propyl]-N-(3-hydroxy-propyl)-4-methyl-benzamide;
*N*-(3-Amino-propyl)-*N*-[1-(5-benzyl-3-methyl-4-oxo-4,5-dihydro-isothiazolo[5,4-*d*]pyrimidin-6-yl)-propyl]-4-methyl-benzamide;
N-(3-Amino-propyl)-N-[1-(5-benzyl-3-methyl-4-oxo-4,5-dihydro-isothiazolo[5,4-*d*]pyrimidin-6-yl)-2-methyl-propyl]-4-methyl-benzamide;
N-(3-Amino-propyl)-N-{1-[5-(4-fluoro-benzyl)-3-methyl-4-oxo-4,5-dihydro-isothiazolo[5,4-d]pyrimidin-6-yl]-2-methyl-propyl}-4-methyl-benzamide;
5-Benzyl-6-{1-[(3-hydroxy-propyl)-(4-methyl-benzyl)-amino]-propyl}-3-methyl-5H-isothiazolo[5,4-d]pyrimidin-4-one;
N-(3-Amino-propyl)-N-{1-[5-(4-fluoro-benzyl)-3-methyl-4-oxo-4,5-dihydro-isoxazolo[5,4-d]pyrimidin-6-yl]-2-methyl-propyl}-4-methyl-benzamide;
N-(3-Amino-propyl)-3-fluoro-N-{1-[5-(4-fluoro-benzyl)-3-methyl-4-oxo-4,5-dihydroisoxazolo[5,4-d]pyrimidin-6-yl]-2-methyl-propyl}-4-methyl-benzamide;
N-(3-Amino-propyl)-N-[1-(5-benzyl-3-methyl-4-oxo-4,5-dihydro-isoxazolo[5,4-d]pyrimidin-6-yl)-2-methyl-propyl]-3-fluoro-4-methyl-benzamide;
N-(3-Amino-propyl)-N-[1-(5-benzyl-3-methyl-4-oxo-4,5-dihydro-isoxazolo[5,4-d]pyrimidin-6-yl)-2-methyl-propyl]-4-methyl-benzamide;
N-(3-Amino-propyl)-N-[1-(5-benzyl-3-methyl-4-oxo-4,5-dihydro-isoxazolo[5,4-d]pyrimidin-6-yl)-propyl]-4-fluoro-benzamide;
N-(3-Amino-propyl)-N-[1-(5-benzyl-3-methyl-4-oxo-4,5-dihydro-isoxazolo[5,4-d]pyrimidin-6-yl)-propyl]-2,3-dichloro-benzamide;
N-(3-Amino-propyl)-N-[1-(5-benzyl-3-methyl-4-oxo-4,5-dihydro-isoxazolo[5,4-d]pyrimidin-6-yl)-propyl]-3-fluoro-4-methyl-benzamide;
N-(3-Amino-propyl)-N-[1-(5-benzyl-3-methyl-4-oxo-4,5-dihydro-isoxazolo[5,4-d]pyrimidin-6-yl)-propyl]-4-methoxy-benzamide.

In a particular embodiment the present invention provides a compound or a pharmaceutically acceptable salt thereof according to any one of claims 1 to 21, for use as a medicament.

In a particular embodiment the present invention provides the use of a compound or a pharmaceutically acceptable salt thereof as defined in any one of claims 1 to 21, in the manufacture of a medicament for the treatment or prophylaxis of disorders associated with cancer.

In a particular embodiment the present invention provides a pharmaceutical composition comprising a compound or a pharmaceutically acceptable salt thereof as defined in any one of claims 1 to 21, or a pharmaceutically acceptable salt thereof, together with at least one pharmaceutically acceptable carrier, diluent or excipient.

In a particular embodiment the present invention provides a process for preparing a compound of structural formula (I) as claimed in claim 1 or a pharmaceutically acceptable salt thereof which process comprises:

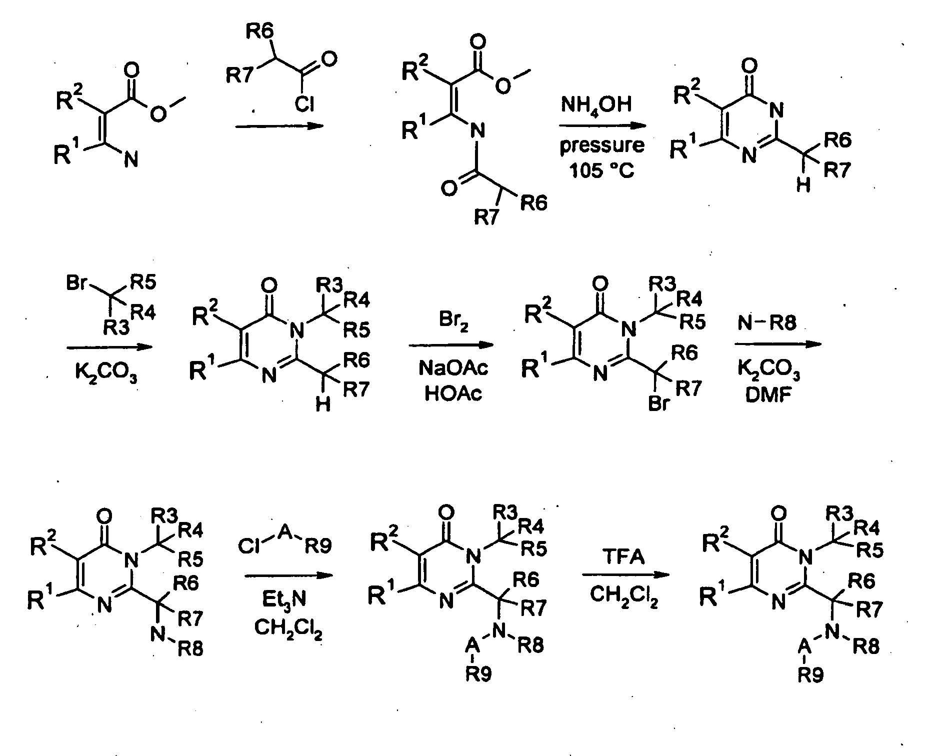

### Definitions

The definitions set forth in this section are intended to clarify terms used throughout this application. The term "herein" means the entire application.

Unless specified otherwise within this specification, the nomenclature used in this specification generally follows the examples and rules stated in Nomenclature of Organic Chemistry, Sections A, B, C, D, E, F, and H, Pergamon Press, Oxford, 1979, which is incorporated by references herein for its exemplary chemical structure names and rules on naming chemical structures.

The term "Cₘ₋ₙ" or "Cₘ₋ₙ group" used alone or as a prefix, refers to any group having m to n carbon atoms. For example C₁₋₆ means 1,2,3,4,5,or 6 carbon atoms.

The term "hydrocarbon" used alone or as a suffix or prefix, refers to any structure comprising only carbon and hydrogen atoms up to 14 carbon atoms.

The term "hydrocarbon radical" or "hydrocarbyl" used alone or as a suffix or prefix, refers to any structure as a result of removing one or more hydrogens from a hydrocarbon.

The term "alkyl" used alone or as a suffix or prefix, refers to monovalent straight or branched chain hydrocarbon radicals comprising 1 to about 12 carbon atoms. Unless otherwise specified, "alkyl" generally includes both saturated alkyl and unsaturated alkyl.

The term "alkylene" used alone or as suffix or prefix, refers to divalent straight or branched chain hydrocarbon radicals comprising 1 to about 12 carbon atoms, which serves to links two structures together.

The term "alkenyl" used alone or as suffix or prefix, refers to a monovalent straight or branched chain hydrocarbon radical having at least one carbon-carbon double bond and comprising at least 2 up to about 12 carbon atoms.

The term "alkynyl" used alone or as suffix or prefix, refers to a monovalent straight or branched chain hydrocarbon radical having at least one carbon-carbon triple bond and comprising at least 2 up to about 12 carbon atoms.

The term "cycloalkyl," used alone or as suffix or prefix, refers to a monovalent ring-containing hydrocarbon radical comprising at least 3 up to about 12 carbon atoms.

The term "cycloalkenyl" used alone or as suffix or prefix, refers to a monovalent ring-containing hydrocarbon radical having at least one carbon-carbon double bond and comprising at least 3 up to about 12 carbon atoms.

The term "cycloalkynyl" used alone or as suffix or prefix, refers to a monovalent ring-containing hydrocarbon radical having at least one carbon-carbon triple bond and comprising about 7 up to about 12 carbon atoms.

The term "aryl" used alone or as suffix or prefix, refers to a hydrocarbon radical having one or more polyunsaturated carbon rings having aromatic character, (*e*.*g*., 4n + 2 delocalized electrons) and comprising 5 up to about 14 carbon atoms, wherein the radical is located on a carbon of the aromatic ring.

The term "non-aromatic group" or "non-aromatic" used alone, as suffix or as prefix, refers to a chemical group or radical that does not contain a ring having aromatic character (*e*.*g*., 4n + 2 delocalized electrons).

The term "arylene" used alone or as suffix or prefix, refers to a divalent hydrocarbon radical having one or more polyunsaturated carbon rings having aromatic character, (*e*.*g*., 4n + 2 delocalized electrons) and comprising 5 up to about 14 carbon atoms, which serves to link two structures together.

The term "heterocycle" used alone or as a suffix or prefix, refers to a ring-containing structure or molecule having one or more multivalent heteroatoms, independently selected from N, O, P and S, as a part of the ring structure and including at least 3 and up to about 20 atoms in the ring(s). Heterocycle may be saturated or unsaturated, containing one or more double bonds, and heterocycle may contain more than one ring. When a heterocycle contains more than one ring, the rings may be fused or unfused. Fused rings generally refer to at least two rings share two atoms therebetween. Heterocycle may have aromatic character or may not have aromatic character.

The term "heteroalkyl" used alone or as a suffix or prefix, refers to a radical formed as a result of replacing one or more carbon atom of an alkyl with one or more heteroatoms selected from N, O, P and S.

The term "heteroaromatic" used alone or as a suffix or prefix, refers to a ring-containing structure or molecule having one or more multivalent heteroatoms, independently selected from N, O, P and S, as a part of the ring structure and including at least 3 and up to about 20 atoms in the ring(s), wherein the ring-containing structure or molecule has an aromatic character (*e*.*g*., 4n + 2 delocalized electrons).

The term "heterocyclic group," "heterocyclic moiety," "heterocyclic," or "heterocyclo" used alone or as a suffix or prefix, refers to a radical derived from a heterocycle by removing one or more hydrogens therefrom.

The term "heterocycle" used alone or as a suffix or prefix, refers a radical derived from a heterocycle by removing one hydrogen from a carbon of a ring of the heterocycle.

The term "heterocyclene" used alone or as a suffix or prefix, refers to a divalent radical derived from a heterocycle by removing two hydrogens therefrom, which serves to links two structures together.

The term "heteroaryl" used alone or as a suffix or prefix, refers to a heterocycle having aromatic character, wherein the radical of the heterocycle is located on a carbon of an aromatic ring of the heterocycle.

The term "heterocycloalkyl" used alone or as a suffix or prefix, refers to a heterocycle that does not have aromatic character.

The term "heteroarylene" used alone or as a suffix or prefix, refers to a heterocyclylene having aromatic character.

The term "heterocycloalkylene" used alone or as a suffix or prefix, refers to a heterocyclylene that does not have aromatic character.

The term "six-membered" used as prefix refers to a group having a ring that contains six ring atoms.

The term "five-membered" used as prefix refers to a group having a ring that contains five ring atoms.

A five-membered ring heteroaryl is a heteroaryl with a ring having five ring atoms wherein 1, 2 or 3 ring atoms are independently selected from N, O and S.

Exemplary five-membered ring heteroaryls are thienyl, furyl, pyrrolyl, imidazolyl, thiazolyl, oxazolyl, pyrazolyl; isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, and 1,3,4-oxadiazolyl.

A six-membered ring heteroaryl is a heteroaryl with a ring having six ring atoms wherein 1, 2 or 3 ring atoms are independently selected from N, O and S.

Exemplary six-membered ring heteroaryls are pyridyl, pyrazinyl, pyrimidinyl, triazinyl and pyridazinyl.

As used in this application, the term "optionally substituted," as used herein, means that substitution is optional and therefore it is possible for the designated atom or molecule to be unsubstituted. In the event a substitution is desired then such substitution means that any number of hydrogens on the designated atom is replaced with a selection from the indicated group, provided that the normal valency of the designated atom is not exceeded, and that the substitution results in a stable compound. For example when a substituent is keto (i.e., =O), then 2 hydrogens on the atom are replaced. If no selection is provided then the substituent shall be selected from: -OC₁₋₆alkyl, -C₁₋₆alkyl, F, Cl, Br, I, =O, =S, -NH₂, -OH, -NHCH₃, -N(CH₃)₂, -NH-cyclopropane, -NH-cyclobutane, azetidine, pyrrolidine, piperidine. Exemplary chemical groups containing one or more heteroatoms include heterocycle, -NO₂, -OR, -CF₃, -C(=O)R, -C(=O)OH, -SH, -NHR, -NR₂, -SR, -SO₃H, -SO₂R, -S(=O)R, -CN, -C(=O)OR, -C(=O)NR₂, -NRC(=O)R, oxo (=O), imino (=NR), thio (=S), and oximino (=N-OR), wherein each "R" is a C₁₋₁₂hydrocarbyl. For example, substituted phenyl may refer to nitrophenyl, pyridylphenyl, methoxyphenyl, chlorophenyl, aminophenyl, etc., wherein the nitro, pyridyl, methoxy, chloro, and amino groups may replace any suitable hydrogen on the phenyl ring.

The term "substituted" used as a suffix of a first structure, molecule or group, followed by one or more names of chemical groups refers to a second structure, molecule or group, which is a result of replacing one or more hydrogens of the first structure, molecule or group with the one or more named chemical groups. For example, a "phenyl substituted by nitro" refers to nitrophenyl.

Heterocycle includes, for example, monocyclic heterocycles such as: aziridine, oxirane, thiirane, azetidine, oxetane, thietane, pyrrolidine, pyrroline, imidazolidine, pyrazolidine, pyrazoline, dioxolane, sulfolane 2,3-dihydrofuran, 2,5-dihydrofuran tetrahydrofuran, thiophane, piperidine, 1,2,3,6-tetrahydro-pyridine, piperazine, morpholine, thiomorpholine, pyran, thiopyran, 2,3-dihydropyran, tetrahydropyran, 1,4-dihydropyridine, 1,4-dioxane, 1,3-dioxane, dioxane, homopiperidine, 2,3,4,7-tetrahydro-1*H*-azepine homopiperazine, 1,3-dioxepane, 4,7-dihydro-1,3-dioxepin, and hexamethylene oxide.

In addition, heterocycle includes aromatic heterocycles, for example, pyridine, pyrazine, pyrimidine, pyridazine, thiophene, furan, furazan, pyrrole, imidazole, thiazole, oxazole, pyrazole, isothiazole, isoxazole, 1,2,3-triazole, tetrazole, 1,2,3-thiadiazole, 1,2,3-oxadiazole, 1,2,4-triazole, 1,2,4-thiadiazole, 1,2,4-oxadiazole, 1,3,4-triazole, 1,3,4-thiadiazole, and 1,3,4- oxadiazole.

Additionally, heterocycle encompass polycyclic heterocycles, for example, indole, indoline, isoindoline, quinoline, tetrahydroquinoline, isoquinoline, tetrahydroisoquinoline, 1,4-benzodioxan, coumarin, dihydrocoumarin, benzofuran, 2,3-dihydrobenzofuran, isobenzofuran, chromene, chroman, isochroman, xanthene, phenoxathiin, thianthrene, indolizine, isoindole, indazole, purine, phthalazine, naphthyridine, quinoxaline, quinazoline, cinnoline, pteridine, phenanthridine, perimidine, phenanthroline, phenazine, phenothiazine, phenoxazine, 1,2-benzisoxazole, benzothiophene, benzoxazole, benzthiazole, benzimidazole, benztriazole, thioxanthine, carbazole, carboline, acridine, pyrolizidine, and quinolizidine.

In addition to the polycyclic heterocycles described above, heterocycle includes polycyclic heterocycles wherein the ring fusion between two or more rings includes more than one bond common to both rings and more than two atoms common to both rings. Examples of such bridged heterocycles include quinuclidine, diazabicyclo[2.2.1]heptane and 7-oxabicyclo[2.2.1]heptane.

Heterocycle includes, for example, monocyclic heterocycles, such as: aziridinyl, oxiranyl, thiiranyl, azetidinyl, oxetanyl, thietanyl, pyrrolidinyl, pyrrolinyl, imidazolidinyl, pyrazolidinyl, pyrazolinyl, dioxolanyl, sulfolanyl, 2,3-dihydrofuranyl, 2,5-dihydrofuranyl, tetrahydrofuranyl, thiophanyl, piperidinyl, 1,2,3,6-tetrahydro-pyridinyl, piperazinyl, morpholinyl, thiomorpholinyl, pyranyl, thiopyranyl, 2,3-dihydropyranyl, tetrahydropyranyl, 1,4-dihydropyridinyl, 1,4-dioxanyl, 1,3-dioxanyl, dioxanyl, homopiperidinyl, 2,3,4,7-tetrahydro-1*H-*azepinyl, homopiperazinyl, 1,3-dioxepanyl, 4,7-dihydro-1,3-dioxepinyl, and hexamethylene oxidyl.

In addition, heterocycle includes aromatic heterocycles or heteroaryl, for example, pyridinyl, pyrazinyl, pyrimidinyl, pyridazinyl, thienyl, furyl, furazanyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, and 1,3,4 oxadiazolyl.

Additionally, heterocycle encompasses polycyclic heterocycles (including both aromatic or non-aromatic), for example, indolyl, indolinyl, isoindolinyl, quinolinyl, tetrahydroquinolinyl, isoquinolinyl, tetrahydroisoquinolinyl, 1,4-benzodioxanyl, coumarinyl, dihydrocoumarinyl, benzofuranyl, 2,3-dihydrobenzofuranyl, isobenzofuranyl, chromenyl, chromanyl, isochromanyl, xanthenyl, phenoxathiinyl, thianthrenyl, indolizinyl, isoindolyl, indazolyl, purinyl, phthalazinyl, naphthyridinyl, quinoxalinyl, quinazolinyl, cinnolinyl, pteridinyl, phenanthridinyl, perimidinyl, phenanthrolinyl, phenazinyl, phenothiazinyl, phenoxazinyl, 1,2-benzisoxazolyl, benzothiophenyl, benzoxazolyl, benzthiazolyl, benzimidazolyl, benztriazolyl, thioxanthinyl, carbazolyl, carbolinyl, acridinyl, pyrolizidinyl, and quinolizidinyl.

In addition to the polycyclic heterocycles described above, heterocycle includes polycyclic heterocycles wherein the ring fusion between two or more rings includes more than one bond common to both rings and more than two atoms common to both rings. Examples of such bridged heterocycles include quinuclidinyl, diazabicyclo[2.2.1]heptyl; and 7-oxabicyclo[2.2.1]heptyl.

The term "alkoxy" used alone or as a suffix or prefix, refers to radicals of the general formula -O-R, wherein -R is selected from a hydrocarbon radical. Exemplary alkoxy includes methoxy, ethoxy, propoxy, isopropoxy, butoxy, t-butoxy, isobutoxy, cyclopropylmethoxy, allyloxy, and propargyloxy.

The term "aryloxy" used alone or as suffix or prefix, refers to radicals of the general formula -O-Ar, wherein -Ar is an aryl.

The term "heteroaryloxy" used alone or as suffix or prefix, refers to radicals of the general formula -O-Ar', wherein -Ar' is a heteroaryl.

The term "amine" or "amino" used alone or as a suffix or prefix, refers to radicals of the general formula NRR', wherein R and R' are independently selected from hydrogen or a hydrocarbon radical.

"Acyl" used alone, as a prefix or suffix, means -C(=O)-R, wherein -R is an optionally substituted hydrocarbyl, hydrogen, amino or alkoxy. Acyl groups include, for example, acetyl, propionyl, benzoyl, phenyl acetyl, carboethoxy, and dimethylcarbamoyl.

Halogen includes fluorine, chlorine, bromine and iodine.

"Halogenated," used as a prefix of a group, means one or more hydrogens on the group is replaced with one or more halogens.

"RT" or "rt" means room temperature.

A first ring group being "fused" with a second ring group means the first ring and the second ring share at least two atoms therebetween.

"Link," "linked," or "linking," unless otherwise specified, means covalently linked or bonded.

When a first group, structure, or atom is "directly connected" to a second group, structure or atom, at least one atom of the first group, structure or atom forms a chemical bond with at least one atom of the second group, structure or atom.

"Saturated carbon" means a carbon atom in a structure, molecule or group wherein all the bonds connected to this carbon atom are single bond. In other words, there is no double or triple bonds connected to this carbon atom and this carbon atom generally adopts an *sp³* atomic orbital hybridization.

"Unsaturated carbon" means a carbon atom in a structure, molecule or group wherein at least one bond connected to this carbon atom is not a single bond. In other words, there is at least one double or triple bond connected to this carbon atom and this carbon atom generally adopts a *sp* or *sp²* atomic orbital hybridization.

When any variable (e.g., R¹, R⁴, R^{a}, R^{c} etc.) occurs more than one time in any constituent or formula for a compound, its definition at each occurrence is independent of its definition at every other occurrence. Thus, for example, if a group is shown to be substituted with 0-3 R¹, then said group may optionally be substituted with 0,1,2 or 3 R¹ groups and R^{e} at each occurrence is selected independently from the definition of R^{e}. Also, combinations of substituents and/or variables are permissible only if such combinations result in stable compounds.

A variety of compounds in the present invention may exist in particular geometric or stereoisomeric forms. The present invention takes into account all such compounds, including cis- and trans isomers, R- and S- enantiomers, diastereomers, (D)-isomers, (L)-isomers, the racemic mixtures thereof, and other mixtures thereof, as being covered within the scope of this invention. Additional asymmetric carbon atoms may be present in a substituent such as an alkyl group. All such isomers, as well as mixtures thereof, are intended to be included in this invention. The compounds herein described may have asymmetric centers. Compounds of the present invention containing an asymmetrically substituted atom may be isolated in optically active or racemic forms. It is well known in the art how to prepare optically active forms, such as by resolution of racemic forms or by synthesis from optically active starting materials. When required, separation of the racemic material can be achieved by methods known in the art. Many geometric isomers of olefins, C=N double bonds, and the like can also be present in the compounds described herein, and all such stable isomers are contemplated in the present invention. Cis and trans geometric isomers of the compounds of the present invention are described and may be isolated as a mixture of isomers or as separated isomeric forms. All chiral, diastereomeric, racemic forms and all geometric isomeric forms of a structure are intended, unless the specific stereochemistry or isomeric form is specifically indicated.

When a bond to a substituent is shown to cross a bond connecting two atoms in a ring, then such substituent may be bonded to any atom on the ring. When a substituent is listed without indicating the atom via which such substituent is bonded to the rest of the compound of a given formula, then such substituent may be bonded via any atom in such substituent. Combinations of substituents and/or variables are permissible only if such combinations result in stable compounds.

As used herein, "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, maleic, tartaric, citric, ascorbic, palmitic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, Pa., 1985, p. 1418, the disclosure of which is hereby incorporated by reference.

### Formulations

Compounds of the present invention may be administered orally, parenteral, buccal, vaginal, rectal, inhalation, insufflation, sublingually, intramuscularly, subcutaneously, topically, intranasally, intraperitoneally, intrathoracially, intravenously, epidurally, intrathecally, intracerebroventricularly and by injection into the joints.

The dosage will depend on the route of administration, the severity of the disease, age and weight of the patient and other factors normally considered by the attending physician, when determining the individual regimen and dosage level as the most appropriate for a particular patient.

An effective amount of a compound of the present invention for use in therapy of infection is an amount sufficient to symptomatically relieve in a warm-blooded animal, particularly a human the symptoms of infection, to slow the progression of infection, or to reduce in patients with symptoms of infection the risk of getting worse.

For preparing pharmaceutical compositions from the compounds of this invention, inert, pharmaceutically acceptable carriers can be either solid or liquid. Solid form preparations include powders, tablets, dispersible granules, capsules, cachets, and suppositories.

A solid carrier can be one or more substances, which may also act as diluents, flavoring agents, solubilizers, lubricants, suspending agents, binders, or tablet disintegrating agents; it can also be an encapsulating material.

In powders, the carrier is a finely divided solid, which is in a mixture with the finely divided active component. In tablets, the active component is mixed with the carrier having the necessary binding properties in suitable proportions and compacted in the shape and size desired.

For preparing suppository compositions, a low-melting wax such as a mixture of fatty acid glycerides and cocoa butter is first melted and the active ingredient is dispersed therein by, for example, stirring. The molten homogeneous mixture is then poured into convenient sized molds and allowed to cool and solidify.

Suitable carriers include magnesium carbonate, magnesium stearate, talc, lactose, sugar, pectin, dextrin, starch, tragacanth, methyl cellulose, sodium carboxymethyl cellulose, a low-melting wax, cocoa butter, and the like.

Some of the compounds of the present invention are capable of forming salts with various inorganic and organic acids and bases and such salts are also within the scope of this invention. Examples of such acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate. Base salts include ammonium salts, alkali metal salts such as sodium, lithium and potassium salts, alkaline earth metal salts such as aluminum, calcium and magnesium salts, salts with organic bases such as dicyclohexylamine salts, N-methyl-D-glucamine, and salts with amino acids such as arginine, lysine, ornithine, and so forth. Also, basic nitrogen-containing groups may be quatemized with such agents as: lower alkyl halides, such as methyl, ethyl, propyl, and butyl halides; dialkyl sulfates like dimethyl, diethyl, dibutyl; diamyl sulfates; long chain halides such as decyl, lauryl, myristyl and stearyl halides; aralkyl halides like benzyl bromide and others. Non-toxic physiologically-acceptable salts are preferred, although other salts are also useful, such as in isolating or purifying the product.

The salts may be formed by conventional means, such as by reacting the free base form of the product with one or more equivalents of the appropriate acid in a solvent or medium in which the salt is insoluble, or in a solvent such as water, which is removed *in vacuo* or by freeze drying or by exchanging the anions of an existing salt for another anion on a suitable ion-exchange resin.

In order to use a compound of the formula (1) or a pharmaceutically acceptable salt thereof for the therapeutic treatment (including prophylactic treatment) of mammals including humans, it is normally formulated in accordance with standard pharmaceutical practice as a pharmaceutical composition.

In addition to the compounds of the present invention, the pharmaceutical composition of this invention may also contain, or be co-administered (simultaneously or sequentially) with, one or more pharmacological agents of value in treating one or more disease conditions referred to herein.

The term composition is intended to include the formulation of the active component or a pharmaceutically acceptable salt with a pharmaceutically acceptable carrier. For example this invention may be formulated by means known in the art into the form of, for example, tablets, capsules, aqueous or oily solutions, suspensions, emulsions, creams, ointments, gels, nasal sprays, suppositories, finely divided powders or aerosols or nebulisers for inhalation, and for parenteral use (including intravenous, intramuscular or infusion) sterile aqueous or oily solutions or suspensions or sterile emulsions.

Liquid form compositions include solutions, suspensions, and emulsions. Sterile water or water-propylene glycol solutions of the active compounds may be mentioned as an example of liquid preparations suitable for parenteral administration. Liquid compositions can also be formulated in solution in aqueous polyethylene glycol solution. Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.

The pharmaceutical compositions can be in unit dosage form. In such form, the composition is divided into unit doses containing appropriate quantities of the active component The unit dosage form can be a packaged preparation, the package containing discrete quantities of the preparations, for example, packeted tablets, capsules, and powders in vials or ampoules. The unit dosage form can also be a capsule, cachet, or tablet itself, or it can be the appropriate number of any of these packaged forms.

### Combinations

The anti-cancer treatment defined herein may be applied as a sole therapy or may involve, in addition to the compound of the invention, conventional surgery or radiotherapy or chemotherapy. Such chemotherapy may include one or more of the following categories of antitumour agents:
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside and hydroxyurea); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-erbb2 antibody trastuzumab [Herceptin^{™}] and the anti-erbb1 antibody cetuximab [C225]), farnesyl transferase inhibitors, tyrosine kinase inhibitors and serine/threonine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab [Avastin^{™}], compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as Combretastatin A4 and compounds disclosed in International Patent Applications WO 99/02166, WO 00/40529, WO 00/41669, WO 01/92224, WO 02/04434 and WO 02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

### Synthesis

The compounds of the present invention can be prepared in a number of ways well known to one skilled in the art of organic synthesis. The compounds of the present invention can be synthesized using the methods described below, together with synthetic methods known in the art of synthetic organic chemistry, or variations thereon as appreciated by those skilled in the art. Such methods include, but are not limited to, those described below. All references cited herein are hereby incorporated in their entirety by reference.

The novel compounds of this invention may be prepared using the reactions and techniques described herein. The reactions are performed in solvents appropriate to the reagents and materials employed and are suitable for the transformations being effected. Also, in the description of the synthetic methods described below, it is to be understood that all proposed reaction conditions, including choice of solvent, reaction atmosphere, reaction temperature, duration of the experiment and workup procedures, are chosen to be the conditions standard for that reaction, which should be readily recognized by one skilled in the art. It is understood by one skilled in the art of organic synthesis that the functionality present on various portions of the molecule must be compatible with the reagents and reactions proposed. Such restrictions to the substituents, which are compatible with the reaction conditions, will be readily apparent to one skilled in the art and alternate methods must then be used.

The starting materials for the Examples contained herein are either commercially available or are readily prepared by standard methods from known materials. For example the following reactions are illustrations but not limitations of the preparation of some of the starting materials and examples used herein.

Chemical abbreviations used in the Examples are defined as follows: "BOC" denotes N-tert-butoxycarbonyl, "CBZ" denotes carbobenzyloxy; "DIEA" denotes N,N-diisopropylethylamine, "DMF" denotes N, N-dimethylformamide; "THF" denotes tetrahydrofuran, "ether" denotes ethyl ether, "min." denotes minutes; "h" or hr denotes hours; "RT" or "r.t". denotes room temperature; "SM" denotes starting material, "MS" denotes mass spectrometry, "RM" denotes reaction mixture, "NMR" denotes nuclear magnetic resonance, "TLC" denotes thin layer chromatography, "LC" denotes liquid chromatography, "HPLC" denotes high pressure liquid chromatography, "TFA" denotes trifluoroacetic acid, "DMSO" denotes dimethyl sulfoxide, "EtOAc" denotes ethyl acetate. Unless otherwise noted, organic solutions were "dried" over anhydrous sodium sulfate. Examples of such processes are illustrated below:

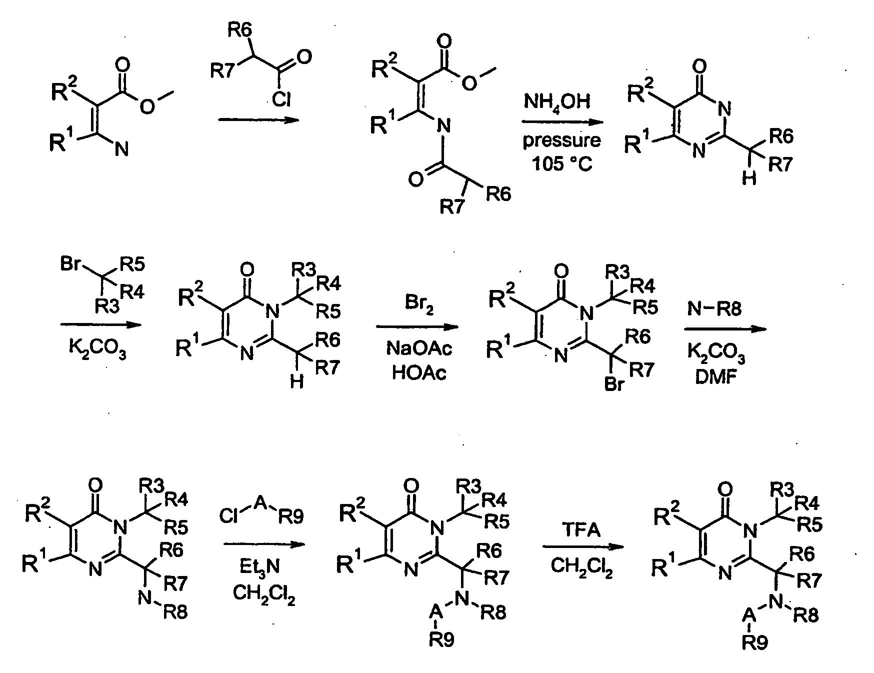

### METHOD 1

### 2-(1-Ethoxy-ethylidene)-malononitrile

Triethyl orthoacetate (97 g, 0.6 mol), malononitrile (33 g, 0.5 mol) and glacial acetic acid (1.5 g) were placed in a 1 L flask equipped with a stirrer, thermometer and a Vigreux column (20 x 1 in.) on top of which a distillation condenser was placed. The reaction mixture was heated and ethyl alcohol began to distill when the temperature of the reaction mixture was about 85-90 °C. After about 40 min., the temperature of the reaction mixture reached 140 °C. Then the reaction was concentrated in a rotary evaporator to remove the low-boiling materials and the residue was crystallized from absolute alcohol to yield the pure product (62.2 g, 91 %) as a light yellow solid [mp 91.6 °C (lit.90-92 °C, MCCall. M. A. J. Org. Chem. 1962, 27, 2433-2439.)].

### METHOD 2

### (E)-2-Cyano-3-ethoxy-but-2-enethioic acid amide

2-(1-Ethoxy-ethylidene)-malononitrile (method 1) (62 g, 0.45 mol) was dissolved in anhydrous benzene (800 mL) and 1 mL of triethylamine was added as catalyst. The mixture was stirred and hydrogen sulfide was bubbled into this solution for 40 min and a solid formed. The precipitated solid was filtered off and dried. The solid was recrystallized from absolute alcohol (100 mL) filtered and dried to isolate the pure (E)-2-cyano-3-ethoxy-but-2-enethioic acid amide (19.3 g, 25%) as a light brown crystals.

### METHOD 3

### (E)-3-Amino-2-cyano-but-2-enethioic acid amide

(E)-2-Cyano-3-ethoxy-but-2-enethioic acid amide (method 2) (19.2 g, 0.136 mol) was dissolved in a saturated solution of ammonia in methanol (500 mL) and stirred at r.t. overnight. The reaction mixture was concentrated and the residue was dissolved in hot water (600 mL) and the undissoved solid was filtered and dried to recover 6 g of the starting thiocrotonamide. The aqueous solution on standing overnight provided the pure (E)-3-amino-2-cyano-but-2-enethioic acid amide (6.85 g, 63%) as off-white crystals. 1H NMR (300 MHz, DMSO-*d*₆) δ 2.22 (s, 3H), 7.73 (bs, 1H), 8.53 (bs, 1H), 9.01 (bs, 1H), 11.60 (bs, 1H).

### METHOD 4

### 5-Amino-3-methylisothiazole-4-carbonitrile

To a stirred solution of (E)-3-amino-2-cyano-but-2-enethioic acid amide (method 3) (6.83 g, 48.4 mmol) in methanol (300 mL) was added dropwise 13.6 mL (124 mmoL) of 30% hydrogen peroxide. The mixture was stirred at 60 °C for 4 h and evaporated to 60 mL in a rotary evaporator and cooled in an ice-bath. The crystallized product was filtered off and recrystallized from ethyl acetate to provide the pure product 5-amino-3-methylisothiazole-4-carbonitrile (5.41 g, 80%) as a white crystalline solid. 1H NMR (300 MHz, DMSO-*d*₆) δ 2.24 (s, 3H), 8.00 (bs, 2H).

### METHOD 5

### N-(4-Cyano-3-methyl-isothiazol-5-yl)-butyramide

To a solution of the amine (method 4) (5.31 g, 38.2 mmol) in CH₂Cl₂ (200 mL) at 0 °C, NEt₃ (5 g, 50 mmol) was added followed by the dropwise addition of a solution of the butyryl chloride (4.88 g, 45.8 mmol) in CH₂Cl₂ (50 mL). After the completion of the addition the reaction mixture was allowed to warm to r.t. and stirred overnight. The reaction mixture was washed with water (100 mL), 1N HCl (100 mL), brine (200 mL) and dried over Na₂SO₄. Concentration of the CH₂Cl₂ layer provided the product which was triturated from CH₂Cl₂/hexanes (1/10) and filtered off to isolate the pure N-(4-cyano-3-methyl-isothiazol-5-yl)-butyramide (7.57 g, 95%) as an orange solid.

### METHOD 6

### 5-Butyrylamino-3-methyl-isothiazole-4-carboxylic acid amide

To a solution of N-(4-cyano-3-methyl-isothiazol-5-yl)-butyramide (method 5) (4.18 g, 20 mmol) in 30% aqueous NH₄OH (250 mL), was added dropwise 100 mL of hydrogen peroxide at r.t. After the completion of the addition the reaction mixture was stirred at 60 °C overnight after which the TLC showed the complete disappearance of SM. The reaction mixture was cooled and extracted with chloroform (3 x 100 mL). The organic layer was dried (Na₂SO₄) and concentrated to get the pure 5-butyrylamino-3-methyl-isothiazole-4-carboxylic acid amide (2.9 g, 72%) as a white solid. 1H NMR (300 MHz, CDCl₃) δ 1.03 (t, 3H), 1.79 (m, 2H), 2.54 (t, 3H), 2.69 (s, 3H), 5.97 (bs, 2H), 11.78 (bs, 1H).

### METHOD 7

### 3-Methyl-6-propyl-5H-isothiazolo[5,4-d]pyrimidin-4-one

5-Butyrylamino-3-methyl-isothiazole-4-carboxylic acid amide (method 6) (1.9 g, 8.3 mmol) was suspended in 75 mL of 30% NH₃ and then was heated to 140 °C for 4h in a pressure reactor. The mixture was cooled and neutralized to pH 8. The precipitated 3-methyl-6-propyl-5*H-*isothiazolo[5,4-*d*]pyrimidin-4-one was filtered off, washed with water (100 mL) and dried in vacuum oven at 40 °C overnight to get 800 mg (34%) of pure product. ¹H NMR (300MHz, CDCl₃) δ 1.03 (t, 3H), 1.74 (m, 2H), 2.67 (t, 3H), 2.78 (s, 3H).

### METHOD 8

### 5-Benzyl-3-methyl-6-propyl-5H-isothiazolo[5,4-d]pyrimidin-4-one

To a solution of 3-methyl-6-propyl-5*H*-isothiazolo[5,4-*d*]pyrimidin-4-one (method 7) (800 mg, 3.8 mmol) in 20 mL of anhydrous DMF was added 1.38 g (10 mmol) of anhydrous K₂CO₃ followed by benzyl bromide (655 mg, 3.8 mmol) and the mixture was stirred at room temperature overnight. The TLC of the reaction mixture showed the complete disappearance of the SM. The reaction mixture was poured into ice cold water and extracted with EtOAc (3X100 mL). The combined extracts were washed with water (100 mL), brine (100 mL), dried (Na₂SO₄) and concentrated. The TLC and the 1H NMR showed the presence of two products N alkylated as well as *O*-alkylated products in a ratio of 1:1. The products were separated by column (silica gel, 116 g) chromatography using 10-20% EtOAc in hexanes. The desired *N*-alkylated product 5-benzyl-3-methyl-6-propyl-5H-isothiazolo[5,4-d]pyrimidin-4-one was isolated as white crystalline solid (369 mg, 32%). 1H NMR (300 MHz, CDCl₃) δ 0.96 (t, 3H), 1.71-1.84 (m, 2H), 2.73 (t, 3H), 2.81 (s, 3H), 5.38 (s, 2H), 7.14-7.38 (m, 5H):

The following compounds were synthesized according to Method 8:

| **Method #** | **Compound Name** | ***m*/*z*** | **Alkylating agent** |
|---|---|---|---|
| **8a** | 5-(4-Fluoro-benzyl)-3-methyl-6-propyl-isothiazolo[5,4-*d*]pyimidin-4-one | 318 (MH⁺) | 4-fluorobenzyl bromide |

### METHOD 9

### 5-Benzyl-6-(1-bromo-propyl)-3-methyl-5-H-isothiazolo[5,4-d]pyrimidin-4-one

To a solution of 5-benzyl-3-methyl-6-propyl-5H-isothiazolo[5,4-d]pyrimidin-4-one (method 8) (369 mg, 1.23 mmol) and sodium acetate (1 g) in acetic acid (5 mL) at 100 °C, a solution of the bromine (318 mg, 2 mmol) in acetic acid (10 mL) was added dropwise [The next drop of bromine was added only after the previous drop had reacted completely by monitoring the decolorization] over a period of 20 minutes. The reaction mixture was cooled after the addition and the TLC (eluent 10% EtOAc in hexanes) and MS showed the complete disappearance of the SM and only the product. The reaction mixture was poured into ice water and extracted with EtOAc (3 X 60 mL) and the organic layers were combined and washed with 2% sodium thiosulfate solution (60 mL), water (100 mL), brine (100 mL) and dried over Na₂SO₄. Concentration of the organic layer provided the pure 5-benzyl-6-(1-bromo-propyl)-3-methyl-5-*H*-isothiazolo[5,4-*d*]pyrimidin-4-one, (460 mg, 100%) as white crystalline solid. 1H NMR (300 MHz, CDCl₃) δ 0.76 (t, 3H), 2.1-2.47 (m, 2H), 2.84 (s, 3H), 4.62 (t, 1H), 4.88 (d, 1H), 6.20 (d, 1H), 7.10-7.40 (m, 5H).

The following compounds were synthesized according to Method 9:

| **Method #** | **Compound Name** | ***m*/*z*** | **SM** |
|---|---|---|---|
| **9a** | 6-(1-bromopropyl)-5-[(4-fluorophenyl)methyl)]-3-methyl-isothiazolo[5,4-*d*]pyrimidin-4(5H)-one | 397 (MH⁺) | Method 8a |

### METHOD 10

### {3-[1-(5-Benzyl-3-methyl-4-oxo-4,5-dihydro-isothiazolo[5,4-d]pyrimidin-6-yl)-propylamino]-propyl}-carbamic Acid tert-Butyl Ester

To a solution of the bromide (method 9) (0.46 g, 1.22 mmol) in anhydrous ethanol (20 mL), was added *tert-* butyl 3-aminopropyl-carbamate (0.211 g, 1.22 mmol) followed by the addition of anhydrous diisopropylethylamine (0.258 g, 2 mmol) and the mixture was stirred at reflux for 16 hours. The TLC of the RM showed the complete disappearance of the starting bromide. The reaction mixture was poured into ice water (200 mL) and extracted with EtOAc (3 X 100 mL). The organic layer was washed with water (100 mL), brine (100 mL) and dried (Na₂SO₄). Concentration of the organic layer provided the product which was purified by column (silica gel) chromatography using 30-50% EtOAc in hexanes to isolate the pure amine {3-[1-(5-Benzyl-3-methyl-4-oxo-4,5-dihydro-isothiazolo[5,4-*d*]pyrimidin-6-yl)-propylamino]-propyl}-carbamic Acid *tert*-Butyl Ester (0.1 g, 17%) as a white foam. 1H NMR (300 MHz, CDCl₃) δ 0.95 (t, 3H), 1.33 (t, 2H), 1.42 (s, 9H), 1.49-1.51 (m, 2H), 1.87-1.99 (m, 1H), 2.35-2.45 (m, 1H), 2.83 (s, 3H), 2.92-3.20 (m, 2H), 3.64-3.70 (m, 1H), 4.98 (d, 1H), 5.17 (bs, 1H), 5.85 (d, 1H), 7.10-7.40 (m, 5H).

The following compounds were synthesized according to Method 10:

| **Method #** | **Compound Name** | ***m*/*z*** | **SM** | **Amine** |
|---|---|---|---|---|
| **10a** | [3-({1-[5-(4-fluorobenzyl)-3-methyl-4-oxo-4,5-dihydro-isothiazolo[5,4-*d*]pyrimidin-6-yl]-propyl}amino)-propyl}-carbamic Acid *tert*-Butyl Ester | 490 (MH⁺) | Method 9a | *tert-* butyl 3-aminopropy 1-carbamate |

### METHOD 11

### {3-[1-(5-Benzyl-3-methyl-4-oxo-4,5-dihydro-isothiazolo[5,4-d]pyrimidin-6-yl)-propylamino]-propyl}-carbamic acid tert-butyl ester

To a solution of the bromide (method 9) (0.1 g, 0.26 mmol) in anhydrous dichloromethane (5 mL), was added anhydrous diisopropylethylamine (100 µl, 0.52 mmol) followed by *tert-* butyl 3-aminopropyl-carbamate (0.10 g, 0.52 mmol). The reaction mixture was microwaved at 120°C for .2h. The LC/MS of the RM showed the complete disappearance of the starting bromide. The reaction mixture was evaporated to dryness the product was purified by column (silica gel) chromatography using 40-60% EtOAc in hexanes to isolate the pure amine {3-[1-(5-Benzyl-3-methyl-4-oxo-4,5-dihydro-isothiazolo[5,4-*d*]pyrimidin-6-yl)-propylamino]-propyl}-carbamic Acid *tert*-Butyl Ester (0.085 g, 64%). *m*/*z* 472 (MH⁺).

The following compounds were synthesized according to Method 11:

| **Method #** | **Compound Name** | ***m*/*z*** | **SM** | **Amine** |
|---|---|---|---|---|
| **11a** | {2-[1-(5-Benzyl-3-methyl-4-oxo-4,5-dihydro-isothiazolo[5,4-d]pyrimidin -6-yl)-propylamino]-ethyl}-carbamic acid tert-butyl ester | 458 (MH⁺) | Method 9 | (2-Amino-ethyl)-carbamic acid tert-butyl ester |
| **11b** | 3-{[1-(5-Benzyl-3-methyl-4-oxo-4,5-dihydro-isothiazolo[5,4-d]pyrimidin-6-yl)-propylamino]-methyl}-azetidine-1-carboxylic acid tert-butyl ester | 484 (MH⁺) | Method 9 | 3-aminomet hylazetidine-1-carboxylic acid tert-butyl ester |
| **11c** | 3-{[1-(5-Benzyl-3-methyl-4-oxo-4,5-dihydro-isothiazolo[5,4-d]pyrimidin-6-yl)-propylamino]-methyl}-piperidine-1-carboxylic acid tert-butyl ester | 512 (MH⁺) | Method 9 | 3-aminomet hylpiperidine -1-carboxylic acid tert-butyl ester |
| **11d** | 5-Benzyl-6-[1-(2-dimethylamino-ethy lamino)-propyl]-3-methyl-5H-isothia zolo[5,4-d]pyrimidin-4-one | 386 (MH⁺) | Method 9 | Dimethylethane-1,2-diamine |
| **11e** | 5-Benzyl-6-[1-(3-dimethylamino-prop ylamino)-propyl]-3-methyl-5H-isothi azolo[5,4-d]pyrimidin-4-one | 400 (MH⁺) | Method 9 | Dimethylpropane-1,3-diamine |
| **11f** | 5-Benzyl-6-[1-(3-hydroxy-propylamin o)-propyl]-3-methyl-5H-isothiazolo[ 5,4-d]pyrimidin-4-one | 373 (MH⁺) | Method 9 | 3-Amino-propan-1-ol |

### METHOD 12

### N-(3-Amino-propyl)-N-[1-(5-benzyl-3-methyl-4-oxo-4,5-dihydro-isothiazolo-[5,4-d]pyrimidin-6-yl)-propyl]-4-methyl-benzamide.

To a solution of the amine 13 (method 10) (0.1 g, 0.21 mmol) and triethylamine (0.303 g, 3 mmol) in dichloromethane (20 mL) at r.t. was added dropwise a solution ofp-toluoyl chloride (0.1 g, 0.6 mmol) in dichloromethane (10 mL). The resulting solution was stirred at r.t. for 30 min. after which the TLC showed the disappearance of the SM. The reaction mixture was diluted with CH₂Cl₂ (60 mL) washed with satd. NaHCO₃ (100 mL), water (100 mL), brine (100 mL) and dried (Na₂SO₄). Concentration of the organic layer provided the product which was purified by column (silica gel) chromatography using 20-30% EtOAc in hexanes as eluent. Yield = 0.117 g (94%). The acylated product was dissolved in 2M HCl in ether and the mixture was stirred at r.t. for 20 h.. The precipitated product was filtered off and washed with ether and dried *in vacuo* to yield the pure *N*-(3-amino-propyl)-*N*-[1-(5-benzyl-3-methyl-4-oxo-4,5-dihydro-isothiazolo-[5,4-*d*]pyrimidin-6-yl)-propyl]-4-methyl-benzamide chloride salt (91 mg, 87%). White powder, mp. 127.8-129.2°C *m*/*z* 490 (MH⁺), ¹H NMR (DMSO-d6 300MHz, 96 °C) δ: 7.79 (bs, 3H), 7.37-6.95 (m, 9H), 5.77 (d, 1H), 5.50 (bs, 1H), 4.83 (d, 1H), 3.36 (t, 2H), 2.72 (s, 3H), 2.46 (t, 2H), 2.39 (s, 3H), 2.20-2.05 (m, 1H), 1.96-1.75 (m, 1H),1.74-1.40 (m, 2H), 0.63 (t, 3H).

The following compounds were synthesized according to Method 12:

| **Method #** | **Compound Name** | ***m*/*z*** | **SM** | **Acylating agent** |
|---|---|---|---|---|
| **12a** | *N*-(3-Amino-propyl)-*N*-[1-(5-{4-fluorobenzyl}-3-methyl-4-oxo-4,5-dihydro-isothiazolo[5,4-*d*]pyrimidin-6-yl)-propyl]-4-methyl-benzamide | 507 (MH ⁺) | Method 10a | 4-methyl-benzoyl chloride |

### METHOD 13

### N-(3-Amino-propyl)-N-[1-(5-benzyl-3-methyl-4-oxo-4,5-dinhydro-isothiazolo[5,4-d]pyrimidin-6-yl)-propyl]-4-bromo-benzamide

To a solution of {3-[1-(5-Benzyl-3-methyl-4-oxo-4,5-dihydro-isothiazolo[5,4-*d*]pyrimidin-6-yl)-propylamino]-propyl}-carbamic acid *tert*-butyl ester (method 11) (0.085 g, 0.167 mmol) in dichloromethane (8 mL) at r.t. was added a saturated solution of potassium carbonate (8ml) followed by the dropwise addition of *p*-bromo benzoyl chloride (0.044 g, 0.2 mmol). The resulting solution was stirred at r.t. for 16h after which the LC/MS showed the disappearance of the SM. The reaction mixture was evaporated to dryness and resuspended in 3ml MeOH and purified by Gilson HPLC using a 20-99% H₂0/CH₃CN (0.05% TFA) gradient. Concentration of the desired fractions gave {3-[1-(5-Benzyl-3-methyl-4-oxo-4,5-dihydro-isothiazolo[5,4-*d*]pyrimidin-6-yl)-propyl] (4-bromobenzoyl)amino]propyl}-carbamic acid *tert*-butyl ester. The product was dissolved in 2M HCl in 1,4 dioxane and the mixture was stirred at r.t. for 1h. The reaction mixture was evaporated to dryness, washed with ether and dried *in vacuo* to yield the pure N-(3-Amino-propyl)-N-[1-(5-benzyl-3-methyl-4-oxo-4,5-dihydro-isothiazolo[5,4-d]pyrimidin-6-yl)-propyl]-4-bromo-benzamide hydrogen chloride salt (33 mg, 34%). *m*/*z* 556 (MH⁺), 1H NMR (DMSO-d6 500MHz, 96 °C) δ: 7.80 (br, 3H), 7.64 (d, 2H), 7.36-7.28 (m, 5H), 7.13 (m, 2H), 5.80 (d, 1H), 5.57 (bs, 1H), 4.95 (d, 1H), 3.38 (t, 2H), 2.77 (s, 3H), 2.47 (t, 2H), 2.17-2.13 (m, 1H), 1.96-1.91 (m, 1H),1.72-1.50 (m, 2H), 0.68 (t, 3H).

The following compounds were synthesized according to Method 13:

| **Method #** | **Compound Name** | ***m*/*z*** | **SM** | **Acylatingagent** |
|---|---|---|---|---|
| **13a** | N-(3-Amino-propyl)-N-[1-(5-benzyl-3-methyl-4-oxo-4,5-dihydro-isothiazolo[5,4-d]pyrimidin-6-yl)-propyl]-4-chloro-benzamide hydrogen chloride | 510 (MH⁺) | Method 11 | 4-chloro-benzoyl chloride |
| **13b** | N-(3-Amino-propyl)-N-[1-(5-benzyl-3-methyl-4-oxo-4,5-dihydro-isothiazolo[5,4-d]pyrimidin-6-yl)-propyl]-3-fluoro-4-methyl-benzamide hydrogen chloride | 508 (MH⁺) | Method 11 | 3-fluoro-4-methyl-benzoyl chloride |
| **13c** | N-(3-Amino-propyl)-N-[1-(5-benzyl-3-methyl-4-oxo-4,5-dihydro-isothiazolo[5,4-d]pyrimidin-6-yl)-propyl]-2,3-dichloro-benzamide hydrogen chloride | 545 (MH⁺) | Method 11 | 2,3-dichloro-benzoyl chloride |
| **13d** | Naphthalene-2-carboxylic acid (3-amino-propyl)-[1-(5-benzyl-3-methyl-4-oxo-4,5-dihydro-isothiazolo[5,4-d]pyrimidin-6-yl)-propyl]-amide hydrogen chloride | 526 (MH⁺) | Method 11 | 2-napthoyl-chloride |
| **13e** | Benzo[b]thiophene-2-carboxylic acid (3-amino-propyl)-[1-(5-benzyl-3-methyl-4-oxo-4,5-dihydro-isothiazolo[5,4-d]pyrimidin-6-yl)-propyl]-amide hydrogen chloride | 532 (MH⁺) | Method 11 | 1-benzothio phene-2-carbonyl chloride |
| **13f** | N-Azetidin-3-ylmethyl-N-[1-(5-benzy 1-3-methyl-4-oxo-4,5-dihydro-isothi azolo[5,4-d]pyrimidin-6-yl)-propyl]-4-methyl-benzamide hydrogen chloride | 502 (MH⁺) 11 | Method 11 | 4-methyl-benzoyl chloride |
| **13g** | N-[1-(5-Benzyl-3-methyl-4-oxo-4,5-d ihydro-isothiazolo[5,4-d]pyrimidin-6-yl)-propyl]-4-methyl-N-piperidin-3-ylmethyl-benzamide hydrogen chloride | 530 (MH⁺) | Method 11 | 4-methyl-benzoyl chloride |
| **13h** | N-(2-Amino-ethyl)-N-[1-(5-benzyl-3-methyl-oxo-4,5-dihydro-isothiazol o[5,4-d]pyrimidin-6-yl)-propyl]4-m ethyl-benzamide hydrogen chloride | 476 (MH⁺) | Method 11 | 4-methyl-benzoyl chloride |

### METHOD 14

### N-[1-(5-Benzyl-3-methyl-4-oxo-4,5-dihydro-isothiazolo[5,4-d]pyrimidin-6-yl)-propyl]-N-(3-dimethylamino-propyl)-4-methyl-benzamide

To a solution of 5-Benzyl-6-[1-(3-dimethylamino-propylamino)-propyl]-3-methyl-5H-isothiazolo[5,4-d]pyrimidin-4-one (method 11e) (0.104g, 0.26 mmol) in dichloromethane (10 mL) at r.t was added a saturated solution of potassium carbonate (10ml) followed by the dropwise addition of *p*-toluoyl chloride (34µL, 0.26 mmol). The resulting solution was stirred at r.t. for 16h after which the LC/MS showed the disappearance of the SM. The reaction mixture was evaporated to dryness and resuspended in 3ml MeOH and purified by Gilson HPLC using a 20-99% H₂0/CH₃CN (0.05% TFA) gradient. Concentration of the desired fractions gave N-[1-(5-Benzyl-3-methyl-4-oxo-4,5-dihydro-isothiazolo[5,4-d]pyrimidin-6-yl)-propyl]-N-(3-dimethylamino-propyl)-4-methyl-benzamide (65 mg, 48%). *m*/*z* 518 (MH⁺), ¹H NMR (DMSO-d6 300MHz, 96 °C) δ: 7.44-7.00 (m, 9H), 5.82 (d, 1H), 5.51 (bs, 1H), 4.86 (d, 1H), 3.41 (t, 2H), 2.75 (s, 3H), 2.50 (s, 6H), 2.39 (bm, 2H), 2.12-2.05 (m, 1H), 1.93-1.90 (m, 1H), 1.75 (m, 1H), 1.50 (m, 1H), 0.66 (t, 3H).

The following compounds were synthesized according to Method 14:

| **Method #** | **Compound Name** | ***m*/*z*** | **SM** | **Acylating** agent |
|---|---|---|---|---|
| **14a** | N-[1-(5-Benzyl-3-methyl-4-oxo-4,5-dihydro-isothiazolo[5,4-d]pyrimidin-6-yl)-propyl]-N-(2-dimethylamino-ethyl)-4-methyl-benzamide | 504 (MH⁺) | Method 11d | 4-methyl-benzoyl chloride |
| **14b** | N-[1-(5-Benzyl-3-methyl-4-oxo-4,5-d ihydro-isothiazolo[5,4-d]pyrimidin-6-yl)-propyl]-N-(3-hydroxy-propyl)-4-methyl-benzamide | 491 (MH⁺) | Method 11f | 4-methyl-benzoyl chloride |

### METHOD 15

### Methanesulfonic acid 3-[[1-(5-benzyl-3-methyl-4-oxo-4,5-dihydro-isothiazolo [5,4-d] pyrimidin-6-yl)-propyl]-(4-methyl-benzoyl)-amino]-propyl ester

To a solution of N-[1-(5-Benzyl-3-methyl-4-oxo-4,5-dihydro-isothiazolo[5,4-d]pyrimidin-6-yl)-propyl]-N-(3-hydroxy-propyl)-4-methyl-benzamide (method 14b) (0.42g, 0.85 mmol) in anhydrous dichloromethane (57 mL), was added anhydrous diisopropylethylamine (295 µl, 1.70 mmol) followed by dropwise addition of methanesulphonyl chloride (71 µl, 0.935 mmol). The reaction mixture was stirred at r.t. for 2h. The LC/MS of the RM showed the complete disappearance of the starting material and complete conversion to the methanesulfonic acid 3-[[1-(5-benzyl-3-methyl-4-oxo-4,5-dihydro-isothiazolo [5,4-d]-pyrimidin-6-yl)-propyl]-(4-methyl-benzoyl)-amino]-propyl ester. The reaction mixture was evaporated to dryness and used further crude.

### METHOD 16

### N-(3-Azetidin-1-yl-propyl)-N-[1-(5-benzyl-3-methyl-4-oxo-4,5-dihydro-isothiazolo[5,4-d]pyrimidin-6-yl)-propyl]-4-methyl-benzamide

To a solution of methanesulfonic acid 3-[[1-(5-benzyl-3-methyl-4-oxo-4,5-dihydro-isothiazolo [5,4-d] pyrimidin-6-yl)-propyl]-(4-methyl-benzoyl)-amino]-propyl ester (method 15) ((assumed from previous reaction) 0.080 g, 0.14 mmol) in DMF (25 mL) at r.t. was added an excess of potassium carbonate (0.097 g, 0.70mmol) followed by the dropwise addition of azetidine (19 µl, 0.28 mmol). The reaction mixture was stirred at 38°C for 16h after which the LC/MS showed the disappearance of the SM. The reaction mixture was evaporated to dryness on a GeneVac HT12 and resuspended in 3ml MeOH and purified by Gilson HPLC using a 20-99% H₂0/CH₃CN (0.05% HCl) gradient. Concentration of the desired fractions gave N-(3-Azetidin-1-yl-propyl)-N-[1-(5-benzyl-3-methyl-4-oxo-4,5-dihydro-isothiazolo[5,4-d]pyrimidin-6-yl)-propyl]-4-methylbenzamide (51 mg, 69%). *m*/*z* 530 (MH⁺), ¹H NMR (DMSO-d6 400MHz, 96 °C) δ: 7.40-7.00 (m, 9H), 5.85 (d, 1H), 5.55 (bs, 1H), 4.85 (d, 1H), 3.40 (b, 2H), 2.90 (b, 2H), 2.78 (s, 3H), 2.50 (b, 2H), 2.40 (s, 3H), 2.35 (bm, 2H), 2.20-2.00 (m, 1H), 1.96-1.80 (m, 1H), 1.65-1.50 (m, 1H), 1.40-1.30 (m, 3H), 0.65 (t, 3H)

The following compounds were synthesized according to Method 16:

| **Method #** | **Compound Name** | ***m*/*z*** | **SM** | **Amine** |
|---|---|---|---|---|
| **16a** | *N*-[1-(5-benzyl-3-methyl-4-oxo-4,5-dihydroisothiazolo-[5,4-*d*]pyrimidin-6-yl)propyl] -N-[3-(isopropylamino)propyl]-4-methylbenzamide | 532 (MH⁺) | Method 15 | Isopropylamine |
| **16b** | *N*-[1-(5-benzyl-3₋methyl-4-oxo-4,5-dihydroisothiazolo-[5,4-*d*]pyrimidin-6-yl)propyl]-N-[3-(cyclopropylamino)propyl]-4-methylbenzamide | 530 (MH⁺) | Method 15 | Cyclopropyl-amine |
| **16c** | *N*-[1-(5-benzyl-3-methyl-4-oxo-4,5-dihydroisothiazolo-[5,4-*d*]pyrimidin-6-yl)propyl] -4-methyl-N-[3-(methylamino) propyl] benzamide | 504 (MH⁺) | Method 15 | Methylamine |
| **16d** | *N*-[1-(5-benzyl-3-methyl4-oxo4,5-dihydroisothiazolo-[5,4-*d*]pyrimidin-6-yl)propyl]-4-methyl-N-[3-(3-pyrrolidin-1-ylpropyl) benzamide | 544 (MH⁺) | Method 15 | Pyrrolidine |

### METHOD 17

### 5-Benzyl-6-{1-[(3-hydroxy-propyl)-(4-methyl-benzyl)-amino]-propyl}-3-methyl-5H-isothiazolo[5,4-d]pyrimidin-4-one

To a solution of 5-Benzyl-6-[1-(3-hydroxy-propylamino)-propyl]-3-methyl-5H-isothiazolo[5,4-d]pyrimidin-4-one (method 11f) (0.098 g, 0.26 mmol) in anhydrous DMF (3 mL) was added potassium carbonate (0.108 g, 0.78 mmol) followed by the dropwise addition of 4-methyl benzyl bromide (0.048 g, 0.26 mmol). The resulting solution was shaken at 40°C for 4h after which the LC/MS showed the disappearance of the SM. The reaction mixture was evaporated to dryness and resuspended in 3ml MeOH and purified by Gilson HPLC using a 20-99% H₂0/CH₃CN (0.05% TFA) gradient. Concentration of the desired fractions gave 5-Benzyl-6-{1-[(3-hydroxypropyl)-(4-methyl-benzyl)-amino]-propyl}-3-methyl-5H-isothiazolo[5,4-d]pyrimidin-4-one (48 mg, 39%). *m*/*z* 477 (MH⁺), ¹H NMR (DMSO-d6 500MHz, 96 °C) δ: 8.20 (s, 1H), 7.40-6.85 (m, 9H), 5.80 (d, 1H), 5.20 (d, 1H), 3.80 (d, 1H), 3.70 (m, 1H), 3.62 (d, 1H), 3.50-3.30 (m, 2H), 2.90 (m, 1H), 2.75 (s, 3H), 2.33 (m, 2H), 2.25 (s, 3H), 2.20-2.16 (m, 1H), 1.90-1.80 (m, 1H), 1.50 (m, 2H), 0.65 (t, 3H).

### METHOD 18

### 5-Butyrylamino-3-methyl-isoxazole-4-carboxylic acid amide

A mixture of 5-amino-3-methyl-isoxazole-4-carboxylic acid amide (2 g, 14.18 mmol) in 10 ml of butyric anhydride was stirred at 150°C for 0.5∼1h. The brown solution was diluted with hexane (100 ml) and cooled to room temperature. The solid crushed out from the mixture was filtered and washed with hexane, dried *in vacuo*. The title amide (2.6 g) was obtained as white solid.

### METHOD 19

### 3-Methyl-6-propyl-5H-isoxazolo[5,4-d]pyrimidin4-one

A suspension of 5-Butyrylamino-3-methyl-isoxazole-4-carboxylic acid amide (method 18) (2.6 g, split into 20 vials) in 3.5 ml of 2N NaOH aq was subjected to microwave irradiation under the temperature of 140°C for 20min. The resulting solution was cooled with an ice bath, and the pH was adjusted to 1∼3 with concentrated HCl. The crushed out solid was filtered, washed with water, dried over vacuum at 40°C overnight. The title pyrimidinone (1.749 g) was obtained as white solid. ¹H NMR (400MHz, DMSO-*d*6): 0.91 (t, 3H), 1.71 (m, 2H), 2.44 (s, 3H), 2.64 (t, 2H), 12.78 (s, 1H).

### METHOD 20

### 5-Benzyl-3-methyl-6-propyl-5H-isoxazolo[5,4-d]pyrimidin-4-one

A suspension of 3-methyl-6-propyl-5H-isoxazolo[5,4-d]pyrimidin-4-one (method 19) (1.698 g, 8.8 mmol), benzylbromide (1.5 g, 8.8 mmol), potassium carbonate (2.43 g, 17.6 mmol) in 10 ml DMF was stirred at room temperature overnight. The mixture was diluted with water, extracted with ethyl acetate (50 ml x 3), the combined organic phases were dried over anhydrous sodium sulfate, concentrated, purified by flash column chromatography (elute: hexane-ethyl acetate = 5:1). 1.69 g (68%) of the title compound was obtained as white solid. ¹H NMR (400MHz, DMSO-*d*6): 0.80 (t, 3H), 1.61 (m, 2H), 2.43 (s, 3H), 2.73 (t, 2H), 5.35 (s, 2H), 7.12-7.35 (m, 5H).

### METHOD 21

### 5-Benzyl-6-(1-bromo-propyl)-3-methyl-5H-isoxazolo[5,4-d]pyrimidin-4-one

A solution of 5-benzyl-3-methyl-6-propyl-5H-isoxazolo[5,4-d]pyrimidin-4-one (method 20) (3.167 g, 11.2 mmol) and sodium acetate (4.59 g, 56 mmol, 5 eq) in glacial acetic acid (26 ml) was treated with a preformed bromine solution (0.7 ml bromine in 10 ml of glacial acetic acid) (8.64 ml, 22.4 mmol, 2 eq). The mixture was stirred at 100°C for 24 hrs. Excess bromine (8.64 ml, 22.4 mmol, 2 eq) was added to the mixture. The mixture was then stirred at 100°C for another 24 hrs. Water was added to the reaction mixture, followed by aq. potassium carbonate. The mixture was extracted with methylene chloride (50 ml x 3), the combined organic phases were washed with water and dried over anhydrous sodium sulfate, then concentrated to give the product which was purified by flash chromatography (elute: hexane-ethyl acetate). 2.5 g product was furnished as a white solid. ¹H NMR (400MHz, DMSO-*d6*): 0.79 (t, 3H), 2.18 (m, 1H), 2.35 (m, 1H), 2.58 (s, 3H), 5.12 (t, 1H), 5.25 (d, 1H), 5.80 (d, 1H), 7.27-7.42 (m, 5H).

### METHOD 22

### 5-Benzyl-6-(1-butylamino-propyl)-3-methyl-5H-isozazolo[5,4-d]pyrimidin-4-one

To a suspension of 5-benzyl-6-(1-bromo-propyl)-3-methyl-5H-isoxazolo[5,4-d]pyrimidin-4-one (method 21) (2.8 g, 7.73 mmol) and potassium carbonate (2.67 g, 19.38 mmol) in acetonitrile (100 ml) was added tert-butyl-N-(3-aminopropyl)-carbamate (1.345 g, 7.73 mmol). The mixture was stirred at 100°C overnight Water (30 ml) was added to the mixture, which was extracted with ethyl acetate (3 x 50 ml). The combined organic phases were washed with brine (10 ml), dried over sodium sulfate, concentrated to obtain the title amine which was purified by flash chromatography column (elute: ethyl acetate-hexane = 1-4 ∼ 1-1) to give 2.6 g (74%) of product as white solid. ¹H NMR (400 MHz, DMSO-d*₆*): 0.85 (t, 3H), 1.32 (m, 2H), 1.41 (s, 9H), 1.58 (m, 1H), 1.65 (m, 1H), 2.09 (m, 1H), 2.40 (m, 1H), 2.60 (s, 3H), 2.81 (m, 2H), 3.29 (m, 1H), 3.75 (m, 1H), 5.42 (d, 1H), 5.63 (d, 1H), 6.72 (br, 1H), 7.25-7.45 (m, 5H).

### METHOD 23

### N-[[1-(5-Benzyl-3-methyl-4-oxo-4,5-dihydro-isoxazolo[5,4-d]pyrimidin-6-yl)-propyl]-(4-methyl-benzoyl)-amino]-propyl)-carbamic acid tert-butyl ester

A solution of 5-benzyl-6-(1-butylamino-propyl)-3-methyl-5H-isoxazolo[5,4-d]pyrimidin-4-one (method 22) (135 mg, 0.297 mmol) in dichloromethane (4 ml) was added to p-toluoyl chloride (46mg, 0.297 mmol) followed by triethylamine (60 mg, 0.594 mmol). The mixture was stirred at room temperature for 1hr. Then diluted with dichloromethane, washed with saturated aq. sodium bicarbonate. The organic phase was dried over sodium sulfate, filtered, and concentrated. The crude oil was purified by flash column chromatography (solvent: ethyl acetate-hexane) to furnish N-[[1-(5-benzyl-3-methyl-4-oxo-4,5-dihydro-isoxazolo[5,4-d]pyrimidin-6-yl)-propyl]-(4-methyl-benzoyl)-amino]-propyl)-carbamic acid tert-butyl ester (130mg) as a white solid.
¹H NMR (500 MHz, 100°C, DMSO-d*₆*): 0.71 (t, 3H), 1.12 (m, 1H), 1.35 (s, 9H), 1.47 (m, 1H), 1.92 (m, 1H), 2.14 (m, 1H), 2.37 (s, 3H), 2.56 (s, 3H), 2.57 (m, 2H), 3.29 (m, 2H), 5.01 (d, 1H), 5.68 (m, br, 1H), 5.79 (d, 1H), 6.06 (br, 1H), 7.14-7.36 (m, 9H).

### METHOD 24

### N-(3-Amino-propyl)-N-[1-(5-benzyl-3-methyl4-oxo-4,5-dihydro-isoxazolo[5,4-d]pyrimidin-6-yl)-propyl]-4-methyl-benzamide

A solution of N-[[1-(5-benzyl-3-methyl-4-oxo-4,5-dihydro-isoxazolo[5,4-d]pyrimidin-6-yl)-propyl]-(4-methyl-benzoyl)-amino]-propyl)-carbamic acid tert-butyl ester (method 23) (0.223 mmol) in 3 ml of 4 M HCl in dioxane was stirred at room temperature for 2hr. The solvent was distilled off by vacuo, the residue was dried at 40∼50°C for overnight under vacuum. The corresponding amine chloride salt was obtained. *m*/*z* 474 (MH⁺) ¹H NMR (500 MHz, 100°C, DMSO-d*₆*): 0.68 (t, 3H), 1.52 (m, 1H), 1.72 (m, 1H), 1.92 (m, 1H), 2.10 (m, 1H), 2.39 (s, 3H), 2.51 (m, 2H), 2.57 (s, 3H), 3.41 (m, 2H), 4.85 (br, 1H), 5.50 (br, 1H), 5.77 (d, 1H), 7.07 (br, 2H), 7.24-7.35 (m, 7H), 7.73 (br, 3H).

The following compounds were synthesized according to Method 24:

| **Method #** | **Compound Name** | ***m*/*z*** | **SM** | **Acylating agent** |
|---|---|---|---|---|
| **24a** | N-(3-Amino-propyl)-N-[1-(5-benzyl-3-methyl-4-oxo-4,5-dihydro-isoxazolo[5,4-d]pyrimidin-6-yl)-propyl]-4-fluoro-benzamide hydrogen chloride | 478 (MH⁺) | Method 23 | 4-fluoro-benzoyl chloride |
| **24b** | N-(3-Amino-propyl)-N-[1-(5-benzyl-3-methyl-4-oxo-4,5-dihydro-isoxazolo[5,4-d]pyrimidin-6-yl)-propyl]-2,3-dichloro-benzamide hydrogen chloride | 529 (MH⁺) | Method 23 | 2,3-dichloro-benzoyl chloride |
| **24c** | N-(3-Amino-propyl)-N-[1-(5-benzyl-3-methyl-4-oxo-4,5-dihydro-isoxazolo[5,4-d]pyrimidin-6-yl)-propyl]-3-fluoro-4-methyl-benzamide hydrogen chloride | 492 (MH⁺) | Method 23 | 3-fluoro-4-methyl-benzoyl chloride |
| **24d** | N-(3-Amino-propyl)-N-[1-(5-benzyl-3-methyl-4-oxo-4,5-dihydro-isoxazolo[5,4-d]pyrimidin-6-yl)-propyl]-4-methoxy-benzamide hydrogen chloride | 490 (MH⁺) | Method 23 | 4-methoxy-benzoyl chloride |

### METHOD 25

### N-(4-Cyano-3-methyl-isothiazol-5-yl)-3-methyl-butyramide

To a solution of 5-amino-3-methyl-isothiazole-4-carbonitrile (method 4) (6.38 g, 45.9 mmol) in pyridine (20 mL) at 0 °C, isovaleryl chloride (6.65 g, 55 mmol) was added dropwise. After the completion of the addition the reaction mixture was allowed to warm to r.t. and stirred overnight. The TLC and the MS showed the complete disappearance of the starting material and the reaction mixture was diluted with CHCl₃ (200 mL), washed with water (200 mL), 2N HCl (225 mL), satd. NaHCO₃ (200 mL), brine (200 mL) and dried over Na₂SO₄. Concentration of the CHCl₃ layer provided the product which was triturated from CH₂Cl₂/hexanes (1/10) and filtered off to isolate N-(4-cyano-3-methyl-isothiazol-5-yl)-3-methyl-butyramide (8.1 g, 79%) as an off-white crystalline solid. 1H NMR (300 MHz, CDCl₃) δ 1.04 (d, 6H), 2.18-2.32 (m, 1H), 2.46 (d, 2H), 2.53 (s, 3H), 9.87 (bs, 1H).

### METHOD 26

### 3-Methyl-5-(3-methyl-butyrylamino)-isothiazole-4-carboxylic acid amide

To a solution of N-(4-cyano-3-methyl-isothiazol-5-yl)-3-methyl-butyramide (method 25) (8 g, 35.8 mmol) in 30% aqueous NH₄OH (200 mL), was added dropwise 100 mL of hydrogen peroxide at r.t. After the completion of the addition the reaction mixture was stirred at 60 °C overnight after which the TLC showed the complete disappearance of SM. The reaction mixture was concentrated to 40 mL and extracted with chloroform (3 x 100 mL). The organic layer was dried (Na₂SO₄) and concentrated to obtain 3-methyl-5-(3-methyl-butyrylamino)-isothiazole-4-carboxylic acid amide (6.1 g, 71%) as a light yellow solid. 1H NMR (300 MHz, CDCl₃) δ 1.03 (d, 6H), 2.24 (m, 1H), 2.43 (d, 2H), 2.69 (s, 3H), 5.98 (bs, 2H), 11.77 (bs, 1H).

### METHOD 27

### 6-Isobutyl-3-methyl-5H-isothiazolo[5,4-d]pyrimidin-4-one

3-Methyl-5-(3-methyl-butyrylamino)-isothiazole-4-carboxylic acid amide (method 26) (6 g, 25 mmol) was suspended in 150 mL of 30% NH₃ and then was heated to 140 °C for 5h in a pressure reactor. The mixture was cooled and neutralized to pH 7. The reaction mixture was extracted with EtOAc (3 x 100 mL) and the combined organic layers were washed with water (100 mL), brine (100 mL) and concentrated to get the product which was further purified by column (silica gel) chromatography using 30% EtOAc in hexanes as eluent. Concentration of the pure product fractions provided 6-isobutyl-3-methyl-5H-isothiazolo[5,4-d]pyrimidin-4-one (2.2 g, 38%) as an off-white powder. 1H NMR (300 MHz, CDCl₃) δ 1.05 (d, 6H), 2.32 (m, 1H), 2.69 (d, 2H), 2.82 (s, 3H).

### METHOD 28

### 5-Benzyl-6-isobutyl-3-methyl-5H-isothiazolo[5,4-d]pyrimidin-4-one

To a solution of 6-isobutyl-3-methyl-5H-isothiazolo[5,4-d]pyrimidin-4-one (method 27) (1.31 g, 5.8 mmol) in 20 mL of anhydrous DMF was added 1.38 g (10 mmol) of anhydrous K₂CO₃ followed by benzyl bromide (1.18 g, 6.9 mmol) and the mixture was stirred at room temperature overnight. The TLC of the reaction mixture showed the complete disappearance of the SM. The reaction mixture was poured into ice-cold water and extracted with EtOAc (3X100 mL). The combined extracts were washed with water (100 mL), brine (100 mL), dried (Na₂SO₄) and concentrated. The TLC and the 1H NMR showed the presence of two products *N* alkylated as well as *O*-alkylated products in a ratio of 7:3. The products were separated by column (silica gel, 116 g) chromatography using 10% EtOAc in hexanes. 5-Benzyl-6-isobutyl-3-methyl-5H-isothiazolo[5,4-d]pyrimidin-4-one was isolated as white crystalline solid (1.3 g, 70%). *m*/*z* 314 (MH⁺), 1H NMR (300 MHz, CDCl₃) δ 0.94 (d, 6H), 2.23-2.37 (m, 1H), 2.64 (d, 2H), 2.82 (s, 3H), 5.38 (s, 2H), 7.10-7.38 (m, 5H).

The following compounds were synthesized according to Method 28:

| **Method #** | **Compound Name** | ***m*/*z*** | **Alkylating agent** |
|---|---|---|---|
| **28a** | 5-(4-Fluoro-benzyl)-6-isobutyl-3-methyl-5H-isothiazolo[5,4-d]pyrimidin-4-one | 332 (MH⁺) | 4-fluorobenzyl bromide |

### METHOD 29

### 5-Benzyl-6-(1-bromo-2-methyl-propyl)-3-methyl-5H-isothiazolo[5,4-d]pyrimidin-4-one

To a solution of 5-benzyl-6-isobutyl-3-methyl-5H-isothiazolo[5,4-d]pyrimidin-4-one (method 28) (1.3 g, 4.2 mmol) and sodium acetate (2 g) in acetic acid (10 mL) at 100 °C, a solution of the bromine (1.32 g, 8.4 mmol) in acetic acid (10 mL) was added dropwise over a period of 20 minutes. The reaction mixture was stirred at that temperature for 30 min and cooled and the TLC (eluent 10% EtOAc in hexanes) and MS showed the complete disappearance of the SM and only the product. The reaction mixture was poured into ice water and extracted with EtOAc (3 X 60 mL) and the organic layers were combined and washed with 2% sodium thiosulfate solution (60 mL), water (100 mL), brine (100 mL) and dried over Na₂SO₄. Concentration of the organic layer provided 5-benzyl-6-(1-bromo-2-methyl-propyl)-3-methyl-5H-isothiazolo[5,4-d]pyrimidin-4-one (1.61 g, 99%) as white crystalline solid. *m*/*z* 394 (MH⁺), 1H NMR (300 MHz, CDCl₃) δ 0.54 (d, 3H), 1.11 (d, 3H), 2.62-2.76 (m, 1H), 2.83 (s, 3H), 4.42 (d, 1H), 4.80 (d, 1H), 6.22 (d, 1H), 7.12-7.42 (m, 5H).

The following compounds were synthesized according to Method 29:

| **Method #** | **Compound Name** | ***m*/*z*** |
|---|---|---|
| **29a** | 6-(1-Bromo-2-methyl-propyl)-5-(4-fluoro-benzyl)-3-methyl-5H-isothiazolo[5,4-d]pyrimidin-4-one | 412 (MH⁺) |

### METHOD 30

### 6-(1-Azido-2-methyl-propyl)-5-benzyl-3-methyl-5H-isothiazolo[5,4-d]pyrimidin-4-one

To a solution of 5-benzyl-6-(1-bromo-2-methyl-propyl)-3-methyl-5H-isothiazolo[5,4-d]pyrimidin-4-one (method 29) (0.6 g, 1.52 mmol) in anhydrous DMF (20 mL), sodium azide (0.65 g, 10 mmol) was added and the mixture was stirred at room temperature for 1 hour. The TLC of the RM showed the complete disappearance of the starting bromide. The reaction mixture was poured into ice water (300 mL) and extracted with EtOAc (3 X 100 mL). The organic layer was washed with water (100 mL), brine (100 mL) and dried (Na₂SO₄). Concentration of the organic layer provided the product which was purified by column (silica gel) chromatography using 30% EtOAc in hexanes as eluent to isolate 6-(1-azido-2-methyl-propyl)-5-benzyl-3-methyl-5H-isothiazolo[5,4-d]pyrimidin-4-one (0.506 g, 94%) as a low melting solid. *m*/*z* 355 (MH⁺),1H NMR (300 MHz, CDCl₃) δ 0.57 (d, 3H), 1.07 (d, 3H), 2.50-2.74 (m, 1H), 2.98 (s, 3H), 3.71 (d, 1H), 5.05 (d, 1H), 5.78 (d, 1H), 7.12-7.40 (m, 5H).

The following compounds were synthesized according to Method 30:

| **Method #** | **Compound Name** | ***m*/*z*** |
|---|---|---|
| **30a** | 6-(1-Azido-2-methyl-propyl)-5-(4-fluoro-benzyl)-3-methyl-5H-isothiazolo[5,4-d]pyrimidin-4-one | 373 (MH⁺) |

### METHOD 31

### 6-(1-Amino-2-methyl-propyl)-5-benzyl-3-methyl-5H-isothiazolo[5,4-d]pyrimidin-4-one

To a solution of 6-(1-azido-2-methyl-propyl)-5-benzyl-3-methyl-5H-isothiazolo[5,4-d]pyrimidin-4-one (method 30) (0.5 g, 1.41 mmol) in methanol (20 mL) was added 5% Pd/C (20% by wt.) and the resulting mixture was stirred at r.t. in an atmosphere of H₂ and the progress of the reaction was monitored by MS. After the disappearance of the starting material the reaction mixture was filtered through celite and washed with EtOAc. Concentration of the filtrate provided 6-(1-amino-2-methyl-propyl)-5-benzyl-3-methyl-5H-isothiazolo[5,4-d]pyrimidin-4-one as a thick oil. The product was used as such in the next reaction with out further purification. *m*/*z* 349 (MH⁺)

The following compounds were synthesized according to Method 31:

| **Method #** | **Compound Name** | ***m*/*z*** |
|---|---|---|
| **31a** | 6-(1-Amino-2-methyl-propyl)-5-(4-fluoro-benzyl)-3-methyl-5H-isothiazolo[5,4-d]pyrimidin-4-one | 367 (MH⁺) |

### METHOD 32

### {3-[1-(5-Benzyl-3-methyl-4-oxo-4,5-dihydro-isothiazolo[5,4-d]pyrimidin-6-yl)-2-methylpropylamino]-propyl}-carbamic acid tert-butyl ester

To a solution of 6-(1-amino-2-methyl-propyl)-5-benzyl-3-methyl-5H-isothiazolo[5,4-d]pyrimidin-4-one (method 31) in dichloromethane (30 mL), 4 Å molecular sieves (5 g) was added followed by (3-oxo-propyl)-carbamic acid tert-butyl ester (1.2 eq) and the reaction mixture was stirred at r.t. for 3 h and the progress of the reaction was monitored by MS. After the complete disappearance of the starting amine, a catalytic amount of acetic acid was added to the reaction followed by sodium triacetoxyborohydride (1.2 eq) and the reaction mixture was stirred at r.t overnight. After the completion of the reaction (MS), the reaction mixture was filtered and the residue was washed with dichloromethane and the filtrate was washed with water (100 mL), brine (100 mL) and concentrated to get the product which was used as such for the next reaction. *m*/*z* 486 (MH⁺)

The following compounds were synthesized according to Method 32:

| **Method #** | **Compound Name** | ***m*/*****z*** |
|---|---|---|
| **32a** | (3-{1-[5-(4-Fluoro-benzyl)-3-methyl-4-oxo-4,5-dihydro-isothiazolo[5,4-d]pyrimidin-6-yl]-2-methyl-propylamino}-propyl)-carbamic acid tert-butyl ester | 504 (MH⁺) |

### METHOD 33

### N-(3-Amino-propyl)-N-[1-(5-benzyl-3-methyl-4-oxo4,5-dihydro-isothiazolo[5,4-d]pyrimidin-6-yl)-2-methyl-propyl]-4-methyl-benzamide

To a solution of the {3-[1-(5-benzyl-3-methyl-4-oxo-4,5-dihydro-isothiazolo[5,4-d]pyrimidin-6-yl)-2-methyl-propylamino]-propyl}-carbamic acid tert-butyl ester (method 31) in pyridine (10 mL) at r.t., a solution of the p-toluoyl chloride (0.616 g, 4 mmol) in dichloromethane (10 mL) was added dropwise and the resulting solution was stirred at r.t. for 2 days. after which the TLC showed the disappearance of most of the SM. The reaction mixture was diluted with CH₂Cl₂ (100 mL) washed with water (2 X 100 mL), brine (100 mL) and dried (Na₂SO₄). Concentration of the organic layer provided the product which was purified by column (silica gel) chromatography using 20-30% EtOAC in hexanes as eluent. Yield = 0.276 g of amide. The acylated product was dissolved in 4M HCl in 1,4-dioxane and the mixture was stirred at r.t. for 20 min and the TLC showed the complete disappearance of the starting material. The reaction mixture was concentrated in a rotory evaporator and the residue was triturated with ether. The precipitated product was filtered off and washed with ether and dried under *vacuo* to yield N-(3-amino-propyl)-N-[1-(5-benzyl-3-methyl-4-oxo-4,5-dihydro-isothiazolo[5,4-d]pyrimidin-6-yl)-2-methyl-propyl]-4-methyl-benzamide as the hydrochloride salt (196 mg, 99%). White powder, mp. 139-140 °C *m*/*z* 504 (MH⁺), ¹H NMR (DMSO-d6 300MHz, 96 °C) δ: 0.45 (d, 3H), 0.90 (d, 3H), 1.12-1.30 (m, 1H), 1.46-1.63 (m, 1H), 2.25 (t, 2H), 2.36 (s, 3H), 2.64-2.7 (m, 1H), 2.68 (s, 3H), 3.34 (t, 2H), 5.06 (d, 1H), 5.59 (d, 1H), 5.90 (d, 1H), 7.20-7.40 (m, 9H), 7.71 (bs, 3H).

The following compounds were synthesized according to Method 33:

| **Method #** | **Compound Name** | ***m*/*z*** |
|---|---|---|
| **33a** | N-(3-Amino-propyl)-N-{1-[5-(4-fluoro-benzyl)-3-methyl-4-oxo-4,5-dihydro-isothiazolo[5,4-d]pyrimidin-6-yl]-2-methyl-propyl}-4-methyl-benzamide | 522 (MH⁺) |

### METHOD 34

### 3-Methyl-5-(3-methyl-butyryl)-isoxazole-4-carboxylic acid amide

A mixture of 5-amino-3-methyl-isoxazole-4-carboxylic acid amide (10 g, 70 mmol) in 25 ml of isovaleric anhydride was stirred at 110-145°C for 1h. The brown solution was diluted with hexane (500 ml) and cooled down. The precipitated gum was separated from the mixture and washed with hexane, dried in vacuo. 3-Methyl-5-(3-methyl-butyryl)-isoxazole-4-carboxylic acid amide was obtained as a yellow gum.Further used without purification in method 35.

### METHOD 35

### 6-Isobutyl-3-methyl -5H-isoxazolo[5,4-d]pyrimidin-4-one

A suspension of 3-methyl-5-(3-methyl-butyryl)-isoxazole-4-carboxylic acid amide (method 34) (split into 40 vials) in 3.5 ml of 2N NaOH aq was subjected to microwave irradiation at 140°C for 20min. The resulting solution was cooled with an ice bath, and the pH was adjusted to 1∼3 with concentrated HCl. The solid was filtered, washed with water, dried over vacuum at 40°C overnight. 6-Isobutyl-3-methyl-5*H*-isoxazolo[5,4-d]pyrimidin-4-one (8 g) was obtained as a white solid. 55% yield for two steps.
*m*/*z*: 208 (MH⁺), ¹H NMR (400MHz, DMSO-*d6*): 0.76 (d, 6H), 1.95 (m, 1H), 2.25 (s, 3H), 2.32 (d, 2H), 12.55 (s, 1H).

### METHOD 36

### 5-Benzyl-6-isobutyl-3-methyl-5H-isoxazolo[5,4-d]pyrimidin4-one

A suspension of 6-isobutyl-3-methyl-5*H*-isoxazolo[5,4-d]pyrimidin-4-one (method 35) (5 g, 24.4 mmol), benzylbromide (4.17 g, 24.4 mmol), potassium carbonate (6.7 g, 48.8 mmol) in 20 ml DMF was stirred at room temperature for 2 days. The mixture was diluted with water, extracted with ethyl acetate (100 ml × 3), the combined organic phases were dried over anhydrous sodium sulfate, concentrated, purified by flash column chromatography (elute: hexane-ethyl acetate = 7:1). 5-benzyl-6-isobutyl-3-methyl-5*H*-isoxazolo[5,4-d]pyrimidin-4-one was obtained as white solid (3 g, 10.1 mmol) (41%).
*m*/*z*: 298 (MH⁺), ¹H NMR (400MHz, DMSO-*d6*): 0.90 (d, 6H), 2.30 (m, 1H), 2.55 (s, 3H), 2.75 (d, 2H), 5.42 (s, 2H), 7.22-7.43 (m, 5H).

The following compounds were synthesized according to Method 36:

| **Method #** | **Compound Name** | ***m*/*z*** |
|---|---|---|
| **36a** | 5-(4-Fluoro-benzyl)-6-isobutyl-3-methyl-5H-isoxazolo[5,4-d]pyrimidin-4-one | 316 (MH⁺) |

### METHOD 37

### 5-Benzyl-6-(1-bromo-2-methyl-propyl)-3-methyl-5H-isozazolo[5,4-d]pyrimidin-4-one

A solution of 5-benzyl-6-isobutyl-3-methyl-5*H*-isoxazolo[5,4-d]pyrimidin-4-one (method 36) (130 mg, 0.44 mmol) and sodium acetate (90 mg, 1.09 mmol, 2.5 eq) in glacial acetic acid (2 ml) was treated with a preformed bromine solution (0.7 ml bromine in 10 ml of glacial acetic acid) (1.54 ml, 2 mmol). The mixture was stirred at 110-120°C for 1 day. Excess bromine (1.54 ml, 2 mmol) was added to the mixture every 4 hours for two times at 110-120°C. Water was added to the mixture to which was subsequently added potassium carbonate and extracted with methylene chloride (20 ml × 3), the combined organic phases were washed with water and dried over anhydrous sodium sulfate, then concentrated to give the product which was purified by ISCO (elute: hexane-ethyl acetate). 100 mg (60%) of 5-benzyl-6-(1-bromo-2-methylpropyl)-3-methyl-5*H-*isoxazolo[5,4-d]pyrimidin-4-one was obtained as a yellow gum.
*m*/*z*: 377 (MH⁺), ¹H NMR (400MHz, DMSO-*d6*): 0.55 (d, 3H), 1.02 (d, 3H), 2.48 (m, 4H), 4.75 (d, 1H), 5.60 (d, 1H), 5.70 (d, 1H), 7.16-7.30 (m, 5H).

The following compounds were synthesized according to Method 37:

| **Method #** | **Compound Name** | ***m*/*z*** |
|---|---|---|
| **37a** | 6-(1-Bromo-2-methyl-propyl)-5-(4-fluoro-benzyl)-3-methyl-5H-isoxazolo[5,4-d]pyrimidin-4-one | 396 (MH⁺) |

### METHOD 38

### 6-(1-Azido-2-methyl-propyl)-5-benzyl-3-methyl-5H-isoxazolo[5,4-d]pyrimidin-4-one

A suspension of 5-benzyl-6-(1-bromo-2-methyl-propyl)-3-methyl-5*H*-isoxazolo[5,4-d]pyrimidin-4-one (method 37) (100 mg, 0.266 mmol) and sodium azide (34.5 mg, 0.53 mmol) in DMF (2 ml) was stirred at 60°C for 1h. Water (5 ml) was added to the mixture and then extracted with ethyl acetate (3 × 20 ml). The combined organic phases were washed with brine (10 ml), dried over sodium sulfate, concentrated to obtain 6-(1-azido-2-methyl-propyl)-5-benzyl-3-methyl-5*H-*isoxazolo[5,4-d]pyrimidin-4-one which was purified by ISCO (Hexane-Ethyl acetate). 50mg (56%) of a colorless oil was obtained.
*m*/*z*: 339 (MH⁺), ¹H NMR (400MHz, DMSO-*d6*): 0.60 (d, 3H), 0.95 (d, 3H), 2.25 (m, 1H), 2.45 (s, 3H), 4.19 (d, 1H), 5.30 (d, 1H), 5.42 (d, 1H), 7.12-7.30 (m, 5H).

The following compounds were synthesized according to Method 38:

| **Method #** | **Compound Name** | ***m*/*z*** |
|---|---|---|
| **38a** | 6-(1-Azido-2-methyl-propyl)-5-(4-fluoro-benzyl)-3-methyl-5H-isoxazolo[5,4-d]pyrimidin-4-one | 357 (MH⁺) |

### METHOD 39

### 6-(1-Amino-2-methyl-propyl)-5-benzyl-3-methyl-5H-isozazolo[5,4-d]pyrimidin-4-one

A mixture of 6-(1-azido-2-methyl-propyl)-5-benzyl-3-methyl-5*H-*isoxazolo[5,4-d]pyrimidin-4-one (method 38) (40 mg, 1.118 mmol), triphenylphosphine (62 mg, 0.237 mmol) and water (4 µl) in THF was stirred at 60°C for 5 hours. Excess amount of water (30 µl) was added to the mixture and stirred at 60°C for another 10 hours. The volatile solvent was distilled out, the product was purified by ISCO (Ethyl acetate : hexane = 60%. 25 mg (68%) of 6-(1-amino-2-methyl-propyl)-5-benzyl-3-methyl-5H-isoxazolo[5,4-d]pyrimidin4-one was obtained as colorless oil.
*m*/*z*: 313 (MH⁺), ¹H NMR (400MHz, DMSO-*d6*): 0.55 (d, 3H), 0.95 (d, 3H), 2.02 (m, 1H), 2.15 (br, 2H), 2.55 (s, 3H), 3.59 (d, 1H), 5.38 (d, 1H), 5.65 (d, 1H), 7.25-7.42 (m, 5H).

The following compounds were synthesized according to Method 39:

| **Method #** | **Compound Name** | ***m*/*z*** |
|---|---|---|
| **39a** | 6-(1-Amino-2-methyl-propyl)-5-(4-fluoro-benzyl)-3-methyl-5H-isoxazolo[5,4-d]pyrimidin-4-one | 331 (MH⁺) |

### METHOD 40

### {3-[1-(5-Benzyl-3-methyl-4-oxo-4,5-dihydro-isoxazolo[5,4-d]pyrimidin-6-yl)-2-methylpropylamino]-propyl}-carbamic acid tert-butyl ester

A mixture of 6-(1-amino-2-methyl-propyl)-5-benzyl-3-methyl-5H-isoxazolo [5,4-d]pyrimidin-4-one (method 39) (20 mg, 0.064 mmol) and (3-oxo-propyl)-carbamic acid tert-butyl ester (11 mg, 0.064 mmol) in methylene chloride (5 ml) with dried 4ÅMS was stirred for 1h at room temperature. Then sodium triacetoxyborohydride (2eq) and 1 drop of acetic acid were added to the mixture. The mixture was stirred at room temperature for 1 day. The mixture was filtered through a 2µ cartridge, the filtrate was concentrated, the mixture was purified by ISCO (elute: ethyl acetate-hexane = 30% ∼ 60%) to give 18 mg (60%) of {3-[1-(5-benzyl-3-methyl-4-oxo-4,5-dihydro-isoxazolo[5,4-d]pyrimidin-6-yl)-2-methyl-propylamino]-propyl}-carbamic acid tert-butyl ester as a white solid.
*m*/*z*: 470 (MH⁺), ¹H NMR (400 MHz, DMSO-d*₆*): 0.65 (d, 3H), 0.80 (d, 3H), 1.10 (m, 2H), 1.25 (s, 9H), 1.32 (d, 1H), 1.70-1.90 (m, 2H), 2.18 (m, 1H), 2.49 (s, 3H), 2.70 (m, 2H), 3.48 (d, 1H), 5.15 (d, 1H), 5.51 (d, 1H), 6.55 (br, 1H), 7.12-7.32 (m, 5H).

The following compounds were synthesized according to Method 40:

| **Method #** | **Compound Name** | ***m*/*z*** |
|---|---|---|
| **40a** | (3-{1-[5-(4-Fluoro-benzyl)-3-methyl-4-oxo-4,5-dihydro-isoxazolo[5,4-d]pyrimidin-6-yl]-2-methyl-propylamino}-propyl)-carbamic acid tert-butyl ester | 488 (MH⁺) |

### METHOD 41

### {3-[[1-(5-Benzyl-3-methyl-4-oxo-4,5-dihydro-isoxazolo[5,4-d]pyrimidin-6-yl)-2-methylpropyl]-(4-methyl-benzoyl)-amino]-propyl}-carbamic acid tert-butyl ester

A solution of {3-[1-(5-benzyl-3-methyl-4-oxo-4,5-dihydro-isoxazolo[5,4-d]pyrimidin-6-yl)-2-methyl-propylamino]-propyl}-carbamic acid tert-butyl ester (method 40) (100 mg, 0.213 mmol) in dichloromethane (4 ml) was added toluoyl chloride (66mg, 0.426 mmol) followed by triethylamine (65 mg, 0.639 mmol). The mixture was stirred at 30-40°C for 2 days. The mixture was then diluted with dichloromethane, washed with saturated sodium bicarbonate aq. The organic phase was dried over sodium sulfate, filtered, and concentrated. The crude oil was purified by ISCO (solvent: ethyl acetate-hexane) to give {3-[[1-(5-benzyl-3-methyl-4-oxo-4,5-dihydro-isoxazolo[5,4-d]pyrimidin-6-yl)-2-methyl-propyl]-(4-methyl-benzoyl)-amino]-propyl}-carbamic acid tert-butyl ester as white solid (115mg, 0.196 mmol). *m*/*z*: 588 (MH⁺)

The following compounds were synthesized according to Method 41:

| **Method #** | **Compound Name** | ***m*/*z*** | **SM** | **Acylating** agent |
|---|---|---|---|---|
| **41a** | {3-[{1-[5-(4-Fluoro-benzyl)-3-methyl-4-oxo-4,5-dihydro-isoxazolo[5,4-d]pyrimidin-6-yl]-2-methyl-propyl}-(4-methyl-benzoyl)-amino]-propyl}-carbamic acid tert-butyl ester | 606 (MH⁺) | Method 40a | 4-methyl-benzoylchloride |
| **41b** | {3-[[1-(5-Benzyl-3-methyl-4-oxo-4,5 -dihydro-isoxazolo[5,4-d]pyrimidin-6-yl)-2-methyl-propyl]-(3-fluoro-4-methyl-benzoyl)-amino]-propyl}-carb amic acid tert-butyl ester | 606 (MH⁺) | Method 40 | 3-Fluoro-4-methyl-benzoyl chloride |
| **41c** | {3-[{1-[5-(4-Fluoro-benzyl)-3-methy 1-4-oxo-4,5-dihydro-isoxazolo[5,4-d]pyrimidin-6-yl]-2-methyl-propyl}-(3-fluoro-4-methyl-benzoyl)-amino]-propyl}-carbamic acid tert-butyl ester | 624 (MH⁺) | Method 40a | 3-Fluoro-4-methyl-benzoyl chloride |

### METHOD 42

### N-(3-Amino-propyl)-N-[1-(5-benzyl-3-methyl-4-oxo-4,5-dihydro-isoxazolo[5,4-d]pyrimidin-6-yl)-2-methyl-propyl]-4-methyl-benzamide

A solution of {3-[[1-(5-benzyl-3-methyl-4-oxo-4,5-dihydro-isoxazolo[5,4-d]pyrimidin-6-yl)-2-methyl-propyl]-(4-memyl-benzoyl)-amino]-propyl}-carbamic acid tert-butyl ester (method 41) (0.2 mmol) in 3 ml of 4 M HCl in dioxane was stirred at room temperature for 2hr. The solvent was distilled off by vacuo, the residue was dried at 40∼50°C for overnight under vacuum. N-(3-Amino-propyl)-N-[1-(5-benzyl-3-methyl-4-oxo-4,5-dihydro-isoxazolo[5,4-d]pyrimidin-6-yl)-2-methyl-propyl]-4-methyl-benzamide was obtained as the HCl salt. *m*/*z* 488 (MH⁺), ¹H NMR (500 MHz, 100°C, DMSO-d₆): 0.48 (d, 3H), 0.94 (d, 3H), 1.30 (m, 1H), 1.60 (m, 1H), 2.35 (m, 2H), 2.38 (s, 3H), 2.58 (s, 3H), 2.70 (m, 1H), 3.37 (m, 2H), 5.11 (d, 1H), 5.64 (d, 1H), 5.90 (d,1H), 7.23-7.39 (m, 9H), 7.63 (br, 3H).

The following compounds were synthesized according to Method 42:

| **Method #** | **Compound Name** | ***m*/*z*** |
|---|---|---|
| **42a** | N-(3-Amino-propyl)-N-{1-[5-(4-fluoro-benzyl)-3-methyl-4-oxo-4,5-dihydro-isoxazolo[5,4-d]pyrimidin-6-yl]-2-methyl-propyl} -4-methyl-benzamide | 506 (MH⁺) |
| **42b** | N-(3-Amino-propyl)-N-[1-(5-benzyl-3-methyl-4-oxo-4,5-dihydro-isoxazolo[5,4-d]pyrimidin-6-yl)-2-methyl-propyl]-3-fluoro-4-methyl-benzamide | 506 (MH⁺) |
| **42c** | N-(3-Amino-propyl)-3-fluoro-N-{1-[5-(4-fluoro-benzyl)-3-methyl-4-oxo-4,5-dihydro-isoxazolo[5,4-d]pyrimidin-6-yl]-2-methyl-propyl}-4-methyl-benzamide | 524 (MH⁺) |

### EXAMPLE A-1

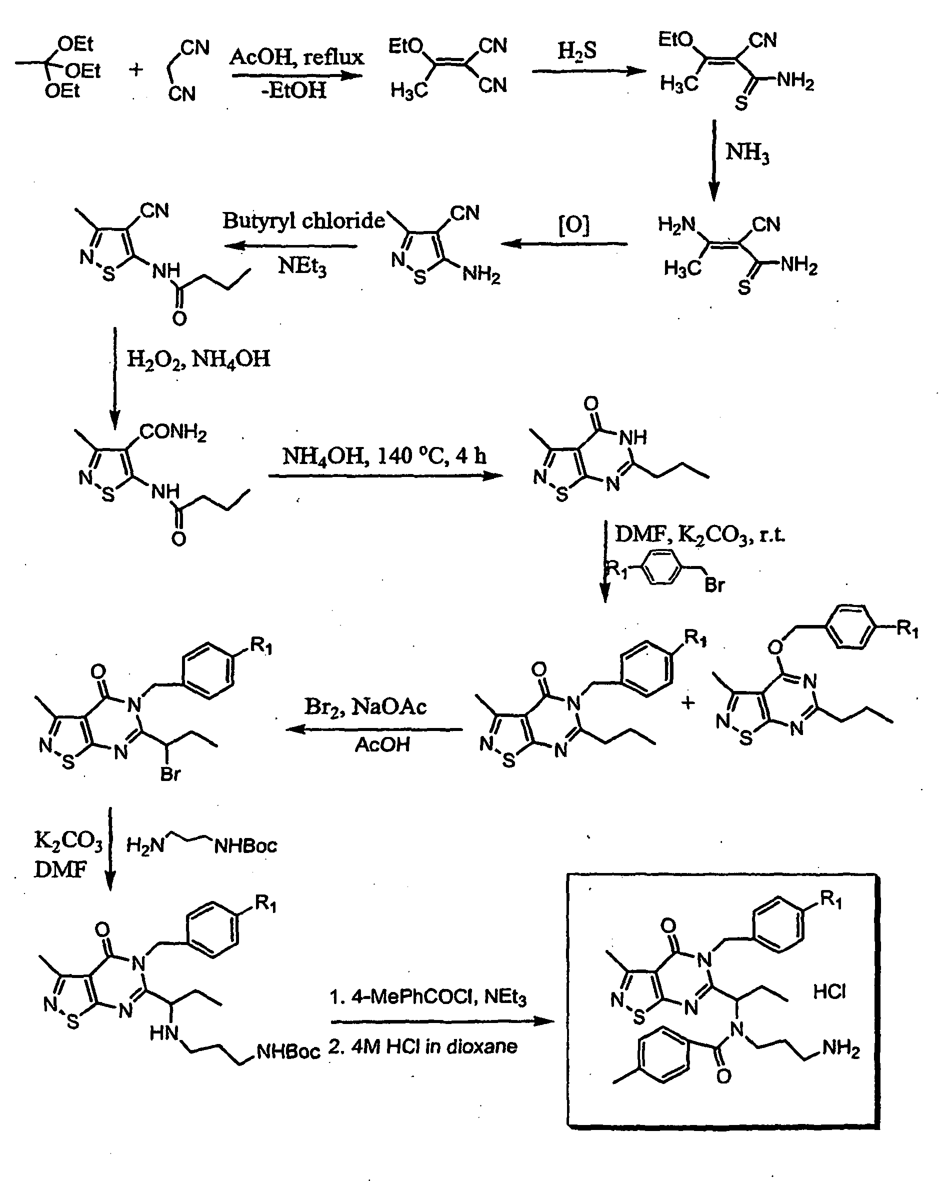

### EXAMPLES A1

The following compounds were synthesized according to synthetic scheme A-1 above:

| **Ex.** | **Compound** | **¹H NMR** | ***m*/*z*** | **SM** |
|---|---|---|---|---|
| **A1-1** | *N*-(3-Amino-propyl)-*N*-[1-(5-benzyl-3-methyl-4-oxo-4,5-dihydro-isothiazolo-[5,4-*d*]pyrimidin-6-yl)-propyl]-4-methyl-benzamide hydrogen chloride | ¹H NMR (DMSO-d6 300MHz, 96 °C) δ: 7.79 (bs, 3H), 7.37-6.95 (m, 9H), 5.77 (d, 1H), 5.50 (bs, 1H), 4.83 (d, 1H), 3.36 (t, 2H), 2.72 (s, 3H), 2.46 (t, 2H), 2.39 (s, 3H), 2.20-2.05 (m, 1H), 1.96-1.75 (m, 1H), 1.74-1.40 (m, 2H), 0.63 (t, 3H) | *m*/*z* 490 (MH⁺) | Method 11 |
| **A1-2** | N-(3-Amino-propyl)-N-{1-[5-(4-fluoro-benzyl)-3-methyl-4-oxo-4,5-dihydro-isothiazolo[5,4-d]pyrimidin-6-yl]-propyl}-4-methyl-benzamide hydrogen chloride | ¹H NMR (DMSO-d6 300MHz, 96 °C) δ: 7.76 (bs, 3H), 7.27-7.05 (m, 8H), 5.70 (d, 1H), 5.49 (bs, 1H), 4.85 (d, 1H), 3.36 (t, 2H), 2.99 (s, 1H), 2.72 (s, 3H), 2.42 (t, 2H), 2.34 (s, 3H), 2.20-2.05 (m, 1H), 1.98-1.82 (m, 1H), 1.74-1.38 (m, 2H), 0.66 (t, 3H) | *m*/*z* 507 (MH⁺) | Method 11 |

### EXAMPLE A-2

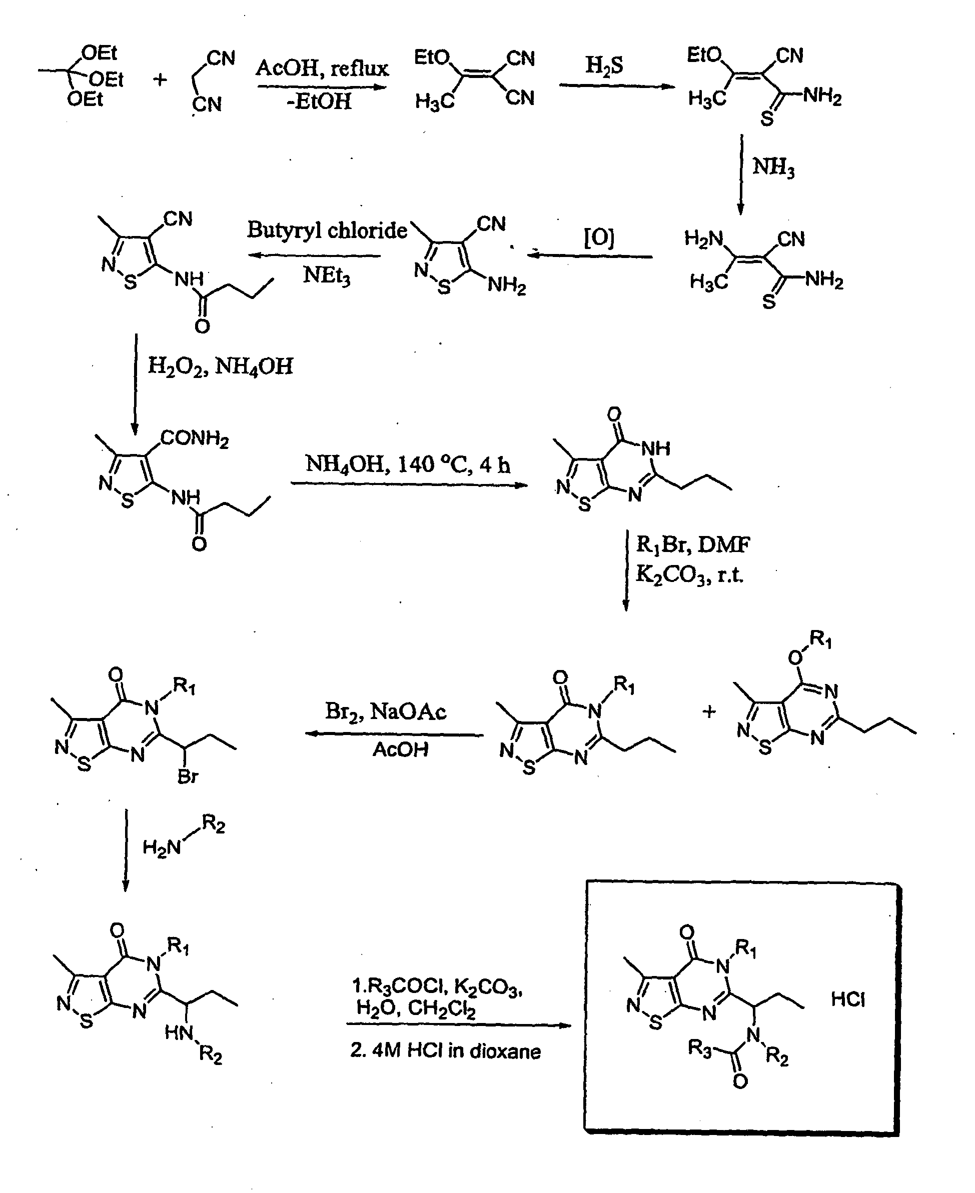

### EXAMPLES A2

The following compounds were synthesized according to synthetic scheme A-2 above:

| **Ex.** | **Compound** | **¹H NMR** | ***m*/*z*** | **SM** |
|---|---|---|---|---|
| **A2-1** | N-(3-Amino-propyl)-N-[1-(5-benzyl-3-methyl-4-oxo-4,5-dihydro-isothiazolo[5,4-d]pyrimidin-6-yl)-propyl]-4-bromo-benzamide hydrogen chloride | ¹H NMR (DMSO-d6 500MHz, 96 °C) δ: 7.80 (br, 3H), 7.64 (d, 2H), 7.36-7.28 (m, 5H), 7.13 (m, 2H), 5.80 (d, 1H), 5.57 (bs, 1H), 4.95 (d, 1H), 3.38 (t, 2H), 2.77 (s, 3H), 2.47 (t, 2H), 2.17-2.13 (m, 1H), 1.96-1.91 (m, 1H),1.72-1.50 (m, 2H), 0.68 (t,3H) | *m*/*z* 556 (MH⁺) | Method 13 |
| **A2-2** | N-(3-Amino-propyl)-N-[1-(5-benzyl-3-methyl-4-oxo-4,5-dihydro-isothiazolo[5,4-d]pyrimidin-6-yl)-propyl]-3-fluoro-4-methyl-benzamide hydrogen chloride | ¹H NMR (DMSO-d6 300MHz, 96 °C) δ: 7.52-7.09 (m, 9H), 5.81 (d, 1H), 5.54 (bs, 1H), 4.93 (d, 1H), 3.39 (t, 2H), 2.76 (s, 3H), 2.46 (2H, hidden by DMSO), 2.31 (s, 3H), 2.16 (m, 1H), 1.92 (m, 1H), 1.70 (m, 1H), 1.45 (m, 1H), 0.67 (t, 3H) | *m*/*z* 508 (MH⁺) | Method 13 |
| **A2-3** | N-(3-Amino-propyl)-N-[1-(5-benzyl-3-methyl-4-oxo-4,5-dihydro-isothiazolo[5,4-d]pyrimidin-6-yl)-propyl]-2,3-dichloro-benzamide hydrogen chloride | ¹H NMR (DMSO-d6 300MHz, 96 °C) δ: 7.72-7.29 (m, 9H), 5.93 (d, 1H), 5.82 (bs, 1H), 5.10 (d, 1H), 3.30 (m, 2H), 2.78 (s, 3H), 2.46 (m, 2H), 2.20 (m, 1H), 1.94 (m, 1H), 1.70 -1.50 (m, 2H), 0.70 (t, 3H) | *m*/*z* 545 (MH⁺) | Method 13 |
| **A2-4** | Naphthalene-2-carboxylic acid(3-amino-propyl)-[1-(5-benzyl-3-methyl-4-oxo-4,5-dihydro-isothiazolo[5,4-d]pyrimidin-6-yl)-propyl]-amide hydrogen chloride | ¹H NMR (DMSO-d6 300MHz, 96 °C) δ: 8.00-7.98 (m, 3H), 7.87 (s, 1H), 7.63-7.61 (m, 2H), 7.45 (d, 1H), 7.27 (m, 3H), 7.10 (m, 2H), 5.83 (d, 1H), 5.67 (bs, 1H), 4.98 (d, 1H), 3.46 (m, 2H), 2.77 (s, 3H), 2.40 (m, 2H), 2.24 (m, 1H), 2.04 (m, 1H), 1.70 (m, 1H), 1.45 (m, 1H), 0.74 (t, 3H) | *m*/*z* 526 (MH⁺) | Method 13 |
| **A2-5** | Benzo[b]thiophene-2-carboxylic acid (3-amino-propyl)-[1-(5-benzyl-3-methyl-4-oxo-4,5-dihydro-isothiazolo[5,4-d]pyrimidin-6-yl)-propyl]-amide hydrogen chloride | ¹H NMR (DMSO-d6 300MHz, 96 °C) δ: 7.99-7.85 (m, 3H), 7.60 (s, 1H), 7.50-7.30 (m, 3H), 7.25 (bm, 2H), 7.05 (bm, 1H), 5.78 (d, 1H), 5.66 (bs, 1H), 4.96 (d, 1H), 3.65 (t, 2H), 2.76 (s, 3H), 2.62 (t, 2H), 2.23-2.21 (m, 1H), 1.97-1.93 (m, 1H), 1.92-1.88 (m, 1H), 1.61-1.46 (m, 1H), 0.713 (t, 3H) | *m*/*z* 532 (MH⁺) | Method 13 |
| **A2-6** | N-Azetidin-3-ylmethyl-N-[1-(5-benzyl-3-methyl-4-oxo-4,5-dihydro-isothiazolo[5,4 d]pyrimidin-6-yl)-propyl] -4-methyl-benzamide | ¹H NMR (DMSO-d6 500MHz, 96 °C) δ: 7.75-7.20 (m, 9H), 5.50 (m, 1H), 5.38 (m, 1H), 4.25 (m, 2H), 4.00 (m, 1H), 3.10 (t, 2H), 2.75 (s, 3H), 2.73 (m, 1H), 2.39 (s, 3H), 2.32 (m, 1H), 1.85-1.75 (m, 1H), 1.60-1.50 (m, 1H), 0.82 (t, 3H) | *m*/*z* 502 (MH⁺) | Method 13 |
| **A2-7** | N-[1-(5-Benzyl-3-methyl-4-oxo-4,5-dihydro-isothiazolo[5,4-d]pyrimidin-6-yl)-propyl]-4-methyl-N-piperidin-3-ylmethyl-benzamide | ¹H NMR (DMSO-d6 300MHz, 96 °C) δ: 7.31-6.94 (m, 9H), 5.81 (m, 1H), 5.32 (bs, 1H), 4.72 (bm, 1H), 3.05 (bm, 2H), 2.76 (s, 3H), 2.65 (bm, 2H), 2.37 (s, 3H), 2.25 (m, 2H), 1.90 (m, 32H), 1.78 (m, 3H), 1.55 (m, 1H), 0.65 (t, 3H) | *m*/*z* 530 (MH⁺) | Method 13 |
| **A2-8** | N-(2-Amino-ethyl)-N-[1-(5-benzyl-3-methyl-4-oxo-4,5-dihydro-isothiazolo[5,4-d]pyrimidin-6-yl)-propyl]-4-methyl-benzamide | ¹H NMR (DMSO-d6 500MHz, 96 °C) δ: 7.35-6.88 (m, 9H), 5.80 (d, 1H), 5.35 (bs, 1H), 4.70 (d, 1H), 3.80 (m, 2H), 2.90 (m, 2H), 2.76 (s, 3H), 2.45 (s, 3H), 2.05-1.85 (m, 2H), 0.60 (t, 3H) | *m*/*z* 476 (MH⁺) | Method 13 |
| **A2-9** | N-[1-(5-Benzyl-3-methyl-4-oxo-4,5-dihydro-isothiazolo[5,4-d]pyrimidin-6-yl)-propyl]-N-(2-dimethylamino-ethyl)-4-methyl-benzamide | ¹H NMR (DMSO-d6 500MHz, 96 °C) δ: 7.35-6.88 (m, 9H), 5.80 (d, 1H), 5.40 (bs, 1H), 4.80 (d, 1H), 3.75 (m, 2H), 3.00 (m, 1H), 2.80 (s, 3H), 2.70 (m, 1H), 2.52-2.50 (2s, 6H), 2.35 (s, 3H), 2.15-1.98 (m, 2H), 0.70 (t, 3H) | *m*/*z* 504 (MH⁺) | Method 14 |
| **A2-10** | N-[1-(5-Benzyl-3-methyl-4-oxo-4,5-dihydro-isothiazolo[5,4-d]pyrimidin-6-yl)-propyl]-N-(3-dimethylamino-propyl)-4-methyl-benzamide | ¹H NMR (DMSO-d6 300MHz, 96 °C) δ: 7.44-7.00 (m, 9H), 5.82 (d, 1H), 5.51 (bs, 1H), 4.86 (d, 1H), 3.41 (t, 2H), 2.75 (s, 3H), 2.50 (s, 6H), 2.39 (bm, 2H), 2.12-2.05 (m, 1H), 1.93-1.90 (m, 1H), 1.75 (m, 1H), 1.50 (m, 1H), 0.66 (t, 3H) | *m*/*z* 518 (MH⁺) | Method 14 |

### EXAMPLE B

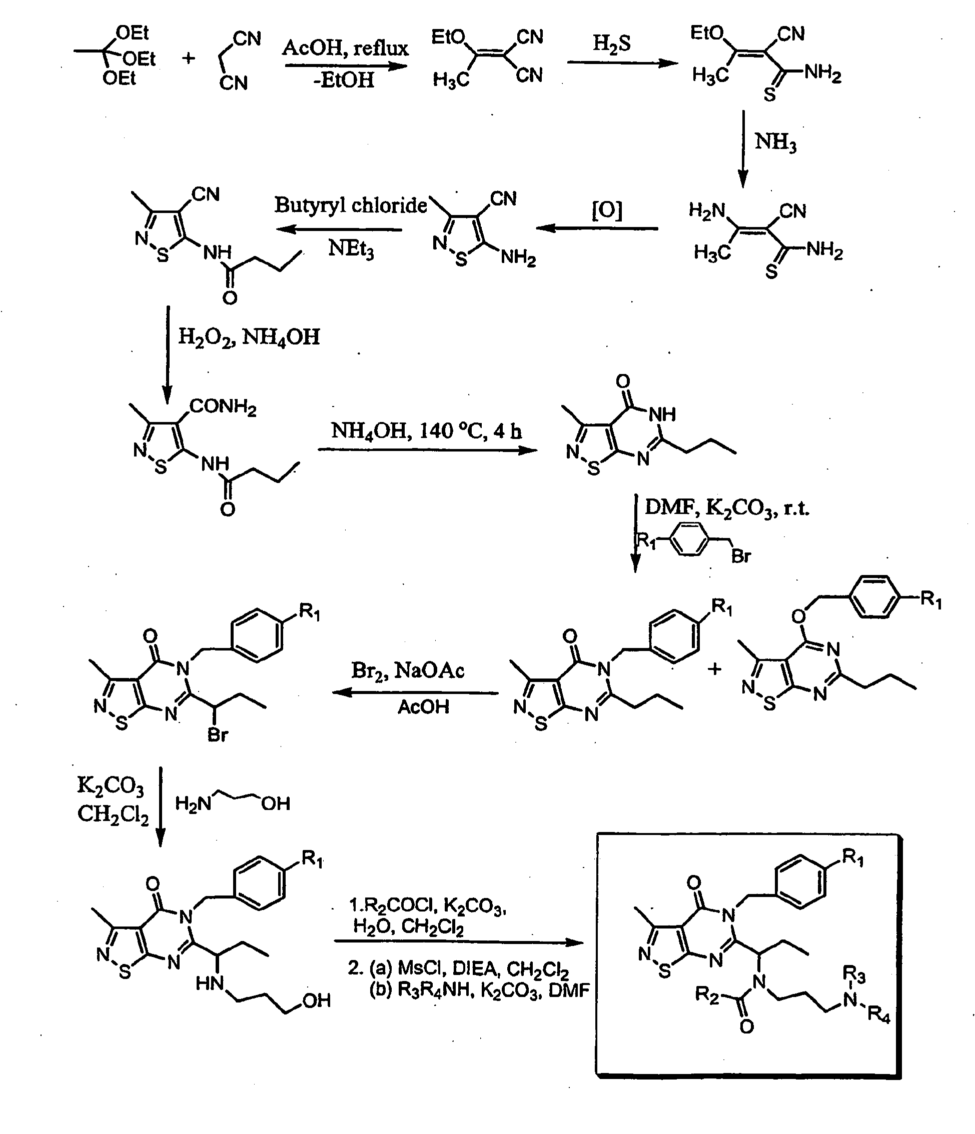

The following compounds were synthesized according to Example B above:

| **Ex.** | **Compound** | **¹H NMR** | ***m*/*z*** | **SM** |
|---|---|---|---|---|
| **B-1** | *N*-[1-(5-benzyl-3-methyl-4-oxo-4,5-dihydroisothiazolo-[5,4-*d*]pyrimidin-6-yl)propyl]-N-[3-(isopropylamino)propyl]-4-methylbenzamide | ¹H NMR (DMSO-d6 400MHz, 96 °C) δ: 7.35-7.00 (m, 9H), 5.80 (d, 1H), 5.50 (bs, 1H), 4.90 (d, 1H), 3.40 (t, 2H), 3.05 (b, 1H), 2.80 (s, 3H), 2.63 (b, 2H), 2.35 (s, 3H), 2.20-2.05 (m, 1H), 2.00-1.85 (m, 1H), 1.74-1.70 (m, 1H), 1.60-1.40 (m, 1H), 1.10 (d, 6H), 0.65 (t, 3H) | *m*/*z* 532 (MH⁺) | Method 16 |
| **B-2** | *N-*[1-(5-benzyl-3-methyl-4-oxo-4,5-dihydroisothiazolo-[5,4-*d*]pyrimidin-6-yl)propyl]-N-[3-(cyclopropylamino)propyl]-4-methylbenzamide | ¹H NMR (DMSO-d6 400MHz, 96 °C) δ: 7.45-7.00 (m, 9H), 5.80 (d, 1H), 5.52 (bs, 1H), 4.85 (d, 1H), 3.43 (b, 2H), 2.78 (s, 3H), 2.65 (b, 2H), 2.35 (s, 3H), 2.20-1.15 (various m, 9H), 0.65 (t, 3H) | *m*/*z* 530 (MH⁺) | Method 16 |
| **B-3** | *N-*(3-azetidin-1-ylpropyl)-N-[1-(5-benzyl-3-methyl-4-oxo-4,5-dihydroisothiazolo-[5,4-*d*]pyrimidin-6-yl)propyl] -4-methylbenzamide | ¹H NMR (DMSO-d6 400MHz, 96 °C) δ: 7.40-7.00 (m, 9H), 5.85 (d, 1H), 5.55 (bs, 1H), 4.85 (d, 1H), 3.40 (b, 2H), 2.90 (b, 2H), 2.78 (s, 3H), 2.50 (b, 2H), 2.40 (s, 3H), 2.35 (bm, 2H), 2.20-2.00 (m, 1H), 1.96-1.80 (m, 1H), 1.65.1.50 (m, 1H), 1.40-1.30 (m, 3H), 0.65 (t, 3H) | *m*/*z* 530 (MH⁺) | Method 16 |
| **B-4** | *N*-[1-(5-benzyl-3-methyl-4-oxo-4,5-dihydroisothiazolo-[5,4-*d*]pyrimidin-6-yl)propyl] -4-methyl-N-[3-(3-pyrrolidin-1-ylpropyl) benzamide | ¹H NMR (DMSO-d6 500MHz, 96 °C) δ: 7.35-7.07 (m, 9H), 5.82 (d, 1H), 5.57 (bs, 1H), 4.91 (d, 1H), 3.60 (b, 2H), 2.76 (s, 3H), 2.50 (6H hidden by DMSO), 2.38 (s, 3H), 2.20-2.10 (m, 1H), 2.00-1.65 (various m, 6H), 1.55-1.40 (m, 1H), 0.66 (1, 3H) | *m*/*z* 544 (MH⁺) | Method 16 |
| **B-5** | *N-*[1-(5-benzyl-3-methyl-4-oxo-4,5-dihydroisothiazolo-[5,4-*d*]pyrimidin-6-yl)propyl]-4-methyl-N-[3-(methylamino) propyl] benzamide | ¹H NMR (DMSO-d6 400MHz, 96 °C) δ: 7.50-6.85 (m, 9H), 5.74 (d, 1H), 5.30 (bs, 1H), 4.60 (d, 1H), 3.80 (t, 2H), 2.82 (s, 3H), 2.60 (s, 3H), 2.41 (s, 3H), 2.39-2.00 (m, 2H), 1.99-1.80 (m, 2H), 0.62 (t, 3H) | *m*/*z* 504 (MH⁺) | Method 16 |

### EXAMPLE C

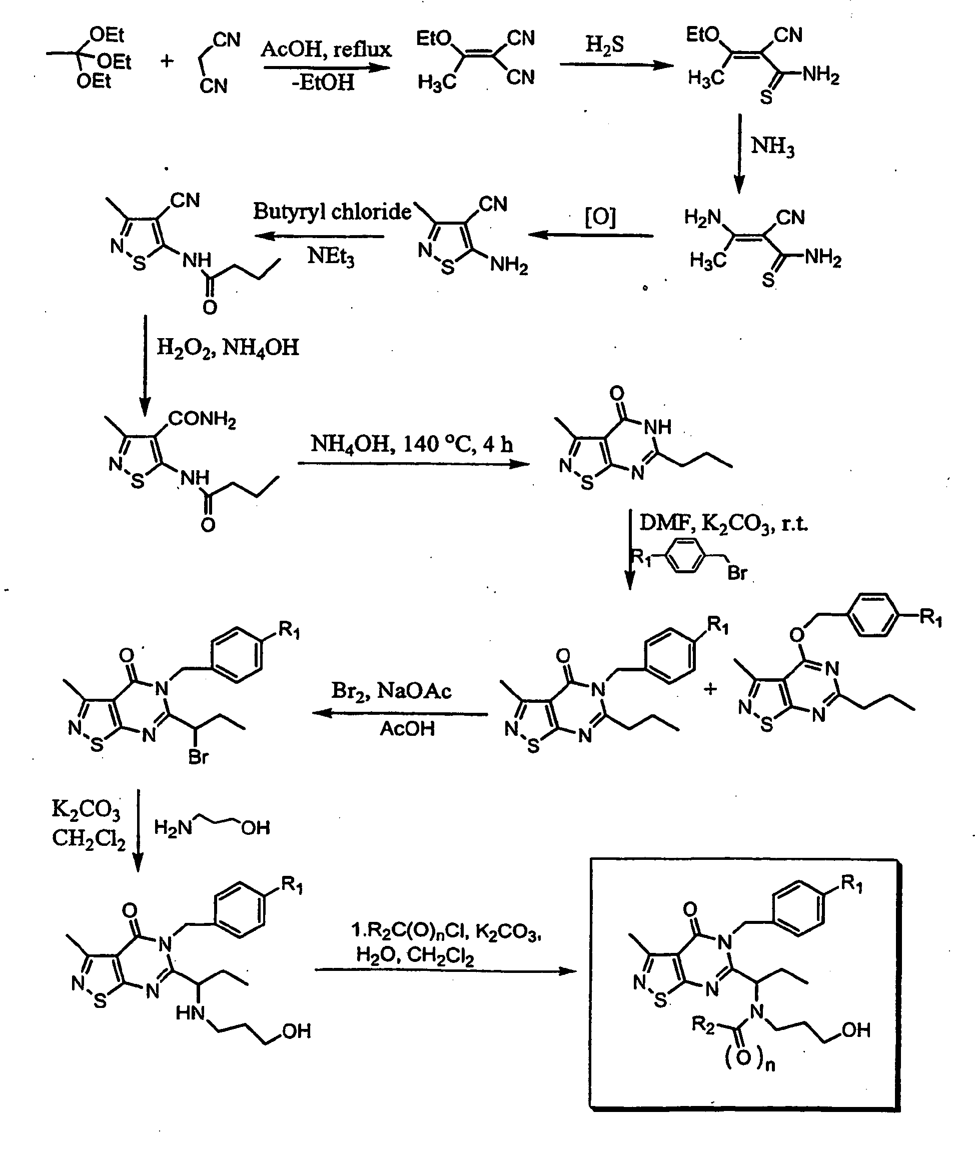

The following compounds were synthesized according to synthetic scheme C above:

| **Ex.** | **Compound** | **¹H NMR** | ***m*/*z*** | **SM** |
|---|---|---|---|---|
| **C-1** | N-[1-(5-Benzyl-3-methyl-4-oxo-4,5-dihydro-isothiazolo[5,4-d]pyrimidin-6-yl)-propyl]-N-(3-hydroxy-propyl)-4-methyl-benzamide | ¹H NMR (DMSO-d6 500MHz, 96 °C) δ: 7.40-7.10 (m, 9H), 5.85 (d, 1H), 5.69 (bs, 1H), 5.00 (d, 1H), 3.37 (t, 2H), 3.05 (bm, 2H, 2.77 (s, 3H), 2.52 (s, 1H), 2.40 (s, 3H), 2.16 (m, 1H), 1.94 (m, 1H), 1.50-1.40 (m, 1H), 1.20-1.10 (m, 1H),0.71 (t, 3H) | *m*/*z* 491 (MH⁺) | Method 14 |
| **C-2** | 5-Benzyl-6-{1-[(3-hydroxy-propyl)-(4-methyl-benzyl)-amino]-propyl}-3-methyl-5H-isothiazolo[5,4-d]pyrimidin-4-one | ¹H NMR (DMSO-d6 500MHz, 96 °C) δ: 7.40-6.85 (m, 9H), 5.80 (d, 1H), 5.20 (d, 1H), 3.80 (d, 1H), 3.70 (m, 1H), 3.62 (d, 1H), 3.50-3.30 (m, 2H), 2.90 (m, 1H), 2.75 (s, 3H), 2.33 (m, 2H), 2.25 (s, 3H), 2.20-2.16 (m, 1H), 1.90-1.80 (m, 1H), 1.50 (m, 2H), 0.65 (t, 3H) | *m*/*z* 477 (MH⁺) | Method 17 |

### EXAMPLE D

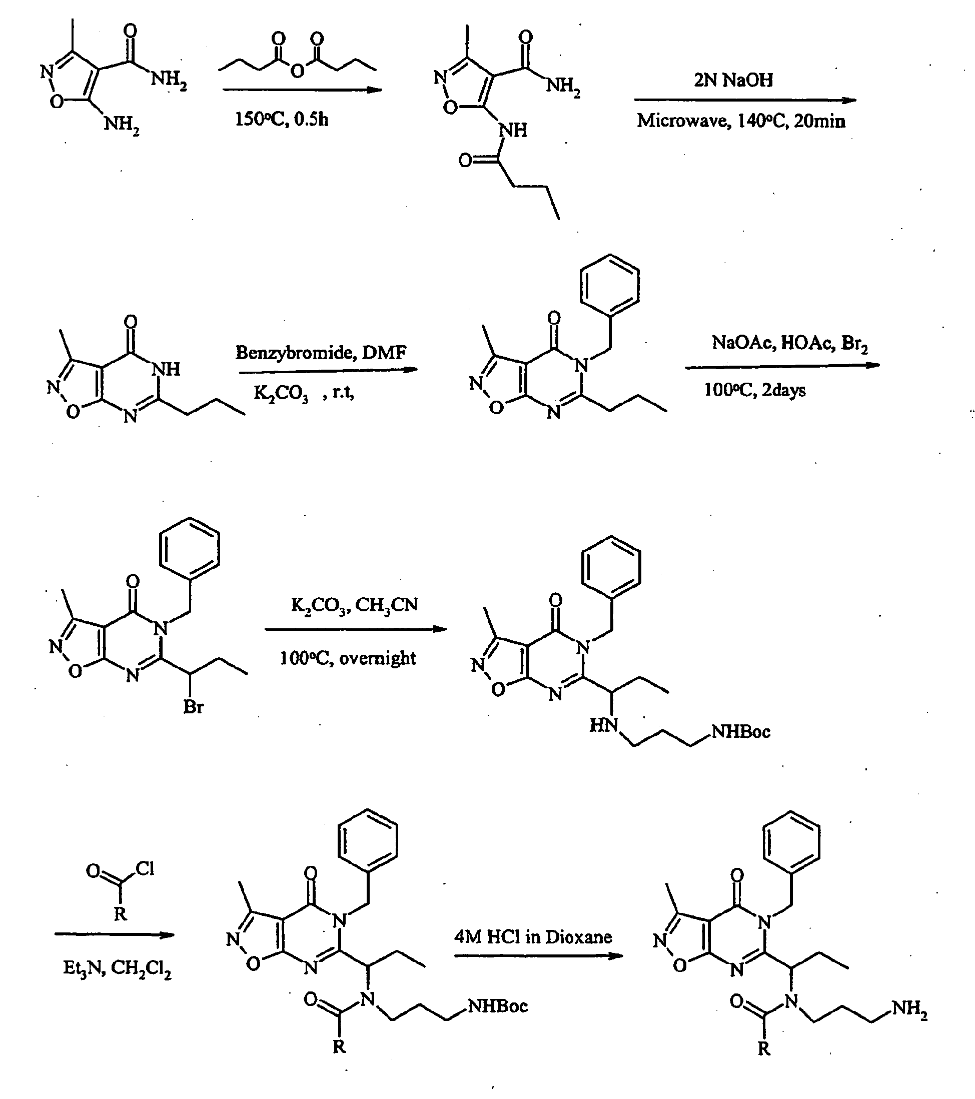

The following compounds were synthesized according to synthetic scheme D above:

| **Ex.** | **Compound** | **¹H NMR** | ***m*/*z*** | **SM** |
|---|---|---|---|---|
| **D-1** | N-(3-Amino-propyl)-N-[1-(5-benzyl-3-methyl-4-oxo-4,5-dihydro-isoxazolo[5,4-d]pyrimidin-6-yl)-propyl]-4-fluoro-benzamide hydrogen chloride | ¹H NMR (500 MHz, 100°C, DMSO-d₆) δ: 7.85 (br, 3H), 7.13-7.42 (m, 9H), 5.78 (d, J=16.2Hz, 1H), 5.55 (br, 1H), 4.98 (d, J=16.2Hz, 1H), 3.39 (m, 2H), 2.57 (s, 3H), 2.48 (m, 2H), 2.11 (m, 1H), 1.94 (m, 1H), 1.72 (m, 1H), 1.51 (m, 1H), 0.70 (t, 3H) | *m*/*z* 478 (MH⁺) | Method 24 |
| **D-2** | N-(3-Amino-propyl)-N-[1-(5-benzyl-3-methyl-4-oxo-4,5-dihydro-isoxazolo[5,4-d]pyrimidin-6-yl)-propyl]-2,3-dichloro-benzamide hydrogen chloride | ¹H NMR (500 MHz, 100°C, DMSO-d*₆*): δ: 7.67-7.80 (m, 4H), 7.29-7.47 (m, 7H), 5.92 (d, 1H), 5.83 (br, 1H), 5.14 (d, 1H), 3.20 (m, 2H), 2.58 (s, 3H), 2.39 (m, 2H), 2.16 (m, 1H), 1.95 (m, 1H), 1.68 (m, 1H), 1.41 (m, 1H), 0.70 (br, 3H) | *m*/*z* 529 (MH⁺) | Method 24 |
| **D-3** | N-(3-Amino-propyl)-N-[1-(5-benzyl-3-methyl-4-oxo-4,5-dihydro-isoxazolo[5,4-d]pyrimidin-6-yl)-propyl]-3-fluoro-4-methyl-benzamide hydrogen chloride | ¹H NMR (500 MHz, 100°C, DMSO-d*₆*): δ: 7.77 (m, 3H), 7.31-7.37 (m, 4H), 7.06-7.12 (m, 4H), 5.77 (d, 1H), 5.50 (br, 1H), 4.90 (br, 1H), 3.40 (m, 2H), 2.57 (s, 3H), 2.49 (m, 2H), 2.31 (d, 3H), 2.13 (m, 1H), 1.94 (m, 1H), 1.73 (m, 1H), 1.51 (m, 1H), 0.69 (t, 3H) | *m*/*z* 492 (MH⁺) | Method 24 |
| **D-4** | N-(3-Amino-propyl)-N-[1-(5-benzyl-3-methyl-4-oxo-4,5-dihydro-isoxazolo[5,4-d]pyrimidin-6-yl)-propyl]-4-methoxy-benzamide hydrogen chloride | ¹H NMR (500 MHz, 100°C, DMSO-d*₆*): δ: 7.71 (br, 1H), 6.99-7.35 (m, 9H), 5.77 (d, J=16.4Hz, 1H), 5.55 (br, 1H), 4.90 (d, J=16.4Hz, 1H), 3.85 (s, 3H), 3.43 (m, 2H), 2.57 (s, 3H), 2.51 (m, 2H), 2.11 (m, 1H), 1.92 (m, 1H), 1.72 (m, 1H), 1.52 (m, 1H), 0.68 (t, 3H) | *m*/*z* 490 (MH⁺) | Method 24 |

### EXAMPLE E

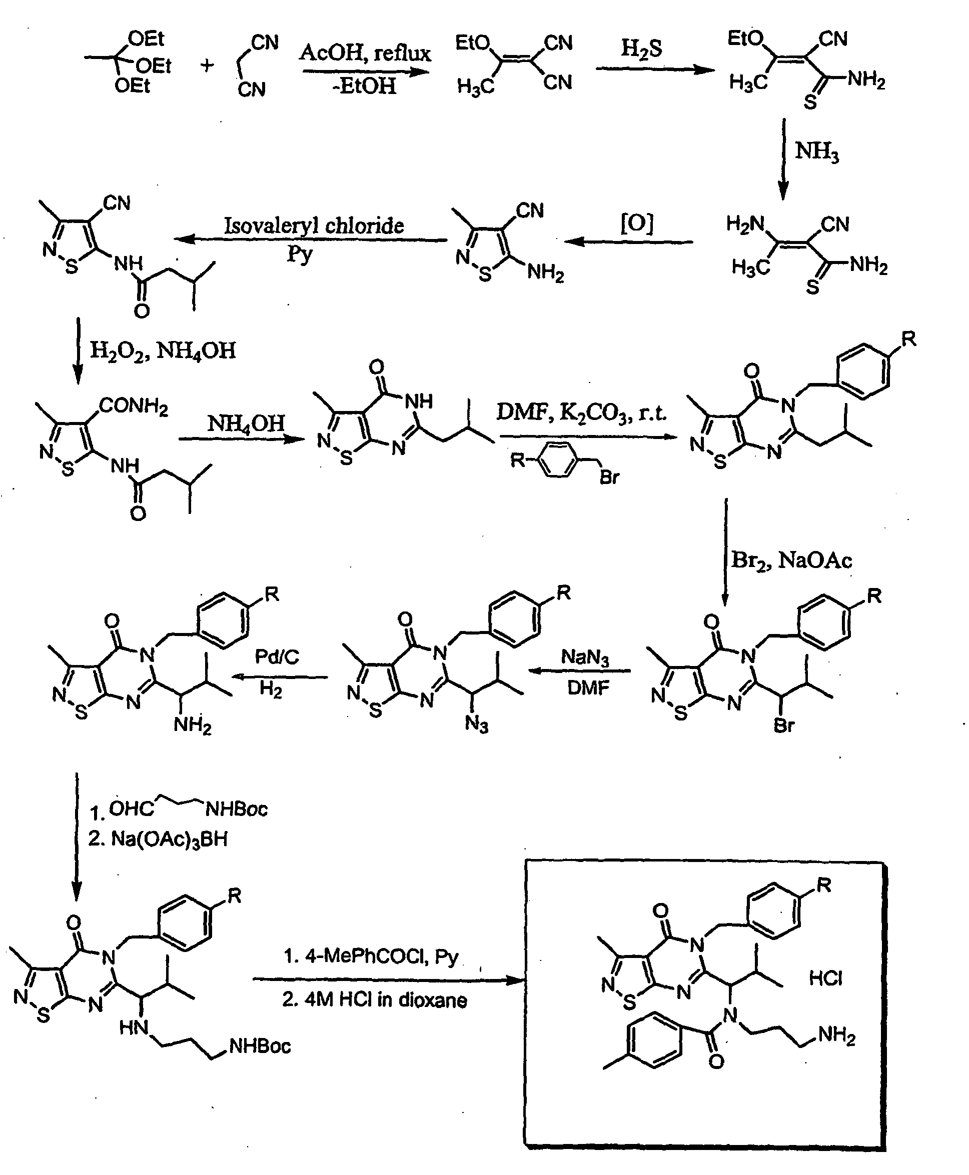

The following compounds were synthesized according to synthetic scheme E above:

| **Ex.** | **Compound** | **¹H NMR** | ***m*/*z*** | **SM** |
|---|---|---|---|---|
| **E-1** | N-(3-Amino-propyl)-N-[1-(5-benzyl-3-methyl-4-oxo-4,5-dihydro-isothiazolo[5,4-d]pyrimidin-6-yl)-2-methyl-propyl]-4-methyl-benzamide hydrogen chloride | ¹H NMR (300 MHz, 96°C, DMSO-d*₆*) δ: 7.71 (bs, 3H), 7.20-7.40 (m, 9H), 5.90 (d, 1H), 5.59 (d, 1H), 5.06 (d, 1H), 3.34 (t, 2H), 2.68 (s, 3H), 2.64-2.7 (m, 1H), 2.36 (s, 3H), 2.25 (t, 2H), 1.46-1.63 (m, 1H), 1.12-1.30 (m, 1H), 0.90 (d, 3H), 0.45 (d, 3H) | *m*/*z* 504 (MH⁺) | Method 33 |
| **E-2** | N-(3-Amino-propyl)-N-{1-[5-(4-fluoro-benzyl)-3-methyl-4-oxo-4,5-dihydro-isothiazolo[5,4-d]pyrimidin-6-yl]-2-methyl-propyl}-4-methyl-benzamide hydrogen chloride | ¹H NMR (300 MHz, 96°C, DMSO-d*₆*) δ: 7.12-7.67 (m, 11H), 5.86 (d, 1H), 5.57 (d, 1H), 5.04 (d, 1H), 3.35 (t, 2H), 2.75 (s, 3H), 2.66-2.72 (m, 1H), 2.36 (s, 3H), 2.27 (t, 2H), 1.44-1.56 (m, 1H), 1.10-1.28 (m, 1H), 0.92 (d, 3H), 0.47 (d, 3H) | *m*/*z* 522 (MH⁺) | Method 33 |

### EXAMPLE F

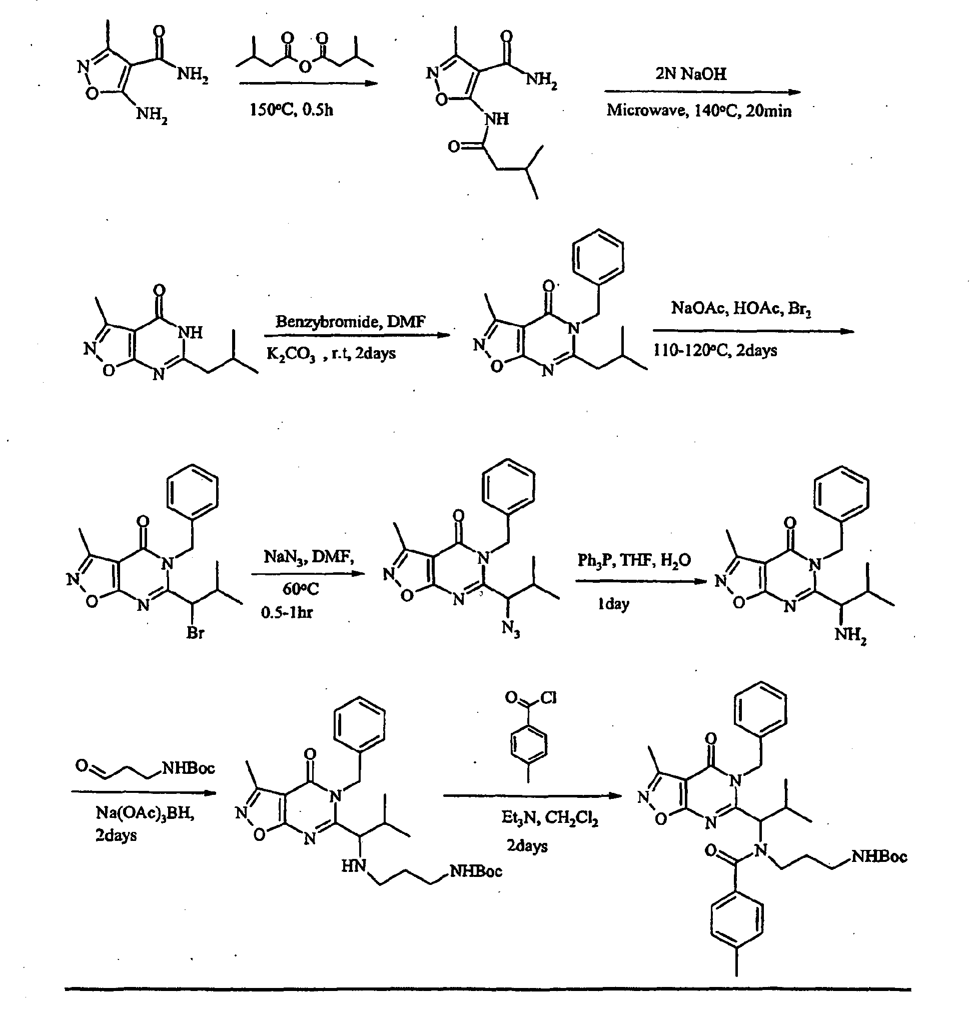

The following compounds were synthesized according to synthetic scheme F above:

| **Ex.** | **Compound** | **¹H NMR** | ***m*/*z*** | **SM** |
|---|---|---|---|---|
| **F-1** | N-(3-Amino-propyl)-N-[1-(5-benzyl-3-methyl-4-oxo-4,5-dihydro-isoxazolo[5,4-d]pyrimidin-6-yl)-2-methyl-propyl]-4-methyl-benzamide hydrogen chloride | ¹H NMR (500 MHz, 100°C, DMSO-d*₆*): δ: 7.63 (br, 3H), 7.23-7.39 (m, 9H), 5.90 (d, 1H), 5.64 (d, 1H), 5.11 (d, 1H), 3.37 (m, 2H), 2.70 (m, 1H), 2.58 (s, 3H), 2.38 (s, 3H), 2.35 (m, 2H), 1.60 (m, 1H), 1.30 (m, 1H), 0.94 (d, 3H), 0.48 (d, 3H) | *m*/*z* 488 (MH⁺) | Method 42 |
| **F-2** | N-(3-Amino-propyl)-N-[1-(5-benzyl-3-methyl-4-oxo-4,5-dihydro-isoxazolo[5,4-d]pyrimidin-6-yl)-2-methyl-propyl]-3-fluoro-4-methyl-benzamide hydrogen chloride | ¹H NMR (500 MHz, 100°C, DMSO-d*₆*): δ: 7.63 (br, 3H), 7.10-7.40 (m, 8H), 5.90 (d, 1H), 5.64 (d, J=10Hz, 1H), 5.10 (d, J=16Hz, 1H), 3.38 (m, 2H), 2.70 (m, 1H), 2.58 (s, 3H), 2.36 (m, 2H), 2.31 (s, 3H), 1.60 (m, 1H), 1.25 (m, 1H), 0.93 (d, 3H), 0.48 (d, 3H) | *m*/*z* 506 (MH⁺) | Method 42 |
| **F-3** | N-(3-Amino-propyl)-N-{1-[5-(4-fluoro-benzyl)-3-methyl-4-oxo-4,5-dihydro-isoxazolo[5,4-d]pyrimidin-6-yl]-2-methyl-propyl}-4-methyl-benzamide hydrogen chloride | ¹H NMR (500 MHz, 100°C, DMSO-d*₆*) δ: 7.60 (br, 3H) 7.17-7.34 (m, 8H), 5.88 (d, J=16Hz, 1H), 5.60 (d, J=10Hz, 1H), 5.10 (d, J=16Hz, 1H), 3.40 (m, 2H), 2.75 (m, 1H), 2.60 (s, 3H), 2.40 (s, 3H), 2.32 (m, 2H), 1.55 (m, 1H), 1.25 (m, 1H), 0.95 (d, 3H), 0.50 (d, 3H) | *m*/*z* 506 (MH⁺) | Method 42 |
| **F-4** | N-(3-Amino-propyl)-3-fluoro-N-{1-[5-(4-fluoro-benzyl)-3-methyl-4-oxo-4,5-dihydro-isoxazolo[5,4-d]pyrimidin-6-yl]-2-methyl-propyl}-4-methyl-benzamide hydrogen chloride | ¹H NMR (500 MHz, 100°C, DMSO-d*₆*): δ: 7.57 (br, 3H) 7.11-7.40 (m, 7H), 5.86 (d, J=16Hz, 1H), 5.61 (d, J=10Hz, 1H), 5.08 (d, J=16Hz, 1H), 3.39 (m, 2H), 2.71 (m, 1H), 2.58 (s, 3H), 2.36 (m, 2H), 2.31 (s, 3H), 1.58 (m, 1H), 1.25 (m, 1H), 0.95 (d, 3H), 0.50 (d, 3H) | *m*/*z* 524 (MH⁺) | Method 42 |

### Utility

The compounds of the present invention have utility for the treatment of neoplastic disease by inhibiting the microtubule motor protein HsEg5. Methods of treatment target Eg5 activity, which is required for the formation of a mitotic spindle and therefore for cell division. Thus, inhibitors of Eg5 have been shown to block cells in the metaphase of mitosis leading to apoptosis of effected cells, and to therefore have anti-proliferative effects. Thus Eg5 inhibitors act as modulators of cell division and are expected to be active against neoplastic disease such as carcinoma of the breast, ovary, lung, colon, prostate or other tissues, as well as leukemias and lymphomas, tumors of the central and peripheral nervous system, and other tumor types such as melanoma, fibrosarcoma and osteosarcoma. Eg5 inhibitors are also expected to be useful for the treatment other proliferative diseases including but not limited to autoimmune, inflammatory, neurological, and cardiovascular diseases.

Compounds of the present invention have been shown to inhibit Eg5, as determined by Malachite Green Assay described herein.

Compounds provided by this invention should also be useful as standards and reagents in determining the ability of a potential pharmaceutical to inhibit Eg5. These would be provided in commercial kits comprising a compound of this invention

### Assays

### Malachite Green Assay

Enzymatic activity of the Eg5 motor and effects of inhibitors was measured using a malchite green assay, which measures phosphate liberated from ATP, and has been used previously to measure the activity of kinesin motors (Hackney and Jiang, 2001). Enzyme was recombinant HsEg5 motor domain (amino acids 1-369-8His) and was added at a final concentration of 6 nM to 100 µl reactions. Buffer consisted of 25 mM PIPES/KOH, pH 6.8, 2 mM MgCl₂, 1 mM EGTA, 1 mM dtt, 0.01% Triton X-100 and 5 µM paclitaxel. Malachite green/ammonium molybdate reagent was prepared as follows: for 800 ml final volume, 0.27 g of Malachite Green (J.T. Baker) was dissolved in 600 ml of H₂O in a polypropylene bottle. 8.4 g ammonium molybdate (Sigma) was dissolved in 200 ml 4N HCl. The solutions were mixed for 20 min and filtered through 0.02 µm filter directly into a polypropylene container.

5 µl of compound diluted in 12% DMSO was added to the wells of 96 well plates. 80 µl of enzyme diluted in buffer solution above was added per well and incubated with compound for 20 min. After this pre-incubation, substrate solution containing 2 mM ATP (final concentration: 300 µM) and 6.053 µM polymerized tubulin (final concentration: 908 nM) in 15 µl of buffer were then added to each well to start reaction. Reaction was mixed and incubated for a particular 20 min at room temperature. The reactions were then quenched by the addition of 150 µl malachite green/ammonium molybdate reagent, and absorbance read at 650 nanometers exactly 5 min after quench using a Spectramax Plus plate reader (Molecular Devices). Data was graphed and IC₅₀s calculated using ExCel Fit (Microsoft).

## Claims

1. A compound having the structural formula (I): 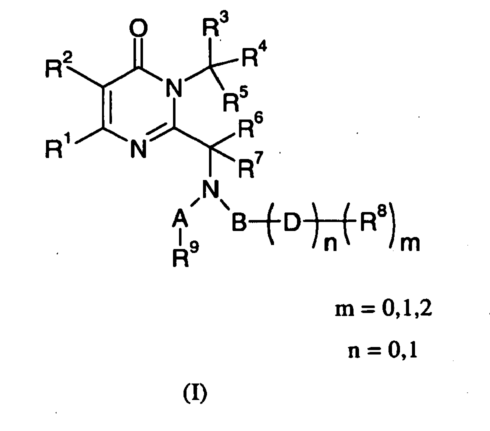 wherein,
A is C=O, CH₂, or SO₂;
B represents optionally substituted C₁₋₁₂alkyl, optionally substituted C₂₋₁₂alkenyl, optionally substituted C₂₋₁₂alkynyl, optionally substituted aryl, optionally substituted C₃₋₁₂cycloalkyl, or optionally substituted heterocycle;
D is O or N wherein O is optionally substituted with one R⁸, wherein N is optionally substituted with one or more R⁸, and when n is 0 and m is not 0, R⁸ is attached directly to B;
R¹ and R² in combination form an optionally substituted fused isothiazole, or an optionally substituted fused isoxazole that is optionally substituted with 1 or 2 substituents;
R³ is independently selected from H, optionally substituted C₁₋₁₂alkyl, optionally substituted C₂₋₁₂alkenyl, optionally substituted C₂₋₁₂alkynyl, optionally substituted C₃₋₁₂cycloalkyl, optionally substituted C₃₋₁₂cycloalkenyl, optionally substituted C₇₋₁₂cycloalkynyl, optionally substituted aryl or optionally substituted heterocycle;
R⁴ and R⁵ are independently selected from H or optionally substituted C₁₋₁₂alkyl, or R⁴ and R⁵ in combination form a 3-, 4-, 5- or 6- membered ring, which may also be optionally substituted;
R⁶ and R⁷ are independently selected from H, optionally substituted C₁₋₁₂alkyl, optionally substituted C₂₋₁₂alkenyl, optionally substituted C₂₋₁₂alkynyl, optionally substituted C₃₋₁₂cycloalkyl, optionally substituted C₃₋₁₂cycloalkenyl, optionally substituted C₇₋₁₂cycloalkynyl, optionally substituted heterocycle, optionally substituted aryl, or R⁶ and R⁷ in combination form a 3-, 4-, 5- or 6- membered ring, which may also be substituted;
R⁸ is independently selected from H, optionally substituted C₁₋₁₂alkyl, optionally substituted C₂₋₁₂alkenyl, optionally substituted C₂₋₁₂alkynyl, optionally substituted C₃₋₁₂cycloalkyl, optionally substituted C₃₋₁₂cycloalkenyl, optionally substituted C₇₋₁₂cycloalkynyl, optionally substituted aryl, or optionally substituted heterocycle;
R⁹ is independently selected from H, optionally substituted C₁₋₁₂alkyl, optionally substituted C₂₋₁₂alkenyl, optionally substituted C₂₋₁₂alkynyl, optionally substituted C_{3-12C}ycloalkyl, optionally substituted C₃₋₁₂cycloalkenyl, optionally substituted C₇₋₁₂cycloalkynyl, optionally substituted aryl, or optionally substituted heterocycle;
wherein:
the term "aryl" refers to a hydrocarbon radical having one or more polyunsaturated carbon rings having aromatic character, comprising 5 up to about 14 carbon atoms;
the term "heterocycle" refers to a ring-containing structure or molecule having one or more multivalent heteroatoms, independently selected from N, O, P and S, as a part of the ring structure and including at least 3 and up to about 20 atoms in the ring(s); heterocycle may be saturated or unsaturated, containing one or more double bonds, and heterocycle may contain more than one ring; when a heterocycle contains more than one ring, the rings may be fused or unfused; heterocycle may have aromatic character or may not have aromatic character; and wherein said substituents are selected from -OC₁₋₆alkyl, -C₁₋₆alkyl, F, Cl, Br, I, =O, =S, -NH₂, -OH, -NHCH₃, -N(CH₃)₂, -NH-cyclopropane, -NH-cyclobutane, azetidine, pyrrolidine and piperidine;
or a pharmaceutically acceptable salt thereof, provided that said compound is not: (±)-N-(3-Amino-propyl)-N-[1-(5-benzyl-3-methyl-4-oxo-4,5-dihydro-isoxazolo[5,4-d]pyrimidin-6-yl)-propyl]-4-methyl-benzamide;
(±)-{3-[[1-(5-Benzyl-3-methyl-4-oxo-4,5-dihydro-isoxazolo[5,4-d]pyrimidin-6-yl)-propyl]-(4-methyl-benzoyl)-amino]-propyl}-carbamic acid tert-butyl ester;
(±)-N-[1-(5-Benzyl-3-methyl-4-oxo-4,5-dihydro-isoxazolo[5,4-d]pyrimidin-6-yl)-propyl]-4-methyl-N-(3-morpholin-4-yl-propyl)-benzamide;
(±)-N-[1-(5-Benzyl-3-methyl-4-oxo-4,5-dihydro-isoxazolo[5,4-d]pyrimidin-6-yl)-propyl]-N-butyl-4-methyl-benzamide;
(±)-N-[1-(5-Benzyl-3-methyl-4-oxo-4,5-dihydro-isoxazolo[5,4-d]pyrimidin-6-yl)-propyl]-N-(3-methoxy-propyl)-4-methyl-benzamide;
(±)-N-[1-(5-Benzyl-3-methyl-4-oxo-4,5-dihydro-isoxazolo[5,4-d]pyrimidin- -6-yl)-propyl]-4-methyl-N-phenethyl-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-4-methyl-benzamide;
(±)-N-[1-(5-Benzyl-3-methyl-4-oxo-4,5-dihydro-isoxazolo[5,4-d]pyrimidin- -6-yl)-propyl]-N-(3-imidazol-1-yl-propyl)-4-methyl-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-3-fluoro-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-3,4-dichloro-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-4-methoxy-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-2-methyl-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-3-methyl-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-4-fluoro-benzamide;
(±)-N-[1-(5-Benzyl-3-methyl-4-oxo-4,5-dihydro-isoxazolo[5,4-d]pyrimidin-6-yl)-propyl]-N-(2-carbamoyl-ethyl)-4-chloro-benzamide;
(±)-N-[1-(5-Benzyl-3-methyl-4-oxo-4,5-dihydro-isoxazolo[5,4-d]pyrimidin-6-yl)-propyl]-4-tert-butyl-N-(2-carbamoyl-ethyl)-benzamide;
(±)-*N*-(3-amino-3-oxopropyl)-*N*-[1-(5-benzyl-3-methyl-4-oxo-4,5-dihydroisoxazolo[5,4-*d*]pyrimidin-6-yl)propyl]-4-butylpyridine-3-carboxamide;
(±)-3-{[1-(5-Benzyl-3-methyl-4-oxo-4,5-dihydro-isoxazolo[5,4-d]pyrimidin-6-yl)-propyl]-[2-(4-chloro-phenyl)-2-oxo-ethyl]-amino}-propionamide;
(±)-3-[[1-(5-Benzyl-3-methyl-4-oxo-4,5-dihydro-isoxazolo[5,4-d]pyrimidin-6-yl)propyl]-(toluene-3-sulfonyl)-amino]-propionamide;
(±)-3-[[1-(5-Benzyl-3-methyl-4-oxo-4,5-dihydro-isoxazolo[5,4-d]pyrimidin-6-yl)-propyl]-(1-methyl-1H-imidazole-4-sulfonyl)-amino]-propionamide;
(±)-*N*-(3-amino-3-oxopropyl)-*N*-[1-(5-benzyl-3-methyl-4-oxo-4,5-dihydroisoxazolo[5,4-*d*]pyrimidin-6-yl)propyl]-5-methylisoxazole-3-carboxamide;
(±)-*N*-(3-amino-3-oxopropyl)-*N*-[1-(5-benzyl-3-methyl-4-oxo-4,5-dihydroisoxazolo[5,4-*d*]pyrimidin-6-yl)propyl]-2,2-dimethylhexanamide;
(±)-*N*-(3-amino-3-oxopropyl)-*N*-[1-(5-benzyl-3-methyl-4-oxo-4,5-dihydroisoxazolo[5,4-*d*]pyrimidin-6-yl)propyl]-2-(1-methylethyl)hexanamide;
(±)-3-{Acetyl-[1-(5-benzyl-3-methyl-4-oxo-4,5-dihydro-isoxazolo[5,4-d]pyrimidin-6-yl)-propyl]-amino}-propionamide;
(±)-3-[[1-(5-Benzyl-3-methyl-4-oxo-4,5-dihydro-isoxazolo[5,4-d]pyrimidin-6-yl)-propyl]-(2-methoxy-acetyl)-amino]-propionamide;
(±)-3-[[1-(5-Benzyl-3-methyl-4-oxo-4,5-dihydro-isoxazolo[5,4-d]pyrimidin-6-yl)-propyl]-(2,2,2-trifluoro-ethanesulfonyl)-amino]-propionamide;
(±)-N-[1-(5-Benzyl-3-methyl-4-oxo-4,5-dihydro-isoxazolo[5,4-d]pyrimidin-6-yl)-propyl]-4-bromo-N-(2-carbamoyl-ethyl)-benzamide;
(±)-N-(3-Amino-propyl)-N-[1-(5-benzyl-3-methyl-4-oxo-4,5-dihydro-isoxazolo[5,4-d]pyrimidin-6-yl)-propyl]-2-methoxy-benzamide;
*tert*-butyl (±)-(3-{[1-(5-benzyl-3-methyl-4-oxo-4,5-dihydroisoxazolo[5,4-*d*]pyrimidin-6-yl)propyl][(2-methoxyphenyl)carbonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-benzamide;
*tert*-butyl (±)-(3-{[1-(5-benzyl-3-methyl-4-oxo-4,5-dihydroisoxazolo[5,4-*d*]pyrimidin-6-yl)propyl](phenylcarbonyl)amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-3-fluoro-benzamide;
*tert*-butyl (±)-(3-{[1-(5-benzyl-3-methyl-4-oxo-4,5-dihydroisoxazolo[5,4-*d*]pyrimidin-6-yl)propyl][(3-fluorophenyl)carbonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-3,4-dichloro-benzamide;
*tert*-butyl (±)-(3-{[1-(5-benzyl-3-methyl-4-oxo-4,5-dihydroisoxazolo[5,4-*d*]pyrimidin-6-yl)propyl][(3,4-dichlorophenyl)carbonyl]amino}propyl)carbamate;
(±)-N-(3-Ainino-propyl)-N-[1-(5-benzyl-3-methyl-4-oxo-4,5-dihydro-isoxazolo[5,4-d]pyrimidin-6-yl)-propyl]-3-propyl-benzamide;
*tert*-butyl (±)-(3-{[1-(5-benzyl-3-methyl-4-oxo-4,5-dihydroisoxazolo[5,4-*d*]pyrimidin-6-yl)propyl][(3-propylphenyl)carbonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-2-methyl-benzamide;
*tert*-butyl (±)-(3-{[1-(5-benzyl-3-methyl-4-oxo-4,5-dihydroisoxazolo[5,4-*d*]pyrimidin-6-yl)propyl][(2-methylphenyl)carbonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-3-methyl-benzamide;
*tert*-butyl (±)-(3-{[1-(5-benzyl-3-methyl-4-oxo-4,5-dihydroisoxazolo[5,4-*d*]pyrimidin-6-yl)propyl][(3-methylphenyl)carbonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-4-chloro-benzamide;
*tert*-butyl (±)-(3-{[1-(5-benzyl-3-methyl-4-oxo-4,5-dihydroisoxazolo[5,4-*d*]pyrimidin-6-yl)propyl][(4-chlorophenyl)carbonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-isonicotinamide;
*tert*-butyl (±)-(3-{[1-(5-benzyl-3-methyl-4-oxo-4,5-dihydroisoxazolo[5,4-*d*]pyrimidin-6-yl)propyl](pyridin-4-ylcarbonyl)amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-benzenesulfonamide;
*tert*-butyl (±)-(3-{[1-(5-benzyl-3-methyl-4-oxo-4,5-dihydroisoxazolo[5,4-*d*]pyrimidin-6-yl)propyl](phenylsulfonyl)amino}propyl)carbamate;
(±)-Biphenyl-4-carboxylic acid (3-amino-propyl)-[1-(5-benzyl-3-methyl-4-oxo-4,5-dihydro-isoxazolo[5,4-d]pyrimidin-6-yl)-propyl]-amide;
*tert*-butyl (±)-acetate-*N*-(3-aminopropyl)-*N*-[1-(5-benzyl-3-methyl-4-oxo-4,5-dihydroisoxazolo[5,4-*d*]pyrimidin-6-yl)propyl]biphenyl-4-carboxamide;
(±)-N-(3-Amino-propyl)-N-[1-(5-benzyl-3-methyl-4-oxo-4,5-dihydro-isoxazolo[5,4-d]pyrimidin-6-yl)-propyl]-4-ethyl-benzamide;
*tert*-butyl (±)-(3-{[1-(5-benzyl-3-methyl-4-oxo-4,5-dihydroisoxazolo[5,4-*d*]pyrimidin-6-yl)propyl][(4-ethylphenyl)carbonyl]amino}propyl)carbamate;
(±)-6-(1-{(3-Amino-propyl)-[2-(4-chloro-phenyl)-2-oxo-ethyl]-amino}-propyl)-5-benzyl-3-methyl-5H-isoxazolo[5,4-d]pyrimidin-4-one;
*tert*-butyl (±)-(3-{[1-(5-benzyl-3-methyl-4-oxo-4,5-dihydroisoxazolo[5,4-*d*]pyrimidin-6-yl)propyl][2-(4-chlorophenyl)-2-oxoethyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-4-chloro-benzenesulfonamide;
*tert*-butyl (±)-(3-{[1-(5-benzyl-3-methyl-4-oxo-4,5-dihydroisoxazolo[5,4-*d*]pyrimidin-6-yl)propyl][(4-chlorophenyl)sulfonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-4-tert-butyl-benzenesulfonamide;
*tert*-butyl (±)-(3-{[1-(5-benzyl-3-methyl-4-oxo-4,5-dihydroisoxazolo[5,4-*d*]pyrimidin-6-yl)propyl][(4-*tert*-butylphenyl)sulfonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-3-methyl-benzenesulfonamide;
*tert*-butyl (±)-(3-{[1-(5-benzyl-3-methyl-4-oxo-4,5-dihydroisoxazolo[5,4-*d*]pyrimidin-6-yl)propyl][(3-methylphenyl)sulfonyl]amino}propyl)carbamate;
(±)-Quinoline-2-carboxylic acid (3-amino-propyl)-[1-(5-benzyl-3-methyl-4-oxo-4,5-dihydro-isoxazolo[5,4-d]pyrimidin-6-yl)-propyl]-amide;
*tert*-butyl (±)-(3-{[1-(5-benzyl-3-methyl-4-oxo-4,5-dihydroisoxazolo[5,4-*d*]pyrimidin-6-yl)propyl](quinolin-2-ylcarbonyl)amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-4-methyl-benzenesulfonamide;
*tert*-butyl (±)-(3-{[1-(5-benzyl-3-methyl-4-oxo-4,5-dihydroisoxazolo[5,4-*d*]pyrimidin-6-yl)propyl][(4-methylphenyl)sulfonyl]amino}propyl)carbamate;
(±)-5-methyl-isoxazole-3-carboxylic acid (3-amino-propyl)-[1-(5-benzyl-3-methyl-4-oxo-4,5-dihydro-isoxazolo[5,4-d]pyrimidin-6-yl)-propyl]-amide;
*tert*-butyl (±)-(3-{[1-(5-benzyl-3-methyl-4-oxo-4,5-dihydroisoxazolo[5,4-*d*]pyrimidin-6-yl)propyl][(5-methylisoxazol-3-yl)carbonyl]amino}propyl)carbamate;
(±)-Cyclobutanecarboxylic acid (3-amino-propyl)-[1-(5-benzyl-3-methyl-4-oxo-4,5-dihydro-isoxazolo[5,4-d]pyrimidin-6-yl)-propyl]-amide;
*tert*-butyl (±)-(3-{[1-(5-benzyl-3-methyl-4-oxo-4,5-dihydroisoxazolo[5,4-*d*]pyrimidin-6-yl)propyl](cyclobutylcarbonyl)amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-isobutyramide;
*tert*-butyl (±)-(3-{[1-(5-benzyl-3-methyl-4-oxo-4,5-dihydroisoxazolo[5,4-*d*]pyrimidin-6-yl)propyl](2-methylpropanoyl)amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-3-methyl-butyramide;
*tert*-butyl (±)-(3-{[1-(5-benzyl-3-methyl-4-oxo-4,5-dihydroisoxazolo[5,4-*d*]pyrimidin-6-yl)propyl](3-methylbutanoyl)amino}propyl)carbamate;
(±)-*N*-(3-aminopropyl)-*N*-[1-(5-benzyl-3-methyl-4-oxo-4,5-dihydroisoxazolo[5,4-*d*]pyrimidin-6-yl)propyl]-4-chlorobenzenesulfonamide;
*tert*-butyl (±)-(3-{[1-(5-benzyl-3-methyl-4-oxo-4,5-dihydroisoxazolo[5,4-*d*]pyrimidin-6-yl)propyl][(4-chlorophenyl)sulfonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-2-methoxy-acetamide;
*tert*-butyl (±)-(3-{[1-(5-benzyl-3-methyl-4-oxo-4,5-dihydroisoxazolo[5,4-*d*]pyrimidin-6-yl)propyl](methoxyacetyl)amino}propyl)carbamate;
(±)-*N*-(3-aminopropyl)-*N*-[1-(5-benzyl-3-methyl-4-oxo-4,5-dihydroisoxazolo[5,4-*d*]pyrimidin-6-yl)propyl]-4-propylbenzamide;
*tert*-butyl (±)-(3-{[1-(5-benzyl-3-methyl-4-oxo-4,5-dihydroisoxazolo[5,4-*d*]pyrimidin-6-yl)propyl] [(4-propylphenyl)carbonyl]amino}propyl)carbamate;
(±)-N-(3-Amino-propyl)-N-[1-(5-benzyl-3-methyl-4-oxo-4,5-dihydro-isoxazolo[5,4-d]pyrimidin-6-yl)-propyl]-4-bromo-benzamide;
*tert*-butyl (±)-(3-{[1-(5-benzyl-3-methyl-4-oxo-4,5-dihydroisoxazolo[5,4-*d*]pyrimidin-6-yl)propyl][(4-bromophenyl)carbonyl]amino}propyl)carbamate;
(±)-N-(3-Aminopropyl)-N-[1-(5-benzyl-3-isopropyl-4-oxo-4,5-dihydroisoxazolo[5,4-d]pyrimidin-6-yl)-propyl]-4-methylbenzamide Hydrochloride;
(±)-N-(3-Aminopropyl)-N-[1-(5-benzyl-3-isopropyl-4-oxo-4,5-dihydroisoxazolo[5,4-d]pyrimidin-6-yl)-propyl]-4-methylbenzamide;
*tert*-butyl (±)-[3-({1-[5-benzyl-3-(1-methylethyl)-4-oxo-4,5-dihydroisoxazolo[5,4-*d*]pyrimidin-6-yl]propyl}[(4-methylphenyl)carbonyl]amino)propyl]carbamate;
(±)-N-(3-Aminopropyl)-N-{1-[5-(4-chlorobenzyl)-3-isopropyl-4-oxo-4,5-dihydro-isoxazolo[5,4-d]pyrimidin-6-yl]-propyl}-4-methylbenzamide Hydrochloride;
(±)-N-(3-Aminopropyl)-N-{1-[5-(4-chlorobenzyl)-3-isopropyl-4-oxo-4,5-dihydro-isoxazolo[5,4-d]pyrimidin-6-yl]-propyl}-4-methylbenzamide;
*tert*-butyl (±)-[3-({1-[5-(4-chlorobenzyl)-3-(1-methylethyl)-4-oxo-4,5-dihydroisoxazolo[5,4-*d*]pyrimidin-6-yl]propyl}[(4-methylphenyl)carbonyl]amino)propyl]-carbamate;
(±)-N-(3-Amino-propyl)-N-[1-(5-benzyl-3-methyl-4-oxo-4,5-dihydro-isothiazolo[5,4-d]pyrimidin-6-yl)-propyl]-4-chloro-benzamide;
(±)-{3-[[1-(5-benzyl-3-methyl-4-oxo-4,5-dihydro-isothiazolo[5,4-d]pyrimidin-6-yl)-propyl]-(4-chloro-benzoyl)-amino]-propyl}-carbamic acid tert-butyl ester;
(±)-N-(3-Amino-3-methyl-butyl)-N-[1-(5-benzyl-3-methyl-4-oxo-4,5-dihydro-isothiazolo[5,4-d]pyrimidin-6-yl)-propyl]-4-chloro-benzamide; or
(±)-{3-[[1-(5-benzyl-3-methyl-4-oxo-4,5-dihydro-isothiazolo[5,4-d]pyrimidin-6-yl)-propyl]-(4-chloro-benzoyl)-amino]-1,1-dimethyl-propyl}-carbamic acid tert-butyl ester.

2. A compound or a pharmaceutically acceptable salt thereof as recited in Claim 1 wherein A is C=O or CH₂.

3. A compound or a pharmaceutically acceptable salt thereof as recited in Claim 1 wherein A is C=O.

4. A compound or a pharmaceutically acceptable salt thereof as recited in Claim 1 wherein , B is optionally substituted C₁₋₁₂alkyl or optionally substituted heterocycle.

5. A compound or a pharmaceutically acceptable salt thereof as recited in Claim 1 wherein B is optionally substituted C₁₋₄alkyl.

6. A compound or a pharmaceutically acceptable salt thereof as recited in Claim 1 wherein B is an optionally substituted C₁₋₄alkyl wherein such substituent is independently selected from -NH₂, -OH, -NHCH₃, -N(CH₃)₂, -NH-cyclopropane, -NH-cyclobutane, azetidine, pyrrolidine, or piperidine.

7. A compound or a pharmaceutically acceptable salt thereof as recited in Claim 1 wherein D is O optionally substituted with R⁸.

8. A compound or a pharmaceutically acceptable salt thereof as recited in Claim 1 wherein D is N optionally substituted with one or more R⁸.

9. A compound or a pharmaceutically acceptable salt thereof as recited in Claim 1 wherein R¹ and R² in combination form an optionally substituted fused isothiazole, or an optionally substituted fused isoxazole that is optionally substituted with 1 or 2 substituents, wherein said substituent is selected from C₁₋₆alkyl, or halogen.

10. A compound or a pharmaceutically acceptable salt thereof as recited in Claim 1 wherein R³ is optionally substituted aryl.

11. A compound or a pharmaceutically acceptable salt thereof as recited in Claim 1 wherein R³ is optionally substituted C₅₋₇aryl.

12. A compound or a pharmaceutically acceptable salt thereof as recited in Claim 1 wherein R³ is optionally substituted C₃₋₇aryl wherein said substituent is independently selected from C₁₋₆alkyl, F, Cl, Br, or I.

13. A compound or a pharmaceutically acceptable salt thereof as recited in Claim 1 wherein R⁴ and R⁵ are H.

14. A compound or a pharmaceutically acceptable salt thereof as recited in Claim 1 wherein R⁶ and R⁷ are independently selected from H, or optionally substituted C₁₋₁₂alkyl.

15. A compound or a pharmaceutically acceptable salt thereof as recited in Claim 1 wherein R⁶ and R⁷ are independently selected from H, or C₁₋₆alkyl.

16. A compound or a pharmaceutically acceptable salt thereof as recited in Claim 1 wherein R⁸ is independently selected from H, optionally substituted C₁₋₁₂alkyl, or optionally substituted heterocycle.

17. A compound or a pharmaceutically acceptable salt thereof as recited in Claim 1 wherein R⁹ is independently selected from optionally substituted aryl or optionally substituted heterocycle.

18. A compound or a pharmaceutically acceptable salt thereof as recited in Claim 1 wherein R⁹ is independently selected from aryl or heterocycle either of which is optionally substituted with 1 or 2 substituents wherein said substituent is independently selected from -C₁₋₆alkyl, -OC₁₋₆alkyl, F, Cl, Br or I.

19. A compound or a pharmaceutically acceptable salt thereof as recited in Claim 1 wherein R⁹ is C₅₋₇aryl optionally substituted with 1 or 2 substituents wherein said substituent is independently selected from -C₁₋₆alkyl, -OC₁₋₆alkyl, F, Cl, Br or I.

20. A compound or a pharmaceutically acceptable salt thereof as recited in Claim 1 wherein:
n is 1;
A is CO or CH₂;
B is optionally substituted C₁₋₆alkyl;
D is N or O;
R¹ and R² in combination form a fused isothiazole or isoxazole;
R³ is optionally substituted phenyl;
R⁴ and R⁵ are H;
R⁶ and R⁷ are H or optionally substituted C₁₋₁₂alkyl;
R⁸ is H or optionally substituted C₁₋₆alkyl;
R⁹ is optionally substituted phenyl.

21. A compound or a pharmaceutically acceptable salt thereof of formula (I) selected from:
N-(3-Amino-propyl)-N-{1-[5-(4-fluoro-benzyl)-3-methyl-4-oxo-4,5-dihydro-isothiazolo[5,4-d]pyrimidin-6-yl]-propyl}-4-methyl-benzamide;
N-(3-Amino-propyl)-N-[1-(5-benzyl-3-methyl-4-oxo-4,5-dihydro-isothiazolo[5,4-d]pyrimidin-6-yl)-propyl]-4-bromo-benzamide;
N-(3-Amino-propyl)-N-[1-(5-benzyl-3-methyl-4-oxo-4,5-dihydro-isothiazolo[5,4-d]pyrimidin-6-yl)-propyl]-3-fluoro-4-methyl-benzamide;
N-(3-Amino-propyl)-N-[1-(5-benzyl-3-methyl-4-oxo-4,5-dihydro-isothiazolo[5,4-d]pyrimidin-6-yl)-propyl]-2,3-dichloro-benzamide;
Naphthalene-2-carboxylic acid (3-amino-propyl)-[1-(5-benzyl-3-methyl-4-oxo-4,5-dihydro-isothiazolo[5,4-d]pyrimidin-6-yl)-propyl]-amide;
Benzo[b]thiophene-2-carboxylic acid (3-amino-propyl)-[1-(5-benzyl-3-methyl-4-oxo-4,5-dihydro-isothiazolo[5,4-d]pyrimidin-6-yl)-propyl]-amide;
N-Azetidin-3-ylmethyl-N-[1-(5-benzyl-3-methyl-4-oxo-4,5-dihydro-isothiazolo[5,4d]pyrimidin-6-yl)-propyl]-4-methyl-benzamide;
N-[1-(5-Benzyl-3-methyl-4-oxo-4,5-dihydro-isothiazolo[5,4-d]pyrimidin-6-yl)-propyl]-4-methyl-N-piperidin-3-ylmethyl-benzamide;
N-(2-Amino-ethyl)-N-[1-(5-benzyl-3-methyl-4-oxo-4,5-dihydro-isothiazolo[5,4-d]pyrimidin-6-yl)-propyl]-4-methyl-benzamide;
N-[1-(5-Benzyl-3-methyl-4-oxo-4,5-dihydro-isothiazolo[5,4-d]pyrimidin-6-yl)-propyl]-N-(2-dimethylamino-ethyl)-4-methyl-benzamide;
N-[1-(5-Benzyl-3-methyl-4-oxo-4,5-dihydro-isothiazolo[5,4-d]pyrimidin-6-yl)-propyl]-N-(3-dimethylamino-propyl)-4-methyl-benzamide;
*N*-[1-(5-Benzyl-3-methyl-4-oxo-4,5-dihydro-isothiazolo[5,4-*d*]pyrimidin-6-yl)propyl]-N-[3-(isopropylamino)propyl]-4-methylbenzamide;
*N*-[1-(5-Benzyl-3-methyl-4-oxo-4,5-dihydro-isothiazolo[5,4-*d*]pyrimidin-6-yl)propyl] - N-[3-(cyclopropylamino)propyl]-4-methylbenzamide;
*N*-(3-Azetidin-1-ylpropyl)-N-[1-(5-benzyl-3-methyl-4-oxo-4,5-dihydro-isothiazolo[5,4-*d*]pyrimidin-6-yl)propyl]-4-methylbenzamide;
*N*-[1-(5-Benzyl-3-methyl-4-oxo-4,5-dihydro-isothiazolo[5,4-*d*]pyrimidin-6-yl)propyl]-4-methyl-N-[3-(3-pyrrolidin-1-ylpropyl) benzamide;
*N*-[1-(5-Benzyl-3-methyl-4-oxo-4,5-dihydro-isothiazolo[5,4-*d*]pyrimidin-6-yl)propyl]-4-methyl-N-[3-(methylamino) propyl] benzamide;
N-[1-(5-Benzyl-3-methyl-4-oxo-4,5-dihydro-isothiazolo[5,4-d]pyrimidin-6-yl)-propyl]-N-(3-hydroxy-propyl)-4-methyl-benzamide;
*N*-(3-Amino-propyl)-*N*-[1-(5-benzyl-3-methyl-4-oxo-4,5-dihydro-isothiazolo[5,4-*d*]pyrimidin-6-yl)-propyl]-4-methyl-benzamide;
N-(3-Amino-propyl)-N-[1-(5-benzyl-3-methyl-4-oxo-4,5-dihydro-isothiazolo[5,4-d]pyrimidin-6-yl)-2-methyl-propyl]-4-methyl-benzamide;
N-(3-Amino-propyl)-N-{1-[5-(4-fluoro-benzyl)-3-methyl-4-oxo-4,5-dihydro-isothiazolo[5,4-d]pyrimidin-6-yl]-2-methyl-propyl}-4-methyl-benzamide;
5-Benzyl-6-{1-[(3-hydroxy-propyl)-(4-methyl-benzyl)-amino]-propyl}-3-methyl-5H-isothiazolo[5,4-d]pyrimidin-4-one;
N-(3-Amino-propyl)-N-{1-[5-(4-fluoro-benzyl)-3-methyl-4-oxo-4,5-dihydro-isoxazolo[5,4-d]pyrimidin-6-yl]-2-methyl-propyl}-4-methyl-benzamide;
N-(3-Amino-propyl)-3-fluoro-N-{1-[5-(4-fluoro-benzyl)-3-methyl-4-oxo-4,5-dihydro-isoxazolo[5,4-d]pyrimidin-6-yl]-2-methyl-propyl}-4-methyl-benzamide;
N-(3-Amino-propyl)-N-[1-(5-benzyl-3-methyl-4-oxo-4,5-dihydro-isoxazolo[5,4-d]pyrimidin-6-yl)-2-methyl-propyl]-3-fluoro-4-methyl-benzamide;
N-(3-Amino-propyl)-N-[1-(5-benzyl-3-methyl-4-oxo-4,5-dihydro-isoxazolo[5,4-d]pyrimidin-6-yl)-2-methyl-propyl]-4-methyl-benzamide;
N-(3-Amino-propyl)-N-[1-(5-benzyl-3-methyl-4-oxo-4,5-dihydro-isoxazolo[5,4-d]pyrimidin-6-yl)-propyl]-4-fluoro-benzamide;
N-(3-Amino-propyl)-N-[1-(5-benzyl-3-methyl-4-oxo-4,5-dihydro-isoxazolo[5,4-d]pyrimidin-6-yl)-propyl]-2,3-dichloro-benzamide;
N-(3-Amino-propyl)-N-[1-(5-benzyl-3-methyl-4-oxo-4,5-dihydro-isoxazolo[5,4-d]pyrimidin-6-yl)-propyl]-3-fluoro-4-methyl-benzamide;
N-(3-Amino-propyl)-N-[1-(5-benzyl-3-methyl-4-oxo-4,5-dihydro-isoxazolo[5,4-d]pyrimidin-6-yl)-propyl]-4-methoxy-benzamide.

22. A compound or a pharmaceutically acceptable salt thereof according to any one of claims 1 to 21, for use as a medicament.

23. The use of a compound or a pharmaceutically acceptable salt thereof as defined in any one of claims 1 to 21, in the manufacture of a medicament for the treatment or prophylaxis of disorders associated with cancer.

24. A pharmaceutical composition comprising a compound as defined in any one of claims 1 to 21, or a pharmaceutically acceptable salt thereof, together with at least one pharmaceutically acceptable carrier, diluent or excipient.

## Patentansprüche

1. Verbindungen mit der Strukturformel (I): 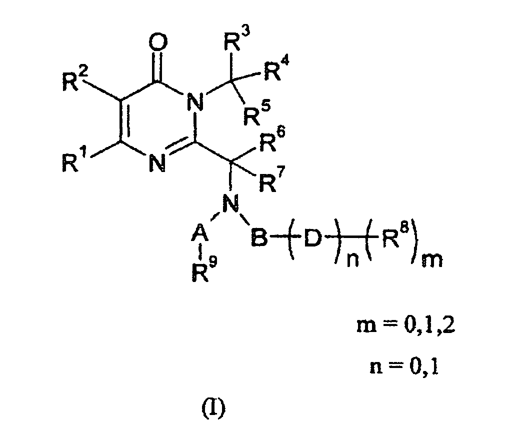 wobei
A für C=O, CH₂ oder SO₂ steht;
B für gegebenenfalls substituiertes C₁₋₁₂-Alkyl, gegebenenfalls substituiertes C₂₋₁₂-Alkenyl, gegebenenfalls substituiertes C₂₋₁₂-Alkinyl, gegebenenfalls substituiertes Aryl, gegebenenfalls substituiertes C₃₋₁₂-Cycloalkyl oder einen gegebenenfalls substituierten Heterocyclus steht;
D für O oder N steht, wobei O gegebenenfalls durch ein R⁸ substituiert ist, wobei N gegebenenfalls durch ein oder mehrere R⁸ substituiert ist, und, wenn n für 0 steht und m nicht für 0 steht, R⁸ direkt an B gebunden ist;
R¹ und R² zusammen ein gegebenenfalls substituiertes kondensiertes Isothiazol oder ein gegebenenfalls substituiertes kondensiertes Isothiazol, das gegebenenfalls durch einen oder zwei Substituenten substituiert ist, bilden;
R³ unabhängig ausgewählt ist aus H, gegebenenfalls substituiertem C₁₋₁₂-Alkyl, gegebenenfalls substituiertem C₂₋₁₂-Alkenyl, gegebenenfalls substituiertem C₂₋₁₂-Alkinyl, gegebenenfalls substituiertem C₃₋₁₂-Cycloalkyl, gegebenenfalls substituiertem C₃₋₁₂-Cycloalkenyl, gegebenenfalls substituiertem C₇₋₁₂-Cycloalkinyl, gegebenenfalls substituiertem Aryl oder einem gegebenenfalls substituierten Heterocyclus;
R⁴ und R⁵ unabhängig ausgewählt sind aus H oder gegebenenfalls substituiertem C₁₋₁₂-Alkyl, oder R⁴ und R⁵ zusammen einen 3-, 4-, 5- oder 6-gliedrigen Ring bilden, der auch gegebenenfalls substituiert sein kann;
R⁶ und R⁷ unabhängig ausgewählt sind aus H, gegebenenfalls substituiertem C₁₋₁₂-Alkyl, gegebenenfalls substituiertem C₂₋₁₂-Alkenyl, gegebenenfalls substituiertem C₂₋₁₂-Alkinyl, gegebenenfalls substituiertem C₃₋₁₂-Cycloalkyl, gegebenenfalls substituiertem C₃₋₁₂-Cycloalkenyl, gegebenenfalls substituiertem C₇₋₁₂-Cycloalkinyl, einem gegebenenfalls substituierten Heterocyclus, gegebenenfalls substituiertem Aryl, oder R⁶ und R⁷ zusammen einen 3-, 4-, 5- oder 6-gliedrigen Ring bilden, der auch substituiert sein kann;
R⁸ unabhängig ausgewählt ist aus H, gegebenenfalls substituiertem C₁₋₁₂-Alkyl, gegebenenfalls substituiertem C₂₋₁₂-Alkenyl, gegebenenfalls substituiertem C₂₋₁₂-Alkinyl, gegebenenfalls substituiertem C₃₋₁₂-Cycloalkyl, gegebenenfalls substituiertem C₃₋₁₂-Cycloalkenyl, gegebenenfalls substituiertem C₇₋₁₂-Cycloalkinyl, gegebenenfalls substituiertem Aryl oder einem gegebenenfalls substituierten Heterocyclus;
R⁹ unabhängig ausgewählt ist aus H, gegebenenfalls substituiertem C₁₋₁₂-Alkyl, gegebenenfalls substituiertem C₂₋₁₂-Alkenyl, gegebenenfalls substituiertem C₂₋₁₂-Alkinyl, gegebenenfalls substituiertem C₃₋₁₂-Cycloalkyl, gegebenenfalls substituiertem C₃₋₁₂-Cycloalkenyl, gegebenenfalls substituiertem C₇₋₁₂-Cycloalkinyl, gegebenenfalls substituiertem Aryl oder einem gegebenenfalls substituierten Heterocyclus;
wobei:
sich der Ausdruck "Aryl" auf einen Kohlenwasserstoffrest mit einem oder mehreren mehrfach ungesättigten Kohlenstoffringen mit aromatischem Charakter und mit 5 bis zu etwa 14 Kohlenstoffatomen bezieht;
sich der Ausdruck "Heterocyclus" auf eine ringhaltige Struktur oder ein ringhaltiges Molekül mit einem oder mehreren mehrwertigen Heteroatomen unabhängig voneinander ausgewählt aus N, O, P und S als Teil der Ringstruktur und einschließlich wenigstens 3 bis zu etwa 20 Atomen in dem Ring/den Ringen bezieht; der Heterocyclus kann gesättigt oder ungesättigt sein und eine oder mehrere Doppelbindungen enthalten, und der Heterocyclus kann mehr als einen Ring enthalten; enthält ein Heterocyclus mehr als einen Ring, so können die Ringe kondensiert oder nicht kondensiert sein; der Heterocyclus kann aromatischen Charakter haben oder keinen aromatischen Charakter haben; und wobei die Substituenten ausgewählt sind aus -OC₁₋₆-Alkyl, -C₁₋₆-Alkyl, F, Cl, Br, I, =O, =S, -NH₂, -OH, -NHCH₃, -N(CH₃)₂, -NH-Cyclopropan, -NH-Cyclobutan, Azetidin, Pyrrolidin und Piperidin;
und deren pharmazeutisch annehmbare Salze, mit der Maßgabe, daß es sich bei der Verbindung nicht um:
(±)-N-(3-Aminopropyl)-N-[1-(5-benzyl-3-methyl-4-oxo-4,5-dihydroisoxazol[5,4-d]pyrimidin-6-yl)propyl]-4-methylbenzamid;
(±)-{3-[[1-(5-Benzyl-3-methyl-4-oxo-4,5-dihydroisoxazolo[5,4-d]pyrimidin-6-yl)propyl]-(4-methylbenzoyl)amino]propyl}carbaminsäure-tert.-butylester;
(±)-N-[1-(5-Benzyl-3-methyl-4-oxo-4,5-dihydroisoxazolo[5,4-d]pyrimidin-6-yl)propyl]-4-methyl-N-(3-morpholin-4-yl-propyl)benzamid;
(±)-N-[1-(5-Benzyl-3-methyl-4-oxo-4,5-dihydroisoxazolo[5,4-d]pyrimidin-6-yl)propyl]-N-butyl-4-methylbenzamid;
(±)-N-[1-(5-Benzyl-3-methyl-4-oxo-4,5-dihydroisoxazolo[5,4-d]pyrimidin-6-yl)propyl]-N-(3-methoxypropyl)-4-methylbenzamid;
(±)-N-[1-(5-Benzyl-3-methyl-4-oxo-4,5-dihydroisoxazolo[5,4-d]pyrimidin-6-yl)propyl]-4-methyl-N-phenethylbenzamid;
(±)-N-[1-(5-Benzyl-3-methyl-4-oxo-4,5-dihydroisoxazolo[5,4-d]pyrimidin-6-yl)propyl]-N-(2-carbamoylethyl)-4-methylbenzamid;
(±)-N-[1-(5-Benzyl-3-methyl-4-oxo-4,5-dihydroisoxazolo[5,4-d]pyrimidin-6-yl)propyl]-N-(3-imidazol-1-yl-propyl)-4-methylbenzamid;
(±)-N-[1-(5-Benzyl-3-methyl-4-oxo-4,5-dihydroisoxazolo[5,4-d]pyrimidin-6-yl)propyl]-N-(2-carbamoylethyl)benzamid;
(±)-N-[1-(5-Benzyl-3-methyl-4-oxo-4,5-dihydroisoxazolo[5,4-d]pyrimidin-6-yl)propyl]-N-(2-carbamoylethyl)-3-fluorbenzamid;
(±)-N-[1-(5-Benzyl-3-methyl-4-oxo-4,5-dihydroisoxazolo[5,4-d]pyrimidin-6-yl)propyl]-N-(2-carbamoylethyl)-3,4-dichlorbenzamid;
(±)-N-[1-(5-Benzyl-3-methyl-4-oxo-4,5-dihydroisoxazolo[5,4-d]pyrimidin-6-yl)propyl]-N-(2-carbamoylethyl)-4-methoxybenzamid;
(±)-N-[1-(5-Benzyl-3-methyl-4-oxo-4,5-dihydroisoxazolo[5,4-d]pyrimidin-6-yl)propyl]-N-(2-carbamoylethyl)-2-methylbenzamid;
(±)-N-[1-(5-Benzyl-3-methyl-4-oxo-4,5-dihydroisoxazolo[5,4-d]pyrimidin-6-yl)propyl]-N-(2-carbamoylethyl)-3-methylbenzamid;
(±)-N-[1-(5-Benzyl-3-methyl-4-oxo-4,5-dihydroisoxazolo[5,4-d]pyrimidin-6-yl)propyl]-N-(2-carbamoylethyl)-4-fluorbenzamid;
(±)-N-[1-(5-Benzyl-3-methyl-4-oxo-4,5-dihydroisoxazolo[5,4-d]pyrimidin-6-yl)propyl]-N-(2-carbamoylethyl)-4-chlorbenzamid;
(±)-N-[1-(5-Benzyl-3-methyl-4-oxo-4,5-dihydroisoxazolo[5,4-d]pyrimidin-6-yl)propyl]-4-tert.-butyl-N-(2-carbamoylethyl)benzamid;
(±)-N-(3-Amino-3-oxopropyl)-N-[1-(5-benzyl-3-methyl-4-oxo-4,5-dihydroisoxazolo[5,4-d]pyrimidin-6-yl)propyl]-4-butylpyridin-3-carbonsäureamid;
(±)-3-{[1-(5-Benzyl-3-methyl-4-oxo-4,5-dihydroisoxazolo[5,4-d]pyrimidin-6-yl)propyl]-[2-(4-chlorphenyl)-2-oxoethyl]amino}propionamid;
(±)-3-[[1-(5-Benzyl-3-methyl-4-oxo-4,5-dihydroisoxazolo[5,4-d]pyrimidin-6-yl)propyl](toluol-3-sulfonyl)amino]propionamid;
(±)-3-[[1-(5-Benzyl-3-methyl-4-oxo-4,5-dihydroisoxazolo[5,4-d]pyrimidin-6-yl)propyl]-(1-methyl-1H-imidazol-4-sulfonyl)amino]propionamid;
(±)-N-(3-Amino-3-oxopropyl)-N-[1-(5-benzyl-3-methyl-4-oxo-4,5-dihydroisoxazolo[5,4-d]pyrimidin-6-yl)propyl]-5-methylisoxazol-3-carbonsäureamid;
(±)-*N*-(3-Amino-3-oxopropyl)-N-[1-(5-benzyl-3-methyl-4-oxo-4,5-dihydroisoxazolo[5,4-d]pyrimidin-6-yl)propyl]-2,2-dimethylhexanamid;
(±)-*N*-(3-Amino-3-oxopropyl)-N-[1-(5-benzyl-3-methyl-4-oxo-4,5-dihydroisoxazolo[5,4-d]pyrimidin-6-yl)propyl]-2-(1-methylethyl)hexanamid;
(±)-3-{Acetyl-[1-(5-benzyl-3-methyl-4-oxo-4,5-dihydroisoxazolo[5,4-d]pyrimidin-6-yl)propyl]amino}propionamid;
(±)-3-[[1-(5-Benzyl-3-methyl-4-oxo-4,5-dihydroisoxazolo[5,4-d]pyrimidin-6-yl)propyl]-(2-methoxy-acetyl)amino]propionamid;
(±)-3-[[1-(5-Benzyl-3-methyl-4-oxo-4,5-dihydroisoxazolo[5,4-d]pyrimidin-6-yl)propyl]-(2,2,2-trifluorethansulfonyl)amino]propionamid;
(±)-N-[1-(5-Benzyl-3-methyl-4-oxo-4,5-dihydroisoxazolo[5,4-d]pyrimidin-6-yl)propyl]-4-brom-N-(2-carbamoylethyl)benzamid;
(±)-N-(3-Aminopropyl)-N-[1-(5-benzyl-3-methyl-4-oxo-4,5-dihydroisoxazolo[5,4-d]pyrimidin-6-yl)propyl]-2-methoxybenzamid;
(±)-(3-{[1-(5-Benzyl-3-methyl-4-oxo-4,5-dihydroisoxazolo[5,4-d]pyrimidin-6-yl)propyl][(2-methoxyphenyl)carbonyl]amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]-3-fluorbenzamid;
(±)-(3-{[1-(5-Benzyl-3-methyl-4-oxo-4,5-dihydroisoxazolo[5,4-d]pyrimidin-6-yl)propyl](phenylcarbonyl)amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]-3-fluorbenzamid;
(±)-(3-{[1-(5-Benzyl-3-methyl-4-oxo-4,5-dihydroisoxazolo[5,4-d]pyrimidin-6-yl)propyl][(3-fluorphenyl)carbonyl]amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]-3,4-dichlorbenzamid;
(±)-(3-{[1-(5-Benzyl-3-methyl-4-oxo-4,5-dihydroisoxazolo[5,4-d]pyrimidin-6-yl)propyl][(3,4-dichlorphenyl)carbonyl]amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]-3-propylbenzamid;
(±)-(3-{[1-(5-Benzyl-3-methyl-4-oxo-4,5-dihydroisoxazolo[5,4-d]pyrimidin-6-yl)propyl][(3-propylphenyl)carbonyl]amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]-2-methylbenzamid;
(±)-(3-{[1-(5-Benzyl-3-methyl-4-oxo-4,5-dihydroisoxazolo[5,4-d]pyrimidin-6-yl)propyl][(2-methylphenyl)carbonyl]amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]-3-methylbenzamid;
(±)-(3-{[1-(5-Benzyl-3-methyl-4-oxo-4,5-dihydroisoxazolo[5,4-d]pyrimidin-6-yl)propyl][(3-methylphenyl)carbonyl]amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]-4-chlorbenzamid;
(±)-(3-{[1-(5-Benzyl-3-methyl-4-oxo-4,5-dihydroisoxazolo[5,4-d]pyrimidin-6-yl)propyl][(4-chlorphenyl)carbonyl]amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]-isonicotinamid;
(±)-(3-{[1-(5-Benzyl-3-methyl-4-oxo-4,5-dihydroisoxazolo[5,4-d]pyrimidin-6-yl)propyl](pyridin-4-ylcarbonyl)amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]benzolsulfonsäureamid;
(±)-(3-{[1-(5-Benzyl-3-methyl-4-oxo-4,5-dihydroisoxazolo[5,4-d]pyrimidin-6-yl)propyl](phenylsulfonyl)amino}propyl)carbaminsäure-tert.-butylester;
(±)-Biphenyl-4-carbonsäure-(3-aminopropyl)-[1-(5-benzyl-3-methyl-4-oxo-4,5-dihydroisoxazolo[5,4-d]pyrimidin-6-yl)propyl]amid;
(±)-Essigsäure-*N*-(3-aminopropyl)-N-[1-(5-benzyl-3-methyl-4-oxo-4,5-dihydroisoxazolo[5,4-d]pyrimidin-6-yl)propyl]biphenyl-4-carbonsäureamid-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]-4-ethylbenzamid;
(±)-(3-{[1-(5-Benzyl-3-methyl-4-oxo-4,5-dihydroisoxazolo[5,4-d]pyrimidin-6-yl)propyl][(4-ethylphenyl)carbonyl]amino}propyl)carbaminsäure-tert.-butylester;
(±)-6-(1-{(3-Aminopropyl)-[2-(4-chlorphenyl)-2-oxoethyl]amino}propyl)-5-benzyl-3-methyl-5H-isoxazolo[5,4-d]pyrimidin-4-on;
(±)-(3-{[1-(5-Benzyl-3-methyl-4-oxo-4,5-dihydroisoxazolo[5,4-d]pyrimidin-6-yl)propyl][2-(4-chlorphenyl)-2-oxoethyl]amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]-4-chlorbenzolsulfonsäureamid;
(±)-(3-{[1-(5-Benzyl-3-methyl-4-oxo-4,5-dihydroisoxazolo[5,4-d]pyrimidin-6-yl)propyl][(4-chlorphenyl)sulfonyl]amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]-4-tert.-butylbenzolsulfonsäureamid;
(±)-(3-{[1-(5-Benzyl-3-methyl-4-oxo-4,5-dihydroisoxazolo[5,4-d]pyrimidin-6-yl)propyl][(4-tert.-butylphenyl)sulfonyl]amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]-3-methyl-benzolsulfonsäureamid;
(±)-(3-{[1-(5-Benzyl-3-methyl-4-oxo-4,5-dihydroisoxazolo[5,4-d]pyrimidin-6-yl)propyl][(3-methylphenyl)sulfonyl]amino}propyl)carbaminsäure-tert.-butylester;
(±)-Chinolin-2-carbansäure-(3-aminopropyl)-[1-(5-benzyl-3-methyl-4-oxo-4,5-dihydroisoxazolo[5,4-d]pyrimidin-6-yl)propyl]amid;
(±)-(3-{[1-(5-Benzyl-3-methyl-4-oxo-4,5-dihydroisoxazolo[5,4-d]pyrimidin-6-yl)propyl](chinolin-2-ylcarbonyl)amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]-4-methylbenzolsulfonsäureamid;
(±)-(3-{[1-(5-Benzyl-3-methyl-4-oxo-4,5-dihydroisoxazolo[5,4-d]pyrimidin-6-yl)propyl][(4-methylphenyl)sulfonyl]amino}propyl)carbaminsäure-tert.-butylester;
(±)-5-Methyl-isoxazol-3-carbonsäure-(3-aminopropyl)-[1-(5-benzyl-3-methyl-4-oxo-4,5-dihydroisoxazolo[5,4-d]pyrimidin-6-yl)propyl]amid;
(±)-(3-{[1-(5-Benzyl-3-methyl-4-oxo-4,5-dihydroisoxazolo[5,4-d]pyrimidin-6-yl)propyl][(5-methylisoxazol-3-yl)carbonyl]amino}propyl)carbaminsäure-tert.-butylester;
(±)-Cyclobutancarbonsäure-(3-aminopropyl)-[1-(5-benzyl-3-methyl-4-oxo-4,5-dihydroisoxazolo[5,4-d]pyrimidin-6-yl)propyl]amid;
(±)-(3-{[1-(5-Benzyl-3-methyl-4-oxo-4,5-dihydroisoxazolo[5,4-d]pyrimidin-6-yl)propyl](cyclobutylcarbonyl)amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]isobutyramid;
(±)-(3-{[1-(5-Benzyl-3-methyl-4-oxo-4,5-dihydroisoxazolo[5,4-d]pyrimidin-6-yl)propyl](2-methylpropanoyl)amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]-3-methylbutyramid;
(±)-(3-{[1-(5-Benzyl-3-methyl-4-oxo-4,5-dihydroisoxazolo[5,4-d]pyrimidin-6-yl)propyl](3-methylbutanoyl)amino}propyl)carbaminsäure-tert.-butylester;
(±)-*N*-(3-Aminopropyl)-N-[1-(5-benzyl-3-methyl-4-oxo-4,5-dihydroisoxazolo[5,4-d]pyrimidin-6-yl)propyl]-4-chlorbenzolsulfonsäureamid;
(±)-(3-{[1-(5-Benzyl-3-methyl-4-oxo-4,5-dihydroisoxazolo[5,4-d]pyrimidin-6-yl)propyl][(4-chlorphenyl)sulfonyl]amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]-2-methoxyacetamid;
(±)-(3-{[1-(5-Benzyl-3-methyl-4-oxo-4,5-dihydroisoxazolo[5,4-d]pyrimidin-6-yl)propyl](methoxyacetyl)amino}propyl)carbaminsäure-tert.-butylester;
(±)-*N*-(3-Aminopropyl)-N-[1-(5-benzyl-3-methyl-4-oxo-4,5-dihydroisoxazolo[5,4-d]pyrimidin-6-yl)propyl]-4-propylbenzamid;
(±)-(3-{[1-(5-Benzyl-3-methyl-4-oxo-4,5-dihydroisoxazolo[5,4-d]pyrimidin-6-yl)propyl][(4-propylphenyl)carbonyl]amino}propyl)carbaminsäure-tert.-butylester;
(±)-N-(3-Aminopropyl)-N-[1-(5-benzyl-3-methyl-4-oxo-4,5-dihydroisoxazolo[5,4-d]pyrimidin-6-yl)propyl]-4-brombenzamid;
(±)-(3-{[1-(5-Benzyl-3-methyl-4-oxo-4,5-dihydroisoxazolo[5,4-d]pyrimidin-6-yl)propyl][(4-bromphenyl)carbonyl]amino}propyl)carbaminsäure-tert.-butylester;
(±)-N-(3-Aminopropyl)-N-[1-(5-benzyl-3-isopropyl-4-oxo-4,5-dihydroisoxazolo[5,4-d]pyrimidin-6-yl)propyl]-4-methylbenzamidhydrochlorid;
(±)-N-(3-Aminopropyl)-N-[1-(5-benzyl-3-isopropyl-4-oxo-4,5-dihydroisoxazolo[5,4-d]pyrimidin-6-yl)propyl]-4-methylbenzamid;
(±)-[3-({1-[5-Benzyl-3-(1-methylethyl)-4-oxo-4,5-dihydroisoxazolo[5,4-d]pyrimidin-6-yl]propyl}[(4-methylphenyl)carbonyl]amino)propyl]carbaminsäure-tert.-butylester;
(±)-N-(3-Aminopropyl)-N-{1-[5-(4-chlorbenzyl)-3-isopropyl-4-oxo-4,5-dihydroisoxazolo[5,4-d]pyrimidin-6-yl]propyl}-4-methylbenzamidhydrochlorid;
(±)-N-(3-Aminopropyl)-N-{1-[5-(4-chlorbenzyl)-3-isopropyl-4-oxo-4,5-dihydroisoxazolo[5,4-d]pyrimidin-6-yl]propyl}-4-methylbenzamid;
(±)-[3-({1-[5-(4-Chlorbenzyl)-3-(1-methylethyl)-4-oxo-4,5-dihydroisoxazolo[5,4-d]pyrimidin-6-yl]propyl}[(4-methylphenyl)carbonyl]amino)propyl]carbaminsäure-tert.-butylester;
(±)-N-(3-Aminopropyl)-N-[1-(5-benzyl-3-methyl-4-oxo-4,5-dihydroisothiazolo[5,4-d]pyrimidin-6-yl)propyl]-4-chlorbenzamid;
(±)-{3-[[1-(5-Benzyl-3-methyl-4-oxo-4,5-dihydroisothiazolo[5,4-d]pyrimidin-6-yl)propyl]-(4-chlorbenzoyl)amino]propyl}carbaminsäure-tert.-butylester;
(±)-N-(3-Amino-3-methylbutyl)-N-[1-(5-benzyl-3-methyl-4-oxo-4,5-dihydroisothiazolo[5,4-d]pyrimidin-6-yl)propyl]-4-chlorbenzamid oder
(±)-{3-[[1-(5-Benzyl-3-methyl-4-oxo-4,5-dihydroisothiazolo[5,4-d]pyrimidin-6-yl)propyl]-(4-chlorbenzoyl)amino]-1,1-dimethylpropyl}carbaminsäure-tert.-butylester handelt.

2. Verbindungen und deren pharmazeutisch annehmbare Salze nach Anspruch 1, wobei A für C=O oder CH₂ steht.

3. Verbindungen und deren pharmazeutisch annehmbare Salze nach Anspruch 1, wobei A für C=O steht.

4. Verbindungen und deren pharmazeutisch annehmbare Salze nach Anspruch 1, wobei B für gegebenenfalls substituiertes C₁₋₁₂-Alkyl oder einen gegebenenfalls substituierten Heterocyclus steht.

5. Verbindungen und deren pharmazeutisch annehmbare Salze nach Anspruch 1, wobei B für gegebenenfalls substituiertes C₁₋₄-Alkyl steht.

6. Verbindungen und deren pharmazeutisch annehmbare Salze nach Anspruch 1, wobei B für gegebenenfalls substituiertes C₁₋₄-Alkyl steht, wobei dieser Substituent unabhängig ausgewählt ist aus -NH₂, -OH, -NHCH₃, -N(CH₃)₂, -NH-Cyclopropan, -NH-Cyclobutan, Azetidin, Pyrrolidin und Piperidin.

7. Verbindungen und deren pharmazeutisch annehmbare Salze nach Anspruch 1, wobei D für gegebenenfalls durch R⁸ substituiertes O steht.

8. Verbindungen und deren pharmazeutisch annehmbare Salze nach Anspruch 1, wobei D für durch ein oder mehrere R⁸ substituiertes N steht.

9. Verbindungen und deren pharmazeutisch annehmbare Salze nach Anspruch 1, wobei R¹ und R² zusammen ein gegebenenfalls substituiertes kondensiertes Isothiazol oder ein gegebenenfalls substituiertes kondensiertes Isothiazol, das gegebenenfalls durch 1 oder 2 Substituenten substuiert ist, bilden, wobei der Substituent ausgewählt ist aus C₁₋₆-Alkyl und Halogen.

10. Verbindungen und deren pharmazeutisch annehmbare Salze nach Anspruch 1, wobei R³ für gegebenenfalls substituiertes Aryl steht.

11. Verbindungen und deren pharmazeutisch annehmbare Salze nach Anspruch 1, wobei R³ für gegebenenfalls substituiertes C₅₋₇-Aryl steht.

12. Verbindungen und deren pharmazeutisch annehmbare Salze nach Anspruch 1, wobei R³ für gegebenenfalls substituiertes C₅₋₇-Aryl steht, wobei der Substituent unabhängig ausgewählt ist aus C₁₋₆-Alkyl, F, Cl, Br und I.

13. Verbindungen und deren pharmazeutisch annehmbare Salze nach Anspruch 1, wobei R⁴ und R⁵ für H stehen.

14. Verbindungen und deren pharmazeutisch annehmbare Salze nach Anspruch 1, wobei R⁶ und R⁷ unabhängig ausgewählt sind aus H und gegebenenfalls substituiertem C₁₋₁₂-Alkyl.

15. Verbindungen und deren pharmazeutisch annehmbare Salze nach Anspruch 1, wobei R⁶ und R⁷ unabhängig ausgewählt sind aus H und C₁₋₆-Alkyl.

16. Verbindungen und deren pharmazeutisch annehmbare Salze nach Anspruch 1, wobei R⁸ unabhängig ausgewählt ist aus H, gegebenenfalls substituiertem C₁₋₁₂-Alkyl und einem gegebenenfalls substituierten Heterocyclus.

17. Verbindungen und deren pharmazeutisch annehmbare Salze nach Anspruch 1, wobei R⁹ unabhängig ausgewählt ist aus gegebenenfalls substituiertem Aryl und einem gegebenenfalls substituierten Heterocyclus.

18. Verbindungen und deren pharmazeutisch annehmbare Salze nach Anspruch 1, wobei R⁹ unabhängig ausgewählt ist aus Aryl und einem Heterocyclus, die beide gegebenenfalls durch 1 oder 2 Substituenten substituiert sind, wobei der Substituent unabhängig ausgewählt ist aus -C₁₋₆-Alkyl, -OC₁₋₆-Alkyl, F, Cl, Br und I.

19. Verbindungen und deren pharmazeutisch annehmbare Salze nach Anspruch 1, wobei R⁹ für gegebenenfalls durch 1 oder 2 Substituenten substituiertes C₅₋₇-Aryl steht,
wobei der Substituent unabhängig ausgewählt ist aus -C₁₋₆-Alkyl, -OC₁₋₆-Alkyl, F, Cl, Br und I.

20. Verbindungen und deren pharmazeutisch annehmbare Salze nach Anspruch 1, wobei:
n für 1 steht;
A für CO oder CH₂ steht;
B für gegebenenfalls substituiertes C₁₋₆-Alkyl steht;
D für N oder 0 steht;
R¹ und R² zusammen ein kondensiertes Isothiazol oder Isoxazol bilden;
R³ für gegebenenfalls substituiertes Phenyl steht;
R⁴ und R⁵ für H stehen;
R⁶ und R⁷ für H oder gegebenenfalls substituiertes C₁₋₁₂-Alkyl stehen;
R⁸ für H oder gegebenenfalls substituiertes C₁₋₆-Alkyl steht;
R⁹ für gegebenenfalls substituiertes Phenyl steht.

21. Verbindungen und deren pharmazeutisch annehmbare Salze der Formel (I), ausgewählt aus:
N-(3-Aminopropyl)-N-{1-[5-(4-fluorbenzyl)-3-methyl-4-oxo-4,5-dihydroisothiazol[5,4-d]pyrimidin-6-yl]propyl}-4-methylbenzamid;
N-(3-Aminopropyl)-N-[1-(5-benzyl-3-methyl-4-oxo-4,5-dihydroisothiazol[5,4-d]pyrimidin-6-yl)-4-brombenzamid;
N-(3-Aminopropyl)-N-[1-(5-benzyl-3-methyl-4-oxo-4,5-dihydroisothiazol[5,4-d]pyrimidin-6-yl)propyl]-3-fluor-4-methylbenzamid;
N-(3-Aminopropyl)-N-[1-(5-benzyl-3-methyl-4-oxo-4,5-dihydroisothiazol[5,4-d]pyrimidin-6-yl)propyl]-2,3-dichlorbenzamid;
Naphthalin-2-carbonsäure-(3-aminopropyl)-[1-(5-benzyl-3-methyl-4-oxo-4,5-dihydroisothiazol[5,4-d]pyrimidin-6-yl)propyl]amid;
Benzo[b]thiophen-2-carbonsäure-(3-aminopropyl)-[1-(5-benzyl-3-methyl-4-oxo-4,5-dihydroisothiazol[5,4-d]pyrimidin-6-yl)propyl]amid;
N-Azetidin-3-ylmethyl-N-[1-(5-benzyl-3-methyl-4-oxo-4,5-dihydroisothiazol[5,4-d]pyrimidin-6-yl)propyl]-4-methylbenzamid;
N-[1-(5-Benzyl-3-methyl-4-oxo-4,5-dihydroisothiazol[5,4-d]pyrimidin-6-yl)propyl]-4-methyl-N-piperidin-3-ylmethylbenzamid;
N-(2-Aminoethyl)-N-[1-(5-benzyl-3-methyl-4-oxo-4,5-dihydroisothiazol[5,4-d]pyrimidin-6-yl)propyl]-4-methylbenzamid;
N-[1-(5-Benzyl-3-methyl-4-oxo-4,5-dihydroisothiazol[5,4-d]pyrimidin-6-yl)propyl]-N-(2-dimethylaminoethyl)-4-methylbenzamid;
N-[1-(5-Benzyl-3-methyl-4-oxo-4,5-dihydroisothiazol[5,4-d]pyrimidin-6-yl)propyl]-N-(3-dimethylaminopropyl)-4-methylbenzamid;
N-[1-(5-Benzyl-3-methyl-4-oxo-4,5-dihydroisothiazol[5,4-d]pyrimidin-6-yl)propyl]-N-[3-(isopropylamino)propyl]-4-methylbenzamid;
N-[1-(5-Benzyl-3-methyl-4-oxo-4,5-dihydroisothiazol[5,4-d]pyrimidin-6-yl)propyl]-N-[3-(cyclopropylamino)propyl]-4-methylbenzamid;
N-(3-Azetidin-1-ylpropyl)-N-[1-(5-benzyl-3-methyl-4-oxo-4,5-dihydroisothiazol[5,4-d]pyrimidin-6-yl)propyl]-4-methylbenzamid;
N-[1-(5-Benzyl-3-methyl-4-oxo-4,5-dihydroisothiazol[5,4-d]pyrimidin-6-yl)propyl]-4-methyl-N-[3-(3-pyrrolidin-1-ylpropyl)benzamid;
N-[1-(5-Benzyl-3-methyl-4-oxo-4,5-dihydrosisothiazol[5,4-d]pyrimidin-6-yl)propyl]-4-methyl-N-[3-(methylamino)propyl]benzamid;
N-[1-(5-Benzyl-3-methyl-4-oxo-4,5-dihydroisothiazol[5,4-d]pyrimidin-6-yl)propyl]-N-(3-hydroxypropyl)-4-methylbenzamid;
N-(3-Aminopropyl)-N-[1-(5-benzyl-3-methyl-4-oxo-4,5-dihydroisothiazol[5,4-d]pyrimidin-6-yl)propyl]-4-methylbenzamid;
N-(3-Aminopropyl)-N-[1-(5-benzyl-3-methyl-4-oxo-4,5-dihydroisothiazol[5,4-d]pyrimidin-6-yl)-2-methylpropyl]-4-methylbenzamid;
N-(3-Aminopropyl)-N-{1-[5-(fluorbenzyl)-3-methyl-4-oxo-4,5-dihydroisothiazol[5,4-d]pyrimidin-6-yl]-2-methylpropyl}-4-methylbenzamid;
5-Benzyl-6-{1-[(3-hydroxypropyl)-(4-methylbenzyl)amino]propyl}-3-methyl-5H-isothiazol[5,4-d]pyrimidin-4-on;
N-(3-Aminopropyl)-N-{1-[5-(4-fluorbenzyl)-3-methyl-4-oxo-4,5-dihydroisoxazol[5,4-d]pyrimidin-6-yl]-2-methylpropyl}-4-methylbenzamid;
N-(3-Aminopropyl)3-fluor-N-{1-[5-(4-fluorbenzyl)-3-methyl-4-oxo-4,5-dihydroisoxazol[5,4-d]pyrimidin-6-yl]-2-methylpropyl}-4-methylbenzamid;
N-(3-Aminopropyl)-N-[1-(5-benzyl-3-methyl-4-oxo-4,5-dihydroisoxazol[5,4-d]pyrimidin-6-yl)-2-methylpropyl]-3-fluor-4-methylbenzamid;
N-(3-Aminopropyl)-N-[5-benzyl-3-methyl-4-oxo-4,5-dihydroisoxazol[5,4-d]pyrimdin-6-yl]-2-methylpropyl]-4-methylbenzamid;
N-(3-Aminopropyl)-N-[1-(5-benzyl-3-methyl-4-oxo-4,5-dihydroisoxazol[5,4-d]pyrimidin-6-yl)propyl]-4-fluorbenzamid;
N-(3-Aminopropyl)-N-[1-(5-benzyl-3-methyl-4-oxo-4,5-dihydroisoxazol[5,4-d]pyrimidin-6-yl)propyl]-2,3-dichlorbenzamid;
N-(3-Aminopropyl)-N-[1-(5-benzyl-3-methyl-4-oxo-4,5-dihydroisoxazol[5,4-d]pyrimidin-6-yl)propyl]-3-fluor-4-methylbenzamid;
N-(3-Aminopropyl)-N-[1-(5-benzyl-3-methyl-4-oxo-4,5-dihydroisoxazol[5,4-d]pyrimidin-6-yl)propyl]-4-methoxybenzamid.

22. Verbindungen und deren pharmazeutisch annehmbare Salze nach einem der Ansprüche 1 bis 21 zur Verwendung als Medikament.

23. Verwendung einer Verbindung oder eines pharmazeutisch annehmbaren Salzes davon nach einem der Ansprüche 1 bis 21 bei der Herstellung eines Medikaments zur Behandlung oder Prophylaxe von mit Krebs assoziierten Krankheiten.

24. Pharmazeutische Zusammensetzung, enthaltend eine Verbindung nach einem der Ansprüche 1 bis 21 oder ein pharmazeutisch annehmbares Salz davon zusammen mit wenigstens einem pharmazeutisch annehmbaren Träger, Verdünnungsmittel oder Exzipienten.

## Revendications

1. Composé ayant la formule structurale (I) : 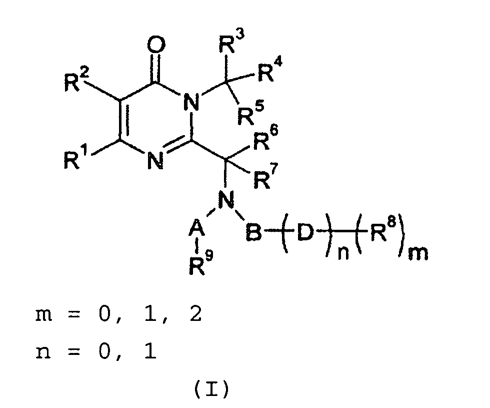
où, A est C=O, CH₂, ou SO₂ ;
B représente un alkyle en C₁₋₁₂ facultativement substitué, un alcényle en C₂₋₁₂ facultativement substitué, un alcynyle en C₂₋₁₂ facultativement substitué, un aryle facultativement substitué, un cycloalkyle en C₃₋₁₂ facultativement substitué, ou un hétérocycle facultativement substitué ;
D est O ou N où O est facultativement substitué par un R⁸, où N est facultativement substitué par un ou plusieurs R⁸, et lorsque n est 0 et m n'est pas 0, R⁸ est lié directement à B ;
R¹ et R² en combinaison forment un isothiazole condensé facultativement substitué, ou un isoxazole condensé facultativement substitué qui est facultativement substitué par 1 ou 2 substituants ;
R³ est indépendamment choisi parmi H, un alkyle en C₁₋₁₂ facultativement substitué, un alcényle en C₂₋₁₂ facultativement substitué, un alcynyle en C₂₋₁₂ facultativement substitué, un cycloalkyle en C₃₋₁₂ facultativement substitué, un cycloalcényle en C₃₋₁₂ facultativement substitué, un cycloalcynyle en C₇₋₁₂ facultativement substitué, un aryle facultativement substitué ou un hétérocycle facultativement substitué ;
R⁴ et R⁵ sont indépendamment choisis parmi H ou un alkyle en C₁₋₁₂ facultativement substitué, ou R⁴ et R⁵ en combinaison forment un cycle à 3, 4, 5 ou 6 chaînons, qui peut également être facultativement substitué ;
R⁶ et R⁷ sont indépendamment choisis parmi H, un alkyle en C₁₋₁₂ facultativement substitué, un alcényle en C₂₋₁₂ facultativement substitué, un alcynyle en C₂₋₁₂ facultativement substitué, un cycloalkyle en C₃₋₁₂ facultativement substitué, un cycloalcényle en C₃₋₁₂ facultativement substitué, un cycloalcynyle en C₇₋₁₂ facultativement substitué, un hétérocycle facultativement substitué, un aryle facultativement substitué, ou R⁶ et R⁷ en combinaison forment un cycle à 3, 4, 5 ou 6 chaînons, qui peut également être substitué ;
R⁸ est indépendamment choisi parmi H, un alkyle en C₁₋₁₂ facultativement substitué, un alcényle en C₂₋₁₂ facultativement substitué, un alcynyle en C₂₋₁₂ facultativement substitué, un cycloalkyle en C₃₋₁₂ facultativement substitué, un cycloalcényle en C₃₋₁₂ facultativement substitué, un cycloalcynyle en C₇₋₁₂ facultativement substitué, un aryle facultativement substitué ou un hétérocycle facultativement substitué ;
R⁹ est indépendamment choisi parmi H, un alkyle en C₁₋₁₂ facultativement substitué, un alcényle en C₂₋₁₂ facultativement substitué, un alcynyle en C₂₋₁₂ facultativement substitué, un cycloalkyle en C₃₋₁₂ facultativement substitué, un cycloalcényle en C₃₋₁₂ facultativement substitué, un cycloalcynyle en C₇₋₁₂ facultativement substitué, un aryle facultativement substitué ou un hétérocycle facultativement substitué ;
où :
le terme « aryle » désigne un radical hydrocarboné ayant un ou plusieurs cycles carbonés polyinsaturés ayant un caractère aromatique, comprenant 5 à environ 14 atomes de carbone ;
le terme « hétérocycle » désigne une structure ou molécule contenant un cycle ayant un ou plusieurs hétéroatomes multivalents, indépendamment choisis parmi N, O, P et S, faisant partie de la structure cyclique et comprenant au moins 3 et jusqu'à environ 20 atomes dans le(s) cycle(s) ; un hétérocycle peut être saturé ou insaturé, contenant une ou plusieurs doubles liaisons, et un hétérocycle peut contenir plus d'un cycle ; lorsqu'un hétérocycle contient plus d'un cycle, les cycles peuvent être condensés ou non condensés ; un hétérocycle peut avoir un caractère aromatique et peut ne pas avoir un caractère aromatique ;
et où lesdits substituants sont choisis parmi -O(alkyle en C₁₋₆), -(alkyle en C₁₋₆), F, Cl, Br, I, =O, =S, -NH₂, -OH, -NHCH₃, -N(CH₃)₂, -NH-cyclopropane, -NH-cyclobutane, l'azétidine, la pyrrolidine et la pipéridine ;
ou un sel pharmaceutiquement acceptable de celui-ci, à condition que ledit composé ne soit pas :
(±)-N-(3-Amino-propyl)-N-[1-(5-benzyl-3-méthyl-4-oxo-4,5-dihydro-isoxazolo[5,4-d]pyrimidin-6-yl)-propyl]-4-méthyl-benzamide ;
Ester *tert*-butylique d'acide (±)-{3-[[1-(5-Benzyl-3-méthyl-4-oxo-4,5-dihydro-isoxazolo[5,4-d]pyrimidin-6-yl)-propyl]-(4-méthyl-benzoyl)-amino]-propyl}-carbamique ;
(±)-N-[1-(5-Benzyl-3-méthyl-4-oxo-4,5-dihydro-isoxazolo[5,4-d]pyrimidin-6-yl)-propyl]-4-méthyl-N-(3-morpholin-4-yl-propyl)-benzamide ;
(±)-N-[1-(5-Benzyl-3-méthyl-4-oxo-4,5-dihydro-isoxazolo[5,4-d]pyrimidin-6-yl)-propyl]-N-butyl-4-méthyl-benzamide ;
(±)-N-[1-(5-Benzyl-3-méthyl-4-oxo-4,5-dihydro-isoxazolo[5,4-d]pyrimidin-6-yl)-propyl]-N-(3-méthoxypropyl)-4-méthyl-benzamide ;
(±)-N-[1-(5-Benzyl-3-méthyl-4-oxo-4,5-dihydro-isoxazolo[5,4-d]pyrimidin-6-yl)-propyl]-4-méthyl-N-phénéthyl-benzamide ;
(±)-N-[1-(5-Benzyl-3-méthyl-4-oxo-4,5-dihydro-isoxazolo[5,4-d]pyrimidin-6-yl)-propyl]-N-(2-carbamoyléthyl)-4-méthyl-benzamide ;
(±)-N-[1-(5-Benzyl-3-méthyl-4-oxo-4,5-dihydro-isoxazolo[5,4-d]pyrimidin-6-yl)-propyl]-N-(3-imidazol-1-yl-propyl)-4-méthyl-benzamide ;
(±)-N-[1-(5-Benzyl-3-méthyl-4-oxo-4,5-dihydro-isoxazolo[5,4-d]pyrimidin-6-yl)-propyl]-N-(2-carbamoyléthyl)-benzamide ;
(±)-N-[1-(5-Benzyl-3-méthyl-4-oxo-4,5-dihydro-isoxazolo[5,4-d]pyrimidin-6-yl)-propyl]-N-(2-carbamoyléthyl)-3-fluoro-benzamide ;
(±)-N-[1-(5-Benzyl-3-méthyl-4-oxo-4,5-dihydro-isoxazolo[5,4-d]pyrimidin-6-yl)-propyl]-N-(2-carbamoyléthyl)-3,4-dichloro-benzamide ;
(±)-N-[1-(5-Benzyl-3-méthyl-4-oxo-4,5-dihydro-isoxazolo[5,4-d]pyrimidin-6-yl)-propyl]-N-(2-carbamoyléthyl)-4-méthoxy-benzamide ;
(±)-N-[1-(5-Benzyl-3-méthyl-4-oxo-4,5-dihydro-isoxazolo[5,4-d]pyrimidin-6-yl)-propyl]-N-(2-carbamoyléthyl)-2-méthyl-benzamide ;
(±)-N-[1-(5-Benzyl-3-méthyl-4-oxo-4,5-dihydro-isoxazolo[5,4-d]pyrimidin-6-yl)-propyl]-N-(2-carbamoyléthyl)-3-méthyl-benzamide ;
(±)-N-[1-(5-Benzyl-3-méthyl-4-oxo-4,5-dihydro-isoxazolo[5,4-d]pyrimidin-6-yl)-propyl]-N-(2-carbamoyléthyl)-4-fluoro-benzamide ;
(±)-N-[1-(5-Benzyl-3-méthyl-4-oxo-4,5-dihydro-isoxazolo[5,4-d]pyrimidin-6-yl)-propyl]-N-(2-carbamoyléthyl)-4-chloro-benzamide ;
(±)-N-[1-(5-Benzyl-3-méthyl-4-oxo-4,5-dihydro-isoxazolo[5,4-d]pyrimidin-6-yl)-propyl]-4-tert-butyl-N-(2-carbamoyl-éthyl)-benzamide ;
(±)-N-(3-amino-3-oxopropyl)-*N*-[1-(5-benzyl-3-méthyl-4-oxo-4,5-dihydroisoxazolo[5,4-d]pyrimidin-6-yl)propyl]-4-butylpyridine-3-carboxamide ;
(±)-3-{[1-(5-Benzyl-3-méthyl-4-oxo-4,5-dihydro-isoxazolo[5,4-d]pyrimidin-6-yl)-propyl]-[2-(4-chlorophényl)-2-oxo-éthyl]-amino}-propionamide ;
(±)-3-[[1-(5-Benzyl-3-méthyl-4-oxo-4,5-dihydro-isoxazolo[5,4-d]pyrimidin-6-yl)propyl]-(toluène-3-sulfonyl)-amino]-propionamide ;
(±)-3-[[1-(5-Benzyl-3-méthyl-4-oxo-4,5-dihydro-isoxazolo[5,4-d]pyrimidin-6-yl)-propyl]-(1-méthyl-1H-imidazole-4-sulfonyl)-amino]-propionamide ;
(±)-*N*-(3-amino-3-oxopropyl)-*N*-[1-(5-benzyl-3-méthyl-4-oxo-4,5-dihydroisoxazolo[5,4-d]pyrimidin-6-yl)propyl]-5-méthylisoxazole-3-carboxamide ;
(±)-*N*-(3-amino-3-oxopropyl)-*N*-[1-(5-benzyl-3-méthyl-4-oxo-4,5-dihydroisoxazolo[5,4-d]pyrimidin-6-yl)propyl]-2,2-diméthylhexanamide ;
(±)-*N*-(3-amino-3-oxopropyl)-*N*-[1-(5-benzyl-3-méthyl-4-oxo-4,5-dihydroisoxazolo[5,4-d]pyrimidin-6-yl)propyl]-2-(1-méthyléthyl)hexanamide ;
(±)-3-{Acétyl-[1-(5-benzyl-3-méthyl-4-oxo-4,5-dihydro-isoxazolo[5,4-d]pyrimidin-6-yl)-propyl]-amino}-propionamide ;
(±)-3-[[1-(5-Benzyl-3-méthyl-4-oxo-4,5-dihydro-isoxazolo[5,4-d]pyrimidin-6-yl)-propyl]-(2-méthoxyacétyl)-amino]-propionamide ;
(±)-3-[[1-(5-Benzyl-3-méthyl-4-oxo-4,5-dihydro-isoxazolo[5,4-d]pyrimidin-6-yl)-propyl]-(2,2,2-trifluoro-éthanesulfonyl)-amino]-propionamide ;
(±)-N-[1-(5-Benzyl-3-méthyl-4-oxo-4,5-dihydro-isoxazolo[5,4-d]pyrimidin-6-yl)-propyl]-4-bromo-N-(2-carbamoyl-éthyl)-benzamide ;
(±)-N-(3-Amino-propyl)-N-[1-(5-benzyl-3-méthyl-4-oxo-4,5-dihydro-isoxazolo[5,4-d]pyrimidin-6-yl)-propyl]-2-méthoxy-benzamide ;
(±)-(3-{[1-(5-Benzyl-3-méthyl-4-oxo-4,5-dihydroisoxazolo[5,4-*d*]pyrimidin-6-yl)propyl][(2-méthoxyphényl)carbonyl]amino}propyl)carbamate de *tert-*butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-benzamide ;
(±)-(3-{[1-(5-Benzyl-3-méthyl-4-oxo-4,5-dihydroisoxazolo[5,4-d]pyrimidin-6-yl)propyl](phénylcarbonyl)amino}propyl)carbamate de *tert*-butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-3-fluoro-benzamide ;
(±)-(3-{[1-(5-Benzyl-3-méthyl-4-oxo-4,5-dihydroisoxazolo[5,4-*d*]pyrimidin-6-yl)propyl][(3-fluorophényl)carbonyl]amino}propyl)carbamate de *tert-*butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-3,4-dichloro-benzamide ;
(±)-(3-{[1-(5-Benzyl-3-méthyl-4-oxo-4,5-dihydroisoxazolo[5,4-d]pyrimidin-6-yl)propyl][(3,4-dichlorophényl)carbonyl]amino}propyl)carbamate de *tert-*butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-3-propyl-benzamide ;
(±)-(3-{[1-(5-Benzyl-3-méthyl-4-oxo-4,5-dihydroisoxazolo[5,4-d]pyrimidin-6-yl)propyl][(3-propylphényl)carbonyl]amino}propyl)carbamate de *tert-*butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-2-méthyl-benzamide ;
(±)-(3-{[1-(5-Benzyl-3-méthyl-4-oxo-4,5-dihydroisoxazolo[5,4-d]pyrimidin-6-yl)propyl][(2-méthylphényl)carbonyl]amino}propyl)carbamate de *tert-*butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-3-méthyl-benzamide ;
(±)-(3-{[1-(5-Benzyl-3-méthyl-4-oxo-4,5-dihydroisoxazolo[5,4-d]pyrimidin-6-yl)propyl][(3-méthylphényl)carbonyl]amino}propyl)carbamate de *tert-*butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-4-chloro-benzamide ;
(±)-(3-{[1-(5-Benzyl-3-méthyl-4-oxo-4,5-dihydroisoxazolo[5,4-d]pyrimidin-6-yl)propyl][(4-chlorophényl)carbonyl]amino}propyl)carbamate de *tert-*butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-isonicotinamide ;
(±)-(3-{[1-(5-Benzyl-3-méthyl-4-oxo-4,5-dihydroisoxazolo[5,4-d]pyrimidin-6-yl)propyl](pyridin-4-ylcarbonyl)amino}propyl)carbamate de *tert*-butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-benzènesulfonamide ;
(±)-(3-{[1-(5-Benzyl-3-méthyl-4-oxo-4,5-dihydroisoxazolo[5,4-d]pyrimidin-6-yl)propyl](phénylsulfonyl)amino}propyl)carbamate de *tert*-butyle ;
(3-Amino-propyl)-[1-(5-benzyl-3-méthyl-4-oxo-4,5-dihydro-isoxazolo[5,4-d]pyrimidin-6-yl)-propyl]-amide d'acide (±)-biphényl-4-carboxylique ;
(±)-Acétate-*N*-(3-aminopropyl)-*N*-[1-(5-benzyl-3-méthyl-4-oxo-4,5-dihydroisoxazolo[5,4-d]pyrimidin-6-yl)propyl]biphényl-4-carboxamide de *tert*-butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-4-éthyl-benzamide ;
(±)-(3-{[1-(5-Benzyl-3-méthyl-4-oxo-4,5-dihydroisoxazolo[5,4-d]pyrimidin-6-yl)propyl][(4-éthylphényl)carbonyl]amino}propyl)carbamate de *tert-*butyle ;
(±)-6-(1-{(3-Amino-propyl)-[2-(4-chloro-phényl)-2-oxo-éthyl]-amino}-propyl)-5-benzyl-3-méthyl-5H-isoxazolo[5,4-d]pyrimidin-4-one ;
(±)-(3-{[1-(5-Benzyl-3-méthyl-4-oxo-4,5-dihydroisoxazolo[5,4-*d*]pyrimidin-6-yl)propyl][2-(4-chlorophényl)-2-oxoéthyl]amino}propyl)carbamate de *tert*-butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-4-chloro-benzènesulfonamide ;
(±)-(3-{[1-(5-Benzyl-3-méthyl-4-oxo-4,5-dihydroisoxazolo[5,4-d]pyrimidin-6-yl)propyl][(4-chlorophényl)sulfonyl]amino}propyl)carbamate de *tert-*butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-4-tert-butyl-benzènesulfonamide ;
(±)-(3-{[1-(5-Benzyl-3-méthyl-4-oxo-4,5-dihydroisoxazolo[5,4-*d*]pyrimidin-6-yl)propyl][(4-*tert-*butylphényl)sulfonyl]amino}propyl)carbamate de *tert-*butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-3-méthyl-benzènesulfonamide ;
(±)-(3-{[1-(5-Benzyl-3-méthyl-4-oxo-4,5-dihydroisoxazolo[5,4-d]pyrimidin-6-yl)propyl][(3-méthylphényl)sulfonyl]amino}propyl)carbamate de *tert-*butyle ;
(3-Amino-propyl)-[1-(5-benzyl-3-méthyl-4-oxo-4,5-dihydro-isoxazolo[5,4-d]pyrimidin-6-yl)-propyl]-amide d'acide (±)-quinoléine-2-carboxylique ;
(±)-(3-{[1-(5-Benzyl-3-méthyl-4-oxo-4,5-dihydroisoxazolo[5,4-d]pyrimidin-6-yl)propyl](quinoléin-2-ylcarbonyl)amino}propyl)-carbamate de *tert*-butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-4-méthyl-benzènesulfonamide ;
(±)-(3-{[1-(5-Benzyl-3-méthyl-4-oxo-4,5-dihydroisoxazolo[5,4-d]pyrimidin-6-yl)propyl][(4-méthylphényl)sulfonyl]amino}propyl)carbamate de *tert-*butyle ;
(3-Amino-propyl)-[1-(5-benzyl-3-méthyl-4-oxo-4,5-dihydro-isoxazolo[5,4-d]pyrimidin-6-yl)-propyl]-amide d'acide (±)-5-méthyl-isoxazole-3-carboxylique ;
(±)-(3-{[1-(5-Benzyl-3-méthyl-4-oxo-4,5-dihydroisoxazolo[5,4-d]pyrimidin-6-yl)propyl][(5-méthylisoxazol-3-yl)carbonyl]amino}propyl)carbamate de *tert*-butyle ;
(3-Amino-propyl)-[1-(5-benzyl-3-méthyl-4-oxo-4,5-dihydro-isoxazolo[5,4-d]pyrimidin-6-yl)-propyl]-amide d'acide (±)-cyclobutanecarboxylique ;
(±)-(3-{[1-(5-Benzyl-3-méthyl-4-oxo-4,5-dihydroisoxazolo[5,4-*d*]pyrimidin-6-yl)propyl](cyclobutylcarbonyl)amino}propyl)carbamate de *tert*-butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-isobutyramide ;
(±)-(3-{[1-(5-Benzyl-3-méthyl-4-oxo-4,5-dihydroisoxazolo[5,4-*d*]pyrimidin-6-yl)propyl](2-méthylpropanoyl)amino}propyl)carbamate de *tert*-butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-3-méthyl-butyramide ;
(±)-(3-{[1-(5-Benzyl-3-méthyl-4-oxo-4,5-dihydroisoxazolo[5,4-d]pyrimidin-6-yl)propyl](3-méthylbutanoyl)amino}propyl)carbamate de *tert*-butyle ;
(±)-N-(3-aminopropyl)-N-[1-(5-benzyl-3-méthyl-4-oxo-4,5-dihydroisoxazolo[5,4-d]pyrimidin-6-yl)propyl]-4-chlorobenzènesulfonamide ;
(±)-(3-{[1-(5-Benzyl-3-méthyl-4-oxo-4,5-dihydroisoxazolo[5,4-*d*]pyrimidin-6-yl)propyl][(4-chlorophényl)sulfonyl]amino}propyl)carbamate de *tert-*butyle ;
(±)-N-(3-Amino-propyl)-N-[1-(5-benzyl-3-méthyl-4-oxo-4,5-dihydro-isoxazolo[5,4-d]pyrimidin-6-yl)-propyl]-2-méthoxy-acétamide ;
(±)-(3-{[1-(5-Benzyl-3-méthyl-4-oxo-4,5-dihydroisoxazolo[5,4-*d*]pyrimidin-6-yl)propyl](méthoxyacétyl)amino}propyl)carbamate de *tert*-butyle ;
(±)-*N*-(3-aminopropyl)-*N*-[1-(5-benzyl-3-méthyl-4-oxo-4,5-dihydroisoxazolo[5,4-*d*]pyrimidin-6-yl)propyl]-4-propylbenzamide ;
(±)-(3-{[1-(5-Benzyl-3-méthyl-4-oxo-4,5-dihydroisoxazolo[5,4-*d*]pyrimidin-6-yl)propyl][(4-propylphényl)carbonyl]amino}propyl)carbamate de *tert-*butyle ;
(±)-N-(3-Amino-propyl)-N-[1-(5-benzyl-3-méthyl-4-oxo-4,5-dihydro-isoxazolo[5,4-*d*]pyrimidin-6-yl)-propyl]-4-bromo-benzamide ;
(±)-(3-{[1-(5-Benzyl-3-méthyl-4-oxo-4,5-dihydroisoxazolo[5,4-*d*]pyrimidin-6-yl)propyl][(4-bromophényl)carbonyl]amino}propyl)carbamate de *tert-*butyle ;
Chlorhydrate de (±)-N-(3-Aminopropyl)-N-[1-(5-benzyl-3-isopropyl-4-oxo-4,5-dihydroisoxazolo[5,4-d]pyrimidin-6-yl)-propyl]-4-méthylbenzamide ;
(±)-N-(3-Aminopropyl)-N-[1-(5-benzyl-3-isopropyl-4-oxo-4,5-dihydroisoxazolo[5,4-d]pyrimidin-6-yl)-propyl]-4-méthylbenzamide ;
(±)-[3-({1-[5-Benzyl-3-(1-méthyléthyl)-4-oxo-4,5-dihydroisoxazolo[5,4-*d*]pyrimidin-6-yl]propyl}[(4-méthylphényl)carbonyl]amino)propyl]carbamate de *tert-*butyle ;
Chlorhydrate de (±)-N-(3-Aminopropyl)-N-{1-[5-(4-chlorobenzyl)-3-isopropyl-4-oxo-4,5-dihydro-isoxazolo[5,4-d]pyrimidin-6-yl]-propyl}-4-méthylbenzamide ;
(±)-N-(3-Aminopropyl)-N-{1-[5-(4-chlorobenzyl)-3-isopropyl-4-oxo-4,5-dihydro-isoxazolo[5,4-d]pyrimidin-6-yl]-propyl}-4-méthylbenzamide ;
(±)-[3-({1-[5-(4-Chlorobenzyl)-3-(1-méthyléthyl)-4-oxo-4,5-dihydroisoxazolo[5,4-*d*]pyrimidin-6-yl]propyl}[(4-méthylphényl)carbonyl]amino)propyl]-carbamate de tert-butyle ;
(±)-N-(3-Amino-propyl)-N-[1-(5-benzyl-3-méthyl-4-oxo-4,5-dihydro-isothiazolo[5,4-d]pyrimidin-6-yl)-propyl]-4-chloro-benzamide ;
Ester *tert*-butylique d'acide (±)-{3-[[1-(5-benzyl-3-méthyl-4-oxo-4,5-dihydro-isothiazolo[5,4-d]pyrimidin-6-yl)-propyl]-(4-chloro-benzoyl)-amino]-propyl}-carbamique ;
(±)-N-(3-Amino-3-méthyl-butyl)-N-[1-(5-benzyl-3-méthyl-4-oxo-4,5-dihydro-isothiazolo[5,4-d]pyrimidin-6-yl)-propyl]-4-chloro-benzamide ; ou
Ester *tert*-butylique d'acide (±)-{3-[[1-(5-benzyl-3-méthyl-4-oxo-4,5-dihydro-isothiazolo[5,4-d]pyrimidin-6-yl)-propyl]-(4-chloro-benzoyl)-amino]-1,1-diméthylpropyl}-carbamique.

2. Composé ou un sel pharmaceutiquement acceptable de celui-ci selon la revendication 1, dans lequel A est C=O ou CH₂.

3. Composé ou un sel pharmaceutiquement acceptable de celui-ci selon la revendication 1, dans lequel A est C=O.

4. Composé ou un sel pharmaceutiquement acceptable de celui-ci selon la revendication 1, dans lequel B est un alkyle en C₁₋₁₂ facultativement substitué ou un hétérocycle facultativement substitué.

5. Composé ou un sel pharmaceutiquement acceptable de celui-ci selon la revendication 1, dans lequel B est un alkyle en C₁₋₄ facultativement substitué.

6. Composé ou un sel pharmaceutiquement acceptable de celui-ci selon la revendication 1, dans lequel B est un alkyle en C₁₋₄ facultativement substitué où un tel substituant est indépendamment choisi parmi -NH₂, -OH, -NHCH₃, -N(CH₃)_{2,} -NH-cyclopropane, -NH-cyclobutane, l'azétidine, la pyrrolidine ou la pipéridine.

7. Composé ou un sel pharmaceutiquement acceptable de celui-ci selon la revendication 1, dans lequel D est O facultativement substitué par R⁸.

8. Composé ou un sel pharmaceutiquement acceptable de celui-ci selon la revendication 1, dans lequel D est N facultativement substitué par un ou plusieurs R⁸.

9. Composé ou un sel pharmaceutiquement acceptable de celui-ci selon la revendication 1, dans lequel R¹ et R² en combinaison forment un isothiazole condensé facultativement substitué, ou un isoxazole condensé facultativement substitué qui est facultativement substitué par 1 ou 2 substituants, où ledit substituant est choisi parmi un alkyle en C₁₋₆ ou un halogène.

10. Composé ou un sel pharmaceutiquement acceptable de celui-ci selon la revendication 1 dans lequel R³ est un aryle facultativement substitué.

11. Composé ou un sel pharmaceutiquement acceptable de celui-ci selon la revendication 1 dans lequel R³ est un aryle en C₅₋₇ facultativement substitué.

12. Composé ou un sel pharmaceutiquement acceptable de celui-ci selon la revendication 1 dans lequel R³ est un aryle en C₁₋₇ facultativement substitué où ledit substituant est indépendamment choisi parmi un alkyle en C₁₋₆, F, Cl, Br ou I.

13. Composé ou un sel pharmaceutiquement acceptable de celui-ci selon la revendication 1 dans lequel R⁴ et R⁵ sont H.

14. Composé ou un sel pharmaceutiquement acceptable de celui-ci selon la revendication 1 dans lequel R⁶ et R⁷ sont indépendamment choisis parmi H, ou un alkyle en C₁₋₁₂ facultativement substitué.

15. Composé ou un sel pharmaceutiquement acceptable de celui-ci selon la revendication 1 dans lequel R⁶ et R⁷ sont indépendamment choisis parmi H, ou un alkyle en C₁₋₆.

16. Composé ou un sel pharmaceutiquement acceptable de celui-ci selon la revendication 1 dans lequel R⁸ est indépendamment choisi parmi H, un alkyle en C₁₋₁₂ facultativement substitué ou un hétérocycle facultativement substitué.

17. Composé ou un sel pharmaceutiquement acceptable de celui-ci selon la revendication 1 dans lequel R⁹ est indépendamment choisi parmi un aryle facultativement substitué ou un hétérocycle facultativement substitué.

18. Composé ou un sel pharmaceutiquement acceptable de celui-ci selon la revendication 1 dans lequel R⁹ est indépendamment choisi parmi un aryle ou un hétérocycle, chacun étant facultativement substitué par 1 ou 2 substituants où ledit substituant est indépendamment choisi parmi - (alkyle en C₁₋₆), -O(alkyle en C₁₋₆),F, Cl, Br ou I.

19. Composé ou un sel pharmaceutiquement acceptable de celui-ci selon la revendication 1 dans lequel R⁹ est un aryle en C₅₋₇ facultativement substitué par 1 ou 2 substituants où ledit substituant est indépendamment choisi parmi - (alkyle en C₁₋₆), -O(alkyle en C₁₋₆), F, Cl, Br ou I.

20. Composé ou un sel pharmaceutiquement acceptable de celui-ci selon la revendication 1 dans lequel :
n est 1 ;
A est CO ou CH₂ ;
B est un alkyle en C₁₋₆ facultativement substitué ;
D est N ou O ;
R¹ et R² en combinaison forment un isothiazole ou isoxazole condensé ;
R³ est un phényle facultativement substitué ;
R⁴ et R⁵ sont H ;
R⁶ et R⁷ sont H ou un alkyle en C₁₋₁₂ facultativement substitué ;
R⁸ est H ou un alkyle en C₁₋₆ facultativement substitué ;
R⁹ est un phényle facultativement substitué.

21. Composé ou un sel pharmaceutiquement acceptable de celui-ci de formule (I) choisi parmi :
N-(3-Amino-propyl)-N-{1-[5-(4-fluoro-benzyl)-3-méthyl-4-oxo-4,5-dihydro-isothiazolo[5,4-d]pyrimidin-6-yl]-propyl}-4-méthyl-benzamide
N-(3-Amino-propyl)-N-[1-(5-benzyl-3-méthyl-4-oxo-4,5-dihydro-isothiazolo[5,4-d]pyrimidin-6-yl)-propyl]-4-bromo-benzamide ;
N-(3-Amino-propyl)-N-[1-(5-benzyl-3-méthyl-4-oxo-4,5-dihydro-isothiazolo[5,4-d]pyrimidin-6-yl)-propyl]-3-fluoro-4-méthyl-benzamide ;
N-(3-Amino-propyl)-N-[1-(5-benzyl-3-méthyl-4-oxo-4,5-dihydro-isothiazolo[5,4-d]pyrimidin-6-yl)-propyl]-2,3-dichloro-benzamide ;
(3-Amino-propyl)-[1-(5-benzyl-3-méthyl-4-oxo-4,5-dihydro-isothiazolo[5,4-d]pyrimidin-6-yl)-propyl]-amide d'acide naphtalène-2-carboxylique ;
(3-Amino-propyl)-[1-(5-benzyl-3-méthyl-4-oxo-4,5-dihydro-isothiazolo[5,4-d]pyrimidin-6-yl)-propyl]-amide d'acide benzo[b]thiophène-2-carboxylique ;
N-Azétidin-3-ylméthyl-N-[1-(5-benzyl-3-méthyl-4-oxo-4,5-dihydro-isothiazolo[5,4d]pyrimidin-6-yl)-propyl]-4-méthyl-benzamide ;
N-[1-(5-Benzyl-3-méthyl-4-oxo-4,5-dihydro-isothiazolo[5,4-d]pyrimidin-6-yl)-propyl]-4-méthyl-N-pipéridin-3-ylméthyl-benzamide ;
N-(2-Amino-éthyl)-N-[1-(5-benzyl-3-méthyl-4-oxo-4,5-dihydro-isothiazolo[5,4-d]pyrimidin-6-yl)-propyl]-4-méthyl-benzamide ;
N-[1-(5-Benzyl-3-méthyl-4-oxo-4,5-dihydro-isothiazolo[5,4-d]pyrimidin-6-yl)-propyl]-N-(2-diméthylamino-éthyl)-4-méthyl-benzamide ;
N-[1-(5-Benzyl-3-méthyl-4-oxo-4,5-dihydro-isothiazolo[5,4-d]pyrimidin-6-yl)-propyl]-N-(3-diméthylamino-propyl)-4-méthyl-benzamide ;
N-[1-(5-Benzyl-3-méthyl-4-oxo-4,5-dihydro-isothiazolo[5,4-d]pyrimidin-6-yl)propyl]-N-[3-(isopropylamino)propyl]-4-méthylbenzamide ;
N-[1-(5-Benzyl-3-méthyl-4-oxo-4,5-dihydro-isothiazolo[5,4-d]pyrimidin-6-yl)propyl]-N-[3-(cyclopropylamino)propyl]-4-méthylbenzamide ;
N-(3-Azétidin-1-ylpropyl)-N-[1-(5-benzyl-3-méthyl-4-oxo-4,5-dihydro-isothiazolo[5,4-d]pyrimidin-6-yl)propyl]-4-méthylbenzamide ;
N-[1-(5-Benzyl-3-méthyl-4-oxo-4,5-dihydro-isothiazolo[5,4-d]pyrimidin-6-yl)propyl]-4-méthyl-N-[3-(3-pyrrolidin-1-ylpropyl)benzamide ;
N-[1-(5-benzyl-3-méthyl-4-oxo-4,5-dihydro-isothiazolo[5,4-d]pyrimidin-6-yl)propyl]-4-méthyl-N-[3-(méthylamino)propyl]benzamide ;
N-[1-(5-Benzyl-3-méthyl-4-oxo-4,5-dihydro-isothiazolo[5,4-d]pyrimidin-6-yl)-propyl]-N-(3-hydroxypropyl)-4-méthyl-benzamide ;
N-(3-Amino-propyl)-N-[1-(5-benzyl-3-méthyl-4-oxo-4,5-dihydro-isothiazolo[5,4-d]pyrimidin-6-yl)-propyl]-4-méthyl-benzamide ;
N-(3-Amino-propyl)-N-[1-(5-benzyl-3-méthyl-4-oxo-4,5-dihydro-isothiazolo[5,4-d]pyrimidin-6-yl)-2-méthylpropyl]-4-méthyl-benzamide ;
N-(3-Amino-propyl)-N-{1-[5-(4-fluoro-benzyl)-3-méthyl-4-oxo-4,5-dihydro-isothiazolo[5,4-d]pyrimidin-6-yl]-2-méthyl-propyl}-4-méthyl-benzamide ;
5-Benzyl-6-{1-[(3-hydroxy-propyl)-(4-méthyl-benzyl)-amino]-propyl}-3-méthyl-5H-isothiazolo[5,4-d]pyrimidin-4-one ;
N-(3-Amino-propyl)-N-{1-[5-(4-fluoro-benzyl)-3-méthyl-4-oxo-4,5-dihydro-isoxazolo[5,4-d]pyrimidin-6-yl]-2-méthyl-propyl}-4-méthyl-benzamide ;
N-(3-Amino-propyl)-3-fluoro-N-{1-[5-(4-fluoro-benzyl)-3-méthyl-4-oxo-4,5-dihydro-isoxazolo[5,4-d]pyrimidin-6-yl]-2-méthyl-propyl}-4-méthyl-benzamide ;
N-(3-Amino-propyl)-N-[1-(5-benzyl-3-méthyl-4-oxo-4,5-dihydro-isoxazolo[5,4-d]pyrimidin-6-yl)-2-méthylpropyl]-3-fluoro-4-méthyl-benzamide ;
N-(3-Amino-propyl)-N-[1-(5-benzyl-3-méthyl-4-oxo-4,5-dihydro-isoxazolo[5,4-d]pyrimidin-6-yl)-2-méthylpropyl]-4-méthyl-benzamide ;
N-(3-Amino-propyl)-N-[1-(5-benzyl-3-méthyl-4-oxo-4,5-dihydro-isoxazolo[5,4-d]pyrimidin-6-yl)-propyl]-4-fluoro-benzamide ;
N-(3-Amino-propyl)-N-[1-(5-benzyl-3-méthyl-4-oxo-4,5-dihydro-isoxazolo[5,4-d]pyrimidin-6-yl)-propyl]-2,3-dichloro-benzamide ;
N-(3-Amino-propyl)-N-[1-(5-benzyl-3-méthyl-4-oxo-4,5-dihydro-isoxazolo[5,4-d]pyrimidin-6-yl)-propyl]-3-fluoro-4-méthyl-benzamide ;
N-(3-Amino-propyl)-N-[1-(5-benzyl-3-méthyl-4-oxo-4,5-dihydro-isoxazolo[5,4-d]pyrimidin-6-yl)-propyl]-4-méthoxy-benzamide.

22. Composé ou un sel pharmaceutiquement acceptable de celui-ci selon l'une quelconque des revendications 1 à 21, pour une utilisation en tant que médicament.

23. Utilisation d'un composé ou un sel pharmaceutiquement acceptable de celui-ci selon l'une quelconque des revendications 1 à 21 dans la fabrication d'un médicament pour le traitement ou la prophylaxie de troubles associés au cancer.

24. Composition pharmaceutique comprenant un composé comme défini dans l'une quelconque des revendications 1 à 21, ou un sel pharmaceutiquement acceptable de celui-ci, conjointement avec au moins un véhicule, diluant ou excipient pharmaceutiquement acceptable.
